# EUROPEAN PATENT APPLICATION

(11) **EP 2 727 465 A1**
(43) Date of publication of application: **07.05.2014**
(21) Application number: 12007446.3
(22) Date of filing: 31.10.2012
(51) Int. Cl.: A01K 67/027, C12N 9/00, G01N 33/50

(54) **Animal model for type 2 diabetes and obesity**

(71) Applicant: Universitätsklinikum Freiburg, 79106 Freiburg (DE)
(72) Inventor: Schüle, Roland, 79367 Weisweil (DE); Duteil, Delphine, 67400 Illkirch (FR); Metzger, Eric, 68600 Neuf-Brisach (FR); Günther, Thomas, 79104 Freiburg (DE)
(74) Representative: Kalhammer, Georg

(57) **Abstract**

The present invention relates to the use of a genetically modified non-human animal as an animal model for obesity or obesity-related disorders, wherein the amount of Lysine-specific Demethylase 1 (LSD1) in at least one tissue or at least one cell type of said animal is reduced.

## Description

### FIELD OF THE INVENTION

The present invention relates to the use of a genetically modified non-human animal as an animal model for type 2 diabetes, obesity and related disorders, wherein the amount of Lysine-specific Demethylase 1 (LSD1) in at least one tissue or at least one cell type of said animal is reduced. The invention further pertains to a method for identifying a compound useful in the treatment and/or prevention of obesity or an obesity-related disorder, and to an LSD1 modulator for use in the treatment and/or prevention of obesity or an obesity-related disorder.

### BACKGROUND OF THE INVENTION

In industrialized countries, characterized by a sedentary lifestyle and high-caloric diet, energy balance is often deregulated leading to the development of obesity, dyslipidemia, hyperglycemia, and insulin resistance (type 2 diabetes). Among other organs, adipose tissue is an important regulator of energy balance (Langin, 2010). There are two major types of adipose tissue in mammals, white adipose tissue (WAT) and brown adipose tissue (BAT), which are present in different compartments and show distinct metabolic characteristics. Unilocular WAT is highly adapted to store excess energy in the form of triglycerides and is mainly located in the abdominal and subcutaneous areas. Conversely, multilocular cells from BAT, which are mainly located in the interscapular area (iBAT), oxidize chemical energy to produce heat as a defense against hypothermia and obesity (Himms-Hagen, 1990; Langin, 2010). Fatty acid oxidation and heat production by brown adipocytes is due to the intense metabolic activity of mitochondria, which express uncoupling protein 1 (Ucp1) (Ricquier and Bouillaud, 2000). WAT and BAT have long been assumed to have the same embryonic origin. However, recent evidence suggests that brown and white adipocytes are derived from two different precursors (Seale et al., 2008). These precursors can be discriminated by the presence or absence of the transcription factor myogenic factor 5 (Myf5), with brown adipocytes being derived from Myf5-positive and white adipocytes derived from Myf5-negative cells, respectively (Seale et al., 2008). In response to environmental cues such as cold or treatment with β3-adrenergic agonists, appearance of brown fat-like cells has been observed in mouse WAT (Himms-Hagen et al., 1994; Himms-Hagen et al., 2000; Young et al., 1984). These brown fat-like cells are called beige or brite cells. Interestingly, these beige fat cells are not derived from Myf5-expressing precursors (Seale et al., 2008), raising questions about their origin. It has been speculated that beige cells might originate from differentiation of a specific pool of precursor cells already present in WAT (Seale et al., 2011; Wu et al., 2012). Alternatively, beige fat cells could arise from direct conversion of white adipocytes (Granneman et al., 2005; Himms-Hagen et al., 2000; Loncar, 1991). Recently, Wu and colleagues proposed that beige cells exhibit a gene expression pattern distinct from either white or brown fat and that previously identified brown fat deposits in adult humans are indeed composed of beige adipocytes (Wu et al., 2012). However, it is neither known whether beige adipocytes constitute metabolically active fat cell nor are the transcriptional cascades that control the transformation of white to beige adipose tissues have been determined so far.

Over the last decade, evidence accumulated that epigenetics contribute to regulation of adipogenesis. In particular, posttranslational modifications of histone H3 lysines have been linked to either transcriptional activation or repression, depending on the modified residue. Methylation of lysine 4 in histone H3 (H3K4) correlates with gene activation, whereas methylation of lysine 9 or 27 in histone H3 (H3K9 or H3K27, respectively) is associated with transcriptional repression. As an example illustrating the key role of lysine methylation in adipocyte differentiation, it was reported that H3K4 methylation was required for Pparg and C/ebpa expression and thus positively regulates adipogenesis (Cho et al., 2009). In contrast, methylation of H3K27 by the methytransferase Ezh2 promotes adipogenesis by repressing the Wnt signaling (Wang et al., 2010). Lysine-specific demethylase 1 (LSD1), the first histone lysine demethylase described, is an amine oxidase that mediates histone demethylation via a FAD-dependent oxidative reaction. It has been shown that LSD1 selectively removes mono- and dimethyl groups from H3K4 or H3K9, thereby causing either repression or activation of gene transcription (Garcia-Bassets et al., 2007; Lee et al., 2005; Metzger et al., 2010; Metzger et al., 2005; Shi et al., 2004; Wang et al., 2009a; Wang et al., 2009b) (Zhu Ms). Recent in vitro studies suggest that LSD1 might play a role during fat cells differentiation in vitro (Hino et al., 2012; Musri et al., 2010). However, the precise function of Lsd1 in adipogenesis in vivo is currently unknown.

### SUMMARY OF THE INVENTION

The inventors of this application have shown that heterozygous mice in which one allele of the LSD1 gene has been disrupted and which show reduced expression of Lsd1 are prone to obesity and type 2 diabetes. These mice and other non-human animals having a reduced expression of Lsd1 are valuable tools for studying established and potential agents to treat type 2 diabetes and related diseases such as or insulin resistance and obesity.

In addition, the inventors found that transgenic overexpression of Lsd1 in mice improved the metabolic profile of the transgenic mice when fed a high-caloric diet, thereby attenuating obesity and diabetes.

The present invention therefore relates to the subject matter defined in items (1) to (15).
(1) The use of a modified non-human animal as an animal model for obesity or obesity-related disorders, wherein the amount of Lysine-specific Demethylase 1 (LSD1) in at least one tissue or at least one cell type of said animal is reduced, wherein the modified non-human animal preferably is a genetically modified non-human animal.
(2) The use of item (1), wherein the genetically modified non-human animal has a nucleic acid inserted in its genome, wherein the presence of the inserted nucleic acid in the genome of the animal results in reduced expression of LSD1.
(3) The use of item (1) or (2), wherein said genetic modification is a disruption of an allele of the endogenous gene encoding LSD1.
(4) The use of any one of the preceding items, wherein said animal is a non-human transgenic animal which is heterozygous for the disruption of the gene encoding LSD1.
(5) The use of any one of the preceding items, wherein the amount of LSD1 is reduced in tissue of said animal, preferably in adipose, liver or muscle of said animal.
(6) The use of any one of the preceding items, wherein said animal develops obesity after high fat diet.
(7) The use of any one of the preceding items, wherein said animal has at least one symptom of type 2 diabetes.
(8) The use of any one of the preceding items, wherein the animal is a rodent, preferably a mouse.
(9) A method for identifying a compound useful in the treatment and/or prevention of obesity or an obesity-related disorder, comprising (a) administering a test compound to a transgenic animal as defined in any one of items (1) to (8), and (b) determining the effect of the test compound on the initiation, maintenance, or progression of at least one obesity-related parameter in said transgenic animal, thereby identifying a compound that inhibits obesity or an obesity-related disorder.
(10) The method of item (9), wherein said obesity-related parameter is selected from the group consisting of the mass of white adipose tissue, the total mass of adipose tissue, glucose intolerance, and expression of a adipose tissue differentiation marker.
(11) A compound for use in the treatment and/or prevention of obesity or an obesity-related disorder, wherein said compound is capable of modulating LSD1 protein, the LSD1 gene or a target gene of LSD1.
(12) The compound for use according to claim 11, wherein the target gene of Lsd1 is selected from the group consisting of *Nrf1, Prdm16* and *Pgc-1*α*.*
(13) A method for identifying a compound useful in the treatment and/or prevention of obesity or an obesity-related disorder, comprising (i) contacting a cell with a test compound; (ii) determining expression level of the LSD1 gene and/or of a target gene of LSD1; and (iii) selecting the compound if the test compound is capable of increasing the expression of the LSD1 gene and/or of the target gene of LSD1, as compared to a control cell which has not been contacted with the test compound.
(14) The method of item (13), wherein the cell is an adipocyte or a preadipocyte, preferably a mouse 3T3-L1 cell, a 3T3-F442 cell or a 10T1/2 cell.
(15) The use according to any one of items (1) to (8), the method of item (9) or (10), the LSD1 inhibitor for use according to item (11) or (12), or the method of item (13) or (14), wherein said obesity-related disorder is selected from the group consisting of insulin resistance, type II diabetes, hypertension, hyperuricemia, fatty liver, non-alcoholic fatty liver disease, polycystic ovarian syndrome, acanthosis nigricans, hyperphagia, endocrine abnormalities, triglyceride storage disease, Bardet-Biedl syndrome, and Lawrence-Moon syndrome.
(16) The use of a non-human transgenic animal whose genome comprises a stably integrated transgenic nucleotide sequence encoding Lysine-specific Demethylase 1 (LSD1) operably linked to a promoter for studying metabolism and/or as an animal model for metabolic disorders.
(17) The use of item (16), wherein said LSD1 is human LSD1.
(18) The use of item (16) or (17), wherein said LSD1 comprises at least amino acids 2 to 852 of the amino acid sequence as shown in SEQ ID NO:3.
(19) The use according to any one of items (16) to (18), wherein said transgenic nucleotide sequence comprises nucleotides 4 to 2556 of the nucleotide sequence as shown in SEQ ID NO:1.
(20) The use according to any one of items (16) to (19), wherein said LSD1 is overexpressed in the transgenic animal.
(21) The use of item (20), wherein said overexpression is present at least in white adipose tissue.
(22) The use according to any one of items (16) to (21), wherein said promoter is a Rosa26 promoter.
(23) The use according to any one of items (16) to (22), wherein said animal has a reduced weight relative to a wild type mouse.
(24) The use according to any one of items (16) to (23), which is a rodent, preferably a mouse.
(25) A use according to any one of items (16) to (24), wherein said animal does not comprise other transgenic nucleotide sequences.
(26) A method for identifying a compound useful in the treatment and/or prevention of a metabolic disorder, comprising (a) administering a test compound to a transgenic animal as defined in any one of items (16 to (25) and (b) determining the effect of the test compound on the initiation, maintenance, or progression of at least one metabolic parameter in said transgenic animal, thereby identifying a compound that inhibits the metabolic disorder, wherein said metabolic parameter is selected from the group consisting of the mass of white adipose tissue, the total mass of adipose tissue, glucose intolerance, expression of a adipose tissue differentiation marker.
(27) A method according to item (26), wherein said test compound is selected from the group consisting of LSD1 inhibitors or LSD1 modulators, modulators of androgen receptor, modulators of p53, and modulators of Rb.
(28) A LSD1 inhibitor or LSD1 modulator for use in the treatment and/or prevention of a metabolic disorder.
(29) The LSD1 inhibitor or LSD1 modulator of item (28), which is siRNA capable of inhibiting expression of the LSD1 gene.
(30) The use according to any one of items (16) to (25), the method of item (26) or (27), or the LSD1 inhibitor for use according to item (28) or (29), wherein said metabolic disorder is selected from the group consisting of eating disorders, body weight disorders, cachexia, anorexia, sarcopenia and wasting syndrome or disease.

### DETAILED DESCRIPTION OF THE INVENTION

In a first embodiment, the present invention provides an animal model for type 2 diabetes, obesity and related disorders. One aspect of the invention is the use of a modified non-human animal as an animal model for obesity or obesity-related disorders, wherein the amount of Lysine-specific Demethylase 1 (LSD1) in at least one tissue or at least one cell type of said animal is reduced. The modified non-human animal may be a genetically modified non-human animal, preferably it is a transgenic non-human animal. The animals in accordance with this embodiment have a reduced amount of LSD1 in at least one tissue. These animals typically exhibit a reduced expression of LSD1 in one or more tissues. The reduced expression may or may not affect all tissues of the animal. Preferably, the amount of LSD1 protein or Lsd1 mRNA is reduced in fat tissue, e.g. in BAT and/or WAT.

In a second embodiment, the invention provides an animal model for metabolic disorders including, but not limited to, eating disorders, body weight disorders, cachexia, anorexia, sarcopenia and wasting syndrome or disease. This embodiment includes the use of a non-human transgenic animal whose genome comprises a stably integrated transgenic nucleotide sequence encoding Lysine-specific Demethylase 1 (LSD1) operably linked to a promoter for studying metabolism and/or as an animal model for metabolic disorders. The animals in accordance with this embodiment have an increased amount of LSD1 in at least one tissue. These animals typically overexpress LSD1 in at least one tissue. The overexpression may or may not affect all tissues of the animal. Preferably, the amount of LSD1 protein or Lsd1 mRNA is increased in fat tissue, e.g. in BAT and/or WAT.

In both embodiments, the reduced expression and the increased expression (overexpression), respectively, is relative to a control animal. The control animal may be a wild type animal which is substantially identical to the modified animal or transgenic animal, except for the genetic manipulation. The amount of LSD1 may be determined at the protein level, e.g. by immunoassays using antibodies against LSD1. The expression level of Lsd1 may be determined by quantitative RT-PCR, detecting the amount of Lsd1 mRNA.

In the first embodiment, the amount of LSD1 protein, or the amount of Lsd1 mRNA may be reduced relative to the control animal by at least 10%, preferably by at least 20%, more preferably by at least 30%, more preferably by at least 40%, e.g. by about 50%. For example, the amount of LSD1 protein, or the amount of Lsd1 mRNA may be reduced relative to the control animal by 10% to 90%, preferably by 20% to 80%, more preferably by 30% to 70%, more preferably by 40% to 60%.

In the animals of the first embodiment the protein and/or mRNA levels of *Prdm16, Pgc-1*α and *Ucp1* are reduced, e.g. by at least 10%, more preferably by at least 25%, most preferably by at least about 50%, relative to a control animal. Body fat mass in these animals may be increased by about 10% relative to a control animal, in particular after high fat diet. Blood glucose levels in these animal may be increased by about 10% to about 50%, or by about 20% to about 40%, relative to a control animal. Moreover, the animals of the first embodiment typically exhibit reduced insulin sensitivity and glucose uptake relative to a control animal, wherein insulin sensitivity and glucose uptake may be determined according to the tests described in the Examples hereinbelow.

In the second embodiment, the amount of LSD1 protein, or the amount of Lsd1 mRNA may be increased relative to the control animal by at least 10%, preferably by at least 25%, more preferably by at least 50%, more preferably by at least 75%, e.g. by about 100%. For example, the amount of LSD1 protein, or the amount of Lsd1 mRNA may be increased relative to the control animal by 10% to 1000%, preferably by 25% to 500%, more preferably by 50% to 200%, more preferably by 75% to 150%. In this embodiment the phrase "LSD1 protein" includes endogenous and exogenous LSD1 protein. The phrase "Lsd1 mRNA" includes endogenous and exogenous Lsd1 mRNA.

The transgenic non-human animal may be a transgenic non-human vertebrate animal, preferably a mammal, preferably a rodent, such as a mouse. Suitable animals are available, or easily generated, using conventional methods, in a variety of genera, including rodents (e.g., rats), rabbits, guinea pigs, dogs, goats, sheep, cows, horses, pigs, llamas, camels or the like. Preferably, the non-human transgenic animal is a transgenic mouse.

The animal from which the progeny animal is descended is referred to as "progenitor animal". "Progeny" of a progenitor mammal are any animals which are descended from the progenitor as a result of sexual reproduction or cloning of the progenitor, and which have inherited genetic material from the progenitor. In this context, cloning refers to production of genetically identical offspring from DNA or a cell(s) of the progenitor animal. As used herein, "development of an animal" from a cell or cells (embryonic cells, for example), or development of a cell or cells into an animal, refers to the developmental process that includes growth, division and differentiation of a fertilized egg or embryonic cells (and their progeny) to form an embryo, and birth and development of that embryonic animal into an adult animal.

An animal is "derived from" a transgenic ovum, sperm cell, embryo or other cell if the transgenic ovum, sperm cell, embryo or other cell contributes DNA to the animal's genomic DNA. For example, a transgenic embryo of the invention can develop into a transgenic animal of the invention. A transgenic ovum of the invention can be fertilized to create a transgenic embryo of the invention that develops into a transgenic animal of the invention. A transgenic sperm of the invention can be used to fertilize an ovum to create a transgenic embryo of the invention that develops into a transgenic animal of the invention. A transgenic cell of the invention can be used to clone a transgenic animal of the invention.

As used herein, a "transgenic non-human mammal" is a non-human mammal into which an exogenous recombinant construct has been introduced, or its progeny. Such a mammal may have developed from (a) embryonic cells into which the construct has been directly introduced or (b) progeny cells of (a). As used herein, an "exogenous construct" is a nucleic acid that is artificially introduced, or was originally artificially introduced, into an animal. The term "artificial introduction" excludes introduction of a construct into an animal through normal reproductive processes (such as by cross breeding). However, animals that have been produced by transfer of an exogenous construct through the breeding of a mammal comprising the construct (into whom the construct was originally "artificially introduced") are considered to "comprise the exogenous construct." Such animals are progeny of animals into which the exogenous construct has been introduced.

In accordance with the first embodiment, the transgenic non-human animal contains a disruption in an endogenous LSD1 gene such that at least one allele of the LSD1 gene is non-functional or does not express a functional LSD1, wherein the disruption is an insertion of a transgene into the endogenous LSD1 gene. The disruption can be, for example, an insertion, missense, frameshift, or deletion mutation. The disruption can also alter a promoter, enhancer, or splice site. The disruption can be insertion of a transgene. The transgene optionally encodes a selectable marker, such as, for example a LacZ reporter gene operably linked to a LSD1 promoter. The provided non-human animals are preferably heterozygous for LSD1. As used herein, the term heterozygous means that the animal has a disruption in one allele (i.e., endogenous gene) while the second allele is unaffected (i.e., does not contain a disruption). In a most preferred embodiment, the genome of the animal comprises a disruption of only one allele of the gene encoding LSD1. An example of this embodiment is the Lsd+/- mouse described in the Examples.

In accordance with the second embodiment, the invention relates to a non-human transgenic animal (e.g., a rodent, preferably a mouse) whose genome comprises a DNA sequence encoding hLSD1, or encoding a biologically active fragment or variant thereof, which is operably linked to an expression control sequence. Typically, the mouse according to this embodiment will overexpress LSD1 (see, for example, the LSD1-overexpressing mice described in the Examples). In this embodiment, the non-human transgenic animal of the invention is preferably one whose somatic and germ cells comprise at least one genomically integrated copy of a recombinant construct of the invention (a recombinant construct comprising a sequence encoding LSD, preferably hLSD1), or an active fragment or variant thereof, which sequence is operably linked to an expression control sequence. Alternatively, the disclosed transgene construct can also be assembled as an artificial chromosome, which does not integrate into the genome but which is maintained and inherited substantially stably in the animal. Artificial chromosomes of more than 200 kb can be used for this purpose. The present invention is also directed to the creation of transgenic mice in whose tissue specific expression of the hLSD1 transgene is driven by a tissue specific promoter, as is discussed more extensively below.

The invention further provides a transgenic gamete, including a transgenic ovum or sperm cell, a transgenic embryo, and any other type of transgenic cell or cluster of cells, whether haploid, diploid, or of higher zygosity having at least one disruption in the LSD1 gene.

As used herein, the term "embryo" includes a fertilized ovum or egg (i.e., a zygote) as well as later multicellular developmental stages of the organism. The disruption referred to above is preferably in the animal's somatic and germ cells.

Also included herein are progeny of the transgenic animal that preferably comprise a disruption of at least one allele of the LSD1 gene, and transgenic animals derived from a transgenic ovum, sperm, embryo or other cell of the invention.

The transgenic animal may be sterile although, preferably, it is fertile. The present invention further includes a cell line derived from a transgenic embryo or other transgenic cell of the invention, which contains a disruption of at least one allele of the LSD1 gene. Methods of isolating such cells and propagating them are known to those of skill in the art.

### Generation of transgenic animals

The transgenic non-human animals of the invention are produced by introducing transgenes into the germline of the non-human animal. Embryonal target cells at various developmental stages are used to introduce the transgenes of the invention. Different methods are used depending on the stage of development of the embryonal target cell(s). Such methods include, but are not limited to, microinjection of zygotes, viral integration, and transformation of embryonic stem cells as described below.

Microinjection of zygotes is the preferred method for incorporating transgenes into animal genomes. A zygote, which is a fertilized ovum that has not undergone pronuclei fusion or subsequent cell division, is the preferred target cell for microinjection of transgenic DNA sequences. The murine male pronucleus reaches a size of approximately 20 micrometers in diameter, a feature which allows for the reproducible injection of 1-2 picoliters of a solution containing transgenic DNA sequences. The use of a zygote for introduction of transgenes has the advantage that, in most cases, the injected transgenic DNA sequences will be incorporated into the host animal's genome before the first cell division. Brinster et al., Proc. Natl. Acad. Sci. USA 82:4438 (1985). As a consequence, all cells of the resultant transgenic animals (founder animals) stably carry an incorporated transgene at a particular genetic locus.

Viral integration can also be used to introduce the transgenes of the invention into an animal. The developing embryos are cultured in vitro to the blastocyte developmental stage. The blastomeres may be infected with appropriate retroviruses. Jaenich, Proc. Natl. Acad. Sci. USA 73:1260. Infection of the blastomeres is enhanced by enzymatic removal of the zona pellucida. Transgenes are introduced via viral vectors which are typically replication-defective but which remain competent for integration of viral-associated DNA sequences, including transgenic DNA sequences linked to such viral sequences, into the host animal's genome. Transfection is easily and efficiently obtained by culture of blastomeres on a monolayer of cells producing the transgene-containing viral vector. Alternatively, infection may be performed using cells at a later developmental stage, such as blastocoeles. In any event, most transgenic founder animals produced by viral integration will be mosaics for the transgenic allele; that is, the transgene is incorporated into only a subset of all the cells that form the transgenic founder animals. Moreover, multiple viral integration events may occur in a single founder animal, generating multiple transgenic alleles which will segregate in future generations of offspring. Introduction of transgenes into germline cells by this method is possible but probably occurs at a low frequency. However, once a transgene has been introduced into germline cells by this method, offspring may be produced in which the transgenic allele is present in all of the animal's cells, i.e., in both somatic and germline cells.

Embryonic stem (ES) cells can also serve as target cells for introduction of the transgenes of the invention into animals. ES cells are obtained from pre-implantation embryos that are cultured *in vitro.* Evans et al., Nature 292:154 (1981). ES cells that have been transformed with a transgene can be combined with an animal blastocyst, after which the ES cells colonize the embryo and contribute to the germline of the resulting animal. Once a transgene has been introduced into germline cells by this method, offspring may be produced in which the transgenic allele is present in all of the animal's cells, i.e., in both somatic and germline cells.

The transgenic nucleic acid may be stably integrated into germ line cells and transmitted to offspring of the transgenic animal as Mendelian loci. Other transgenic techniques result in mosaic transgenic animals, in which some cells carry the transgenes and other cells do not. In mosaic transgenic animals in which germ line cells do not carry the transgenes, transmission of the transgenes to offspring does not occur. Nevertheless, mosaic transgenic animals are capable of demonstrating phenotypes associated with the transgenes.

In practicing the invention, animals of the transgenic maintenance line are crossed with animals having a genetic background in which expression of the transgene results in symptoms of obesity or obesity-related disorders. Offspring that have inherited the transgenic nucleic acids of the invention are distinguished from littermates that have not inherited transgenic nucleic acids by analysis of genetic material from the offspring for the presence of nucleic acid sequences derived from the transgenic nucleic acids of the invention. For example, biological fluids that contain polypeptides uniquely encoded by the transgenic nucleic acids of the invention may be immunoassayed for the presence of the polypeptides. A simpler and more reliable means of identifying transgenic offspring comprises obtaining a tissue sample from an extremity of an animal, such as, for example, a tail, and analyzing the sample for the presence of nucleic acid sequences corresponding to the DNA sequence of a unique portion or portions of the transgenic nucleic acids of the invention. The presence of such nucleic acid sequences may be determined by, e.g., hybridization ("Southern") analysis with DNA sequences corresponding to unique portions of the transgene, analysis of the products of PCR reactions using DNA sequences in a sample as substrates, oligonucleotides derived from the transgene's DNA sequence, and the like.

### Targeting vectors

As used herein, the term "polynucleotide" is interchangeable with "nucleic acid." A polynucleotide of the present invention may be recombinant, natural, or synthetic or semi-synthetic, or any combination thereof. Polynucleotides of the invention may be RNA, PNA, LNA, or DNA, or combinations thereof. As used herein, the terms peptide, polypeptide and protein are also interchangeable.

A "recombinant construct" (also referred to herein as a "construct" for short) or a "transgene" of "transgenic nucleic acid" which is used to generate a transgenic animal of the second embodiment of the invention is a polynucleotide which comprises a sequence encoding LSD1 (preferably hLSD1), or an active fragment or variant thereof, which is operably linked to an expression control sequence. The coding sequence comprises LSD1 exon sequences, although it may optionally include intron sequences which are either derived from an hLSD1 genomic DNA or DNA of an unrelated chromosomal gene. The recombinant construct may comprise a sequence encoding mLSD1 or at least a biologically active fragment thereof. Preferably, the recombinant construct comprises a sequence encoding human LSD1 (hLSD1) or a biologically active fragment thereof. The amino acid sequences of hLSD1 and mLSD1 are shown in SEQ ID NO:3 and 5, respectively. The nucleotide sequence encoding mLSD1 is shown in SEQ ID NO:4. The nucleotide sequence encoding hLSD1 is shown in SEQ ID NO:1. The hLSD1 cDNA sequence including 5'- and 3'- untranslated regions is shown in SEQ ID NO:2. The nucleotide sequence of an exemplary recombinant construct is shown in SEQ ID NO:6. The amino acid sequence encoded by this construct is shown in SEQ ID NO:7.

For tissue-specific expression of the transgene in the transgenic animal, the coding sequence must be operably linked to an expression control sequence that drives expression specifically in that tissue. Suitable tissue-specific expression control sequences include the following: MMTV-LTR (for mammary-specific expression), etc.

### InduciblelRepressible Expression Control Systems

An inducible promoter is one which, in response to the presence of an inducer, is activated. Hence, a coding sequence driven by an inducible promoter can be turned on or off by providing or withdrawing the inducer. A promoter may be homologous, derived from the same species as the coding sequence. Preferably, the promoter is heterologous, that is, derived from another species, or even from a virus. hLSD1 constructs in accordance with the present invention may be operably linked to an inducible or repressible control elements. An repressible system, described by Gossen, M. et al., Proc Natl Acad Sci USA 89:5547-51 (1992), is based on the use of control elements of the tetracycline-resistance operon encoded in Tn10 of E. coli. The tet repressor is fused with the activating domain of Herpes simplex virus VP16 to generate a tetracycline-controlled transactivator. Such a transactivator is used to stimulate transcription from a promoter sequence, such as the CMV promoter IE.

A gene controlled by a promoter acting under the influence of the tetracycline-controlled transactivator can be constitutively expressed and turned off by using an effective concentration of tetracycline. Such a system can regulate a gene over about five orders of magnitude. The tetracycline-repressible system functions in vivo in mice, where tetracycline administration via the diet is used to keep the expression of the inducible gene off. Tetracycline analogs which cross the blood-brain barrier can be used if gene activity is desired in the brain.

Two steps of transfection may be used to produce the appropriate system. A first transfection is used to isolate clones expressing the transactivator. The best clones are identified by testing each in a transient transfection assay for the ability to express a marker gene, such as an estrogen-dependent luciferase. The second transfection involves the hLSD1 coding sequence under control of an inducible promoter into a transactivator-containing clone. One strategy involves first isolating a stable cell line expressing the inducible hLSD1 protein or peptide by cotransfection of both plasmids into appropriate target cells. After selection, for example with G418, clones showing estrogen-dependent expression of hLSD1 may be detected by an immunoassay or biological assay. To increase the rate of plasmid integration and to stabilize the integrated plasmids in the host genome, the plasmids are preferably linearized and cotransfected into cells in the presence of mammalian high molecular weight DNA as a carrier.

The relative advantages of a two vector system, as described above, over a single vector system involving a larger plasmid is that in a two vector system, multiple copies of the reporter plasmid (encoding the gene of interest) may be needed to obtain a detectable biological effect in a cell, while one or only a few copies of the transactivator-carrying plasmid may suffice.

According to the present invention, the hLSD1 DNA molecule is placed under the control of a promoter subject to regulation by a tetracycline-controlled transactivator. Such a construct (in a single vector or preferably two vector form) is delivered into target cells, whether embryonic, adult normal or tumor, either in vitro or in vivo. To express the hLSD1, tetracycline is withheld so that the hLSD1 DNA is expressed. To prevent the action of the hLSD1, for example, locally, tetracycline or an active congener of tetracycline is administered locally to the cells transfected with the constructs. Effective systemic doses (oral or parenteral) of tetracycline are in the range of about 0.1 mg to 1 g per day. In a preferred embodiment, the transactivator is maintained in the "on" position by withholding tetracycline.

An estrogen-inducible system described by Braselmann, S. et al. Proc Natl Acad Sci USA (1993) 90:1657-61, is based on the fact that most mammalian cells neither express any Gal4-like activity nor endogenous estrogen receptor (ER), thus rendering estrogen an inert signal for them. The authors developed a selective induction system based on the estrogen-regulatable transcription factor Gal-ER. Gal-ER consists of the DNA-binding domain of the yeast Gal4 protein fused to the hormone-binding domain of the human ER and hence exclusively regulates a transfected coding sequence under the control of a Gal4-responsive promoter in mammalian cells. This system includes a synthetic Gal4-responsive promoter which consists of four Gal4-binding sites, an inverted CCAAT element, a TATA box, and the adenovirus major late initiation region. This promoter shows extremely low basal activity in the absence of, and high inducibility in the presence of, ligand-activated Gal-ER. The transcription factor Gal-ER is rendered more potent and less susceptible to cell type-specific variation by fusing the strong activating domain of the herpesvirus protein VP16 onto its C-terminus. In response to estrogen, e.g., 17-β estradiol, Gal-ER-VP16 may induce the Gal4-responsive promoter at least 100-fold in transfected cells. Thus, the Gal-ER induction system is a powerful genetic switch for regulating heterologous genes. For induction of expression of the DNA molecules of the present invention in an estrogen inducible system in an animal, local or systemic treatment with estrogen would be required. An effective dose of an estrogen is a dose which would trigger the expression of an hLSD1-encoding nucleic acid of the present invention to produce hLSD1 and promote growth of hLSD1-expressing adipose cells. Such doses can be ascertained by one skilled in the art. Preferably, doses in the range of about 0.05 to 100 mg/kg of an estrogen are used in a single dose or in multiple doses over a period of about one week days to about 6 months, or even longer. Forms and preparations of estrogen and their usage in animals, particularly in humans, are well-known in the art. Estrogen analogues which are capable of specifically activating the exogenous transactivator while having fewer biological effects and side effects are preferred.

Ionizing radiation has been used to activate the transcription of exogenous genes, for example, encoding a cytotoxic protein TNF-I (Weichselbaum, R R et al., Int J Radiation Oncology Biol Phys 24:565-67 (1992)) This may be accomplished through the use of radiation-responsive elements distal to the transcription start site of such genes. See, for example, Hallahan, D et al., Proc Natl Acad Sci USA 88:2152-20 (1991); Datta, R et al., Proc Natl Acad Sci USA 89:10149-53 (1992); Weichselbaum et al., supra; Hallahan, D E et al. J Biol Chem 268:4903-07 (1993); Weichselbaum, R R et al., Intl J Radiation Oncology Bio. Phys 30:229-34 (1994); Hallahan, D E et al. Nature Med 1:786-91 (1995), which references are hereby incorporated by reference in their entirety. Thus, the present invention provides methods for the spatial and temporal control of gene expression with such radiation-inducible promoters to activate hLSD1. The hLSD1 coding sequence is placed in a vector under control of a radiation-inducible promoter.

In a preferred embodiment, the expression control sequence (either a ubiquitously acting expression control sequence or a tissue-specific one) is expressed in a regulatable fashion, meaning that it is preferably a component of any of a number of well-known regulatable expression systems.

Methods of making recombinant constructs are conventional. Such methods, as well as many other molecular biological methods used in conjunction with the present invention, are discussed, e.g., in Sambrook, et al. (1989), Molecular Cloning, a Laboratory Manual, Cold Harbor Laboratory Press, Cold Spring Harbor, N.Y.; Ausubel et al. (1995). Current Protocols in Molecular Biology, N.Y., John Wiley & Sons; Davis et al. (1986), Basic Methods in Molecular Biology, Elsevier Sciences Publishing, Inc., New York; Hames et al. (1985), Nucleic Acid Hybridization, IL Press; Dracopoli et al. Current Protocols in Human Genetics, John Wiley & Sons, Inc.; and Coligan et al. Current Protocols in Protein Science, John Wiley & Sons, Inc. See, also, the Examples herein.

In a specific embodiment of this invention, the transgenic animal does not comprise additional transgenic sequences. This means that the transgenic construct present in the transgenic animal is the only transgenic construct present in the animal of this invention. The transgenic animal preferably does not comprise further genetic germ line modifications other than that described hereinabove. The genetic background of the transgenic animal of the invention is 'wild type' except for the genetic modifications described hereinabove. This embodiment is preferred, as it allows studying the effect of any methods interfering with the action of LSD1.

### Method for identifying potential therapeutic agents

Another aspect of this invention is a method for identifying a compound which inhibits the growth of adipose tissue, comprising (a) administering a test compound to a transgenic animal according to the present invention and (b) determining the effect of the test compound on the growth or survival of the adipocytes or adipose tissue in said transgenic animal, thereby identifying a compound that inhibits the growth of adipose tissue.

Another aspect of this invention is a method for identifying a compound which is useful in the treatment of type 2 diabetes, obesity and/or a related disorder, comprising (a) administering a test compound to a transgenic animal according to the first embodiment of the present invention and (b) determining the effect of the test compound on at least one metabolic parameter, at least one obesity-related parameter or at least one diabetes-related parameter, and optionally (c) selecting the compound if the effect is indicative of inhibition or amelioration of type 2 diabetes, obesity and/or a related disorder.

The methods may comprise the steps (i) administering a test compound to a transgenic animal according to the present invention, (ii) administering the same test compound to a control animal, and (iii) determining the effect of the test compound on the transgenic animal as compared to said control animal. The control animal is preferably an animal of the same species as the transgenic animal. The control animal does not show reduced or increased expression of LSD1 or only to a significantly lower degree, relative to a wild type animal. In one embodiment, the control animal does not carry the transgenic nucleotide sequence present in the test animal of step (i). In another embodiment, the control animal is also carrying the same transgenic nucleotide sequence as the transgenic animal used in step (i) but there is no or only a slight reduction or increase in expression. This can be achieved by the use of inducible transgene constructs, see *supra.*

The effect to be determined in step (iii) may be any change in any clinically or biologically relevant parameter including body weight, adipose tissue mass, adipose tissue size, presence of WAT markers, number of adipocytes, presence of lipomas, survival rate and the like (relative to the control animal). The methods of analysis include but are not limited to determining the body weight, adipose tissue mass, adipose tissue size, presence of WAT markers, number of adipocytes, presence of lipomas, survival rate and the like, e.g. by histological methods and/or biochemical methods. A particularly preferred parameter is insulin sensitivity and glucose tolerance.

The test compound may be selected as a compound which is useful in the treatment of type 2 diabetes, obesity or a related disorder if there is a significant difference in at least one of the clinically or biologically relevant parameters tested, for example when the parameter (e.g. adipose tissue mass, adipose tissue size, presence of WAT markers, number of adipocytes, presence of lipomas, survival rate etc.) in the transgenic test animal used in step (i) is significantly reduced (e.g. by at least 10%, preferably by at least 25%, more preferably by at least 50%) relative to that of the control animal.

The test compound may be selected as a compound which is useful in the treatment of type 2 diabetes, obesity or a related disorder if blood glucose levels are reduced by the test compound, or if insulin sensitivity and/or glucose uptake are significantly increased.

The compound may be selected if any one the the target genes of LSD1 (e.g. *Nrf1, Prdm16* and *Pgc-1*α) is upregulated by the test compound, e.g. by at least about 10% or at least about 25%, or at least about 50%, or at least about 100%. The quantitation is preferably done via RT-PCR.

In another embodiment, the compound may be selected if any one the the target genes of LSD1 (e.g. *Nrf1, Prdm16* and *Pgc-1*α) is downregulated by the test compound, e.g. by at least about 10% or at least about 25%, or at least about 50%. The quantitation is preferably done via RT-PCR.

The compounds used as test compounds may be modulators of the LSD1 gene, of the LSD1 protein, or of any target gene of LSD1. Modulators include activators and inhibitors. The modulators are preferably activators of the LSD1 protein or activators of the LSD1 gene (see *infra*).

The invention further pertains to a method for identifying a compound which inhibits adipose cell or tissue growth, the method comprising
- providing a cell expressing LSD1
- contacting the cell with a test compound
- determining the amount of LSD1 protein or LSD1 mRNA expressed by the cell
- selecting the test compound as a compound which inhibits adipose cell or tissue growth if the amount of LSD1 protein or LSD1 mRNA expressed by the cell is greater than that expressed by a control cell.

The invention further relates to a LSD1 modulator for use in the treatment and/or prevention of type 2 diabetes, obesity or an obesity-related disorder. The invention further relates to a method of treating type 2 diabetes, obesity or an obesity-related disorder, comprising administering to an individual in need thereof, a pharmaceutically effective amount of a LSD1 modulator. Obesity-related disorders include, but are not limited to, insulin resistance, hypertension, hyperuricemia, fatty liver, non-alcoholic fatty liver disease, polycystic ovarian syndrome, acanthosis nigricans, hyperphagia, endocrine abnormalities, triglyceride storage disease, Bardet-Biedl syndrome, and Lawrence-Moon syndrome. Preferably, the disorder is type II diabetes.

### LSD1 modulators

An LSD1 activator to be used in accordance with this invention is a compound capable of increasing the amount of LSD1 mRNA or LSD1 protein in a cell and/or activating at least one function or activity of the LSD1 gene or the LSD1 protein. These functions include (1) the ability of LSD1 to interact with androgen receptor,(2) the ability of LSD1 to assemble with other proteins, in particular transcription factors into protein complexes, and (3) the catalytic activity of LSD1. The protein complexes may be important for transcriptional activation.

An LSD1 inhibitor to be used in accordance with this invention is a compound capable of reducing the amount of LSD1 mRNA or LSD1 protein in a cell and/or inhibiting at least one function of the LSD1 gene or the LSD1 protein. These functions include (1) the ability of LSD1 to interact with androgen receptor,(2) the ability of LSD1 to assemble with other proteins, in particular transcription factors into protein complexes, and (3) the catalytic activity of LSD1. The protein complexes may be important for transcriptional activation.

The LSD1 modulator to be used in accordance with this invention may be a compound capable of activating or inhibiting expression of the LSD1 gene in a cell. Various methods for activating or inhibiting expression of genes in a cell are known to one of skill in the art (e.g. RNA interference or antisense technology).

In another embodiment the invention, the LSD1 modulator is capable of modulating the interaction of LSD1 protein with the androgen receptor. Such activators include "small molecules," also referred to herein as "compounds," which are isolated from natural sources or made synthetically. In general, such molecules may be identified from large libraries of natural products or synthetic (or semi-synthetic) extracts or chemical libraries according to methods known in the art. Those skilled in the field of drug discovery and development will understand that the precise source of test extracts or compounds is not critical to the methods of the invention. Accordingly, virtually any number of chemical extracts or compounds can be used in the methods described herein. The types of extracts or compounds that may be tested include plant, fungal, prokaryotic or eukaryotic cell or organism-based extracts, fermentation broths, and synthetic compounds including modifications of existing compounds. Numerous methods are also available for generating random or directed synthesis (e.g., semi-synthesis or total synthesis) of any number of chemical compounds, including, but not limited to, saccharides, lipids, peptides, polypeptides and nucleic acids and derivatives thereof. Synthetic compound libraries are commercially available.

In yet another embodiment, the LSD1 modulator may modulate (promote or inhibit) the stability the the LSD1 protein or Lsd1 mRNA.

In yet another embodiment, the LSD1 modulator may modulate (promote or inhibit) degradation of LSD1 protein or Lsd1 mRNA. For example, the pathways leading to degradation of LSD1 protein or Lsd1 mRNA in the cells may be inhibited, thus leading to an increased amount of LSD1 protein or Lsd1 mRNA in the cells.

In yet another embodiment, the LSD1 modulator may modulate (promote or inhibit) the translation efficiency of the Lsd1 gene.

Alternatively, libraries of natural compounds in the form of bacterial, fungal, plant, and animal extracts are commercially available from a number of sources. In addition, natural and synthetically produced libraries can be generated according to methods known in the art, e.g., by standard extraction and fractionation methods. Furthermore any library or compound may readily be modified using standard chemical, physical, or biochemical methods.

Another class of agents that can be screened are antibodies, in particular monoclonal antibodies.

The LSD1 activators are preferably useful in the treatment and/or prevention of type 2 diabetes, obesity and/or related disorders. LSD1 inhibitors may be useful in the treatment and/or prevention of metabolic disorders such as eating disorders, body weight disorders, cachexia, anorexia, sarcopenia and wasting syndrome or disease.

"Cachexia" refers to a state of general ill health and malnutrition. It is often associated with and induced by malignant cancer, and is characterized by loss of appetite, lass of body mass, especially lean body mass, and muscle wasting.

"Anorexia" refers simply to a loss of appetite, whether brought on by medical or psychological factors. Anorexia is often closely associated with, and generally contributes to, the cachexia seen in patients with advanced cancers.

### Modulators of target genes of LSD1

Target genes of LSD1 include, but are not limited to, *Nrf1, Prdm16* and *Pgc-1*α*.*

In one embodiment, the modulator of a target gene of LSD1 is an activator of the target gene.

In another embodiment, the modulator of a target gene of LSD1 is an inhibitor of the target gene.

In another embodiment, the modulator of a target gene of LSD1 is an activator of *Nrf1.*

In another embodiment, the modulator of a target gene of LSD1 is an activator of *Prdm16.*

In another embodiment, the modulator of a target gene of LSD1 is an activator of *Pgc-1*α. In another embodiment, the modulator of a target gene of LSD1 is an inhibitor of *Nrf1.*

In another embodiment, the modulator of a target gene of LSD1 is an inhibitor of *Prdm16.*

In another embodiment, the modulator of a target gene of LSD1 is an inhibitor of *Pgc-1*α.

Preferably, an activator is capable of increasing the expression of the target gene, or activating a function of the gene product of said target gene. Typically, an inhibitor is capable of inhibiting the expression of the target gene, or inhibiting a function of the gene product of said target gene.

The above-mentioned modulators are useful in the treatment and/or prevention of type 2 diabetes, obesity and/or related disorders. The above-mentioned modulators may further be useful in the treatment and/or prevention of a metabolic disorder.

One of skill in the art will appreciate that the LSD1 modulators can be used alone or in combination with other compounds and therapeutic regimens to inhibit obesity or obesity-related disorders or obesity-related symptoms, or metabolic disorders.

An effective amount of the activator will be determined by the existence, nature, and extent of any adverse side-effects that accompany the administration of the composition; the LD₅₀ of the composition; and the side-effects of the composition at various concentrations. Typically, the amount of the composition administered will range from about 0.01 to about 20 mg per kg, more typically about 0.05 to about 15 mg per kg, even more typically about 0.1 to about 10 mg per kg body weight.

The activator can be administered, for example, by intravenous infusion, orally, intraperitoneally, or subcutaneously. Oral administration is the preferred method of administration. The formulations of compounds can be presented in unit-dose or multidose sealed containers, such as ampoules and vials.

The LSD1 activators are typically formulated with a pharmaceutically acceptable carrier before administration to an individual or subject. Pharmaceutically acceptable carriers are determined, in part, by the particular composition being administered, as well as by the particular method used to administer the composition. Accordingly, there are a wide variety of suitable formulations of pharmaceutical compositions of the present invention (see, e.g., Remington's Pharmaceutical Sciences, 17th ed., 1989).

### DESCRIPTION OF THE DRAWINGS

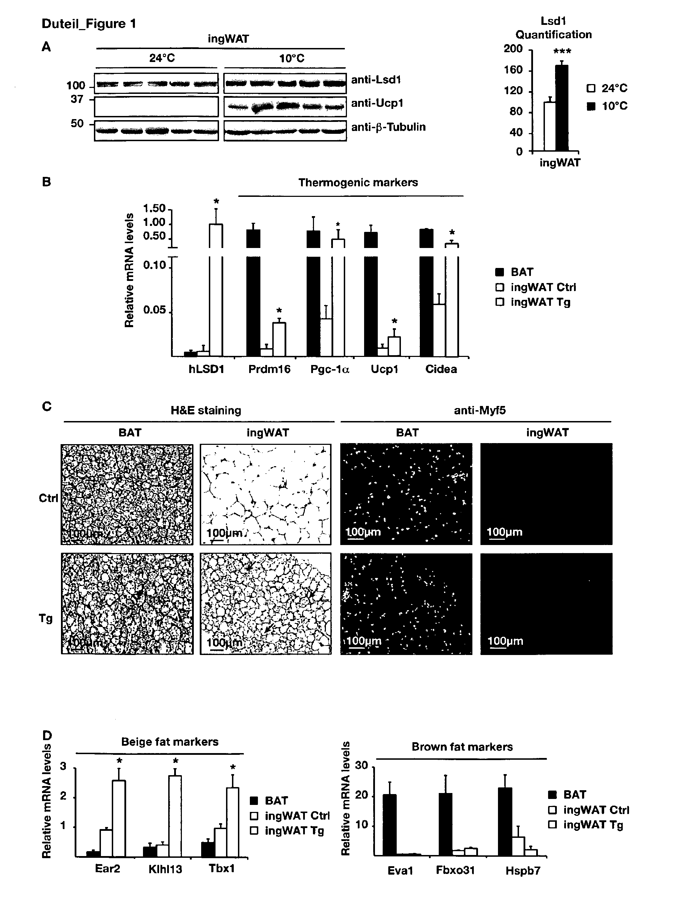

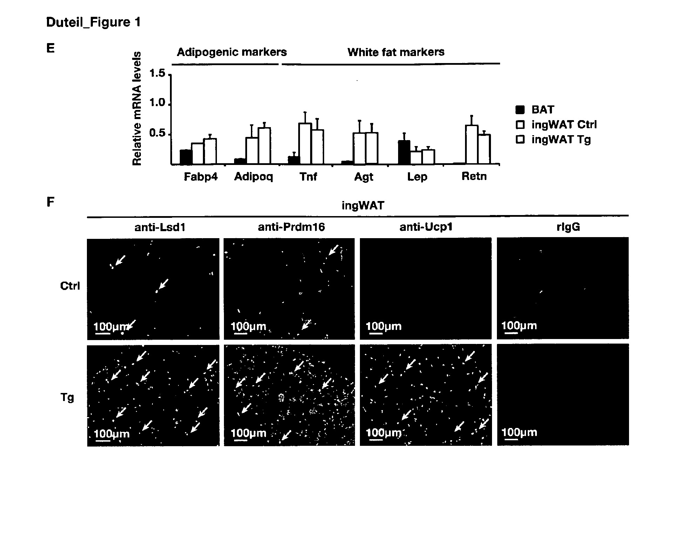

**Figure 1****. Overexpression of LSD1 in transgenic mice promotes brown fat formation in mouse white adipose tissue.**
   (A). Western blot analysis (left panel) and quantification (right panel) of Lsd1 and Ucp1 protein levels in inguinal white adipose tissue (ingWAT) of C57/Bl6 mice maintain during 10 days at either 24 or 10 °C. β-Tubulin has been used as a loading control.
   (B). Relative transcript levels of human (*h*)*LSD1, Prdm16, Pgc-1*α*, Ucp1* and *Cidea* in ingWAT of Ctrl and Tg mice. Interscapular brown adipose tissue (iBAT) of Ctrl mice was used as a positive control for brown fat markers expression.
   (C). Representative hematoxylin and eosin staining and immunohistochemical analysis of Myf5 expression (yellow) in iBAT and ingWAT of Ctrl and Tg mice. Rabbit IgG has been used as a negative control.
   (D). Relative transcript levels of the beige fat markers *Ear2, KIhl13,* and *Tbx1,* and of the brown fat markers *Eva1, Fbxo31,* and *Hspb7* in ingWAT of Ctrl and Tg mice. Interscapular brown adipose tissue (iBAT) of Ctrl mice was used as a positive control for brown fat markers expression.
   (E). Immunohistochemical analysis of Lsd1, Prdm16 and Ucp1 (yellow) expression in ingWAT of Ctrl and Tg mice. Rabbit IgG has been used as a negative control.
   (F). Ultrastructure of iBAT and ingWAT of Ctrl and Tg mice. Arrowheads point to some mitochondria.
   (G). Body weight of Ctrl and Tg mice fed a high fat diet.
   (H). Representative hematoxylin and eosin staining of ingWAT of Ctrl and Tg mice fed a high fat diet.
   (I). Oximax analysis of Ctrl and Tg mice fed a high fat diet.
      For (A) to (E), and (G) to (I) n=10; for (F) n = 3; standard deviation represents + SEM, *: p<0.05, **: p<0.01, ***: p<0.001.

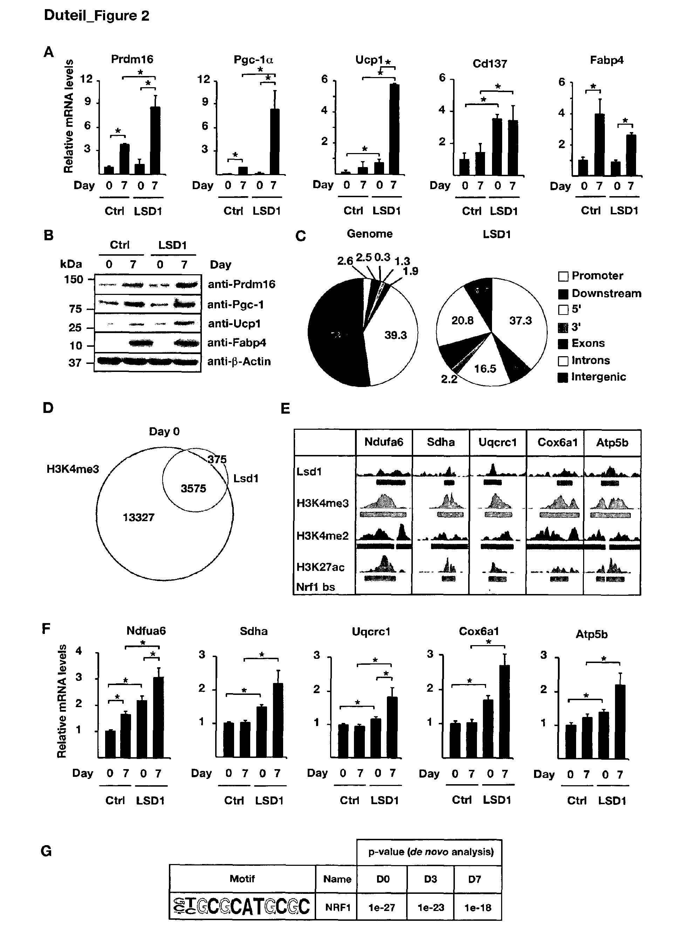

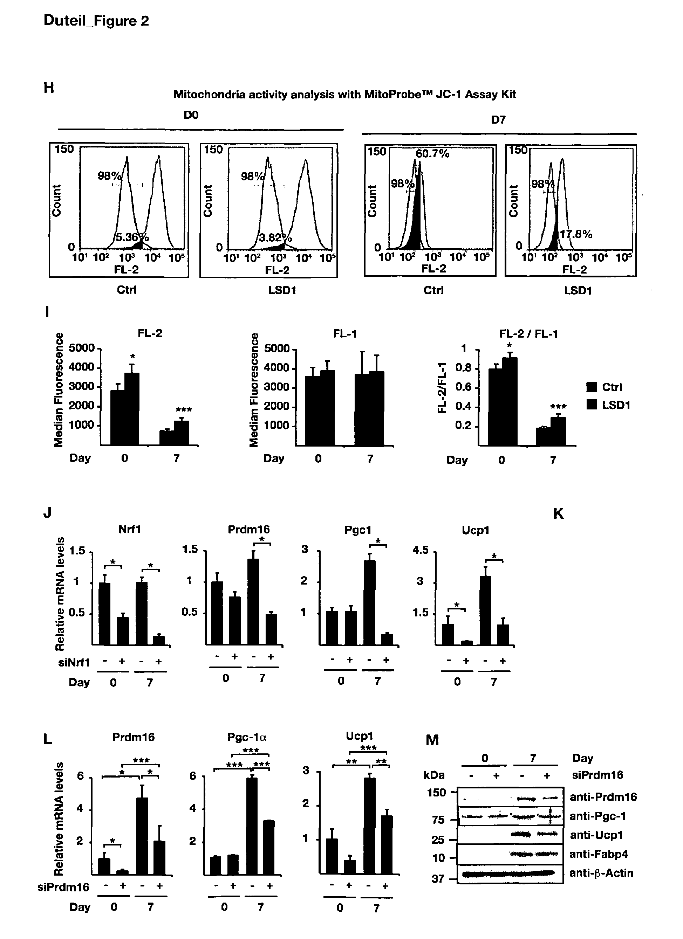

**Figure 2****. Lsd1 promotes beige fat program through Nrf1 in 3T3-L1 cells.**
   (A). Relative transcript levels of *Prdm16, Pgc-1*α*, Ucp1, Cd137,* and *Fabp4* in undifferentiated (day 0) and differentiated (day 7) LSD1 overexpressing (3T3-L1_LSD1) and control (Ctrl) 3T3-L1 cells.
   (B). Western blot analysis of Prdm16, Pgc-1, Ucp1, and Fabp4 protein levels in day 0 and day 7 3T3-L1_LSD1 and Ctrl cells. β-Actin has been used as a loading control.
   (C). Pie graph displaying genomic distribution of LSD1 enriched regions identified in 3T3-L1 cells. The genomic features (exons, introns, and intergenic regions) were defined regarding known genes of Refseq calculated with cis-regulatory element annotation system (CEAS) (Ji et al., 2006).
   (D). Venn diagram showing overlap of the ChIP-Seq of Lsd1 and H3K4me3 regions in 3T3-L1 cells.
   (E). Representative genes co-occupied by Lsd1 peaks in the promoter region. ChIP-Seq showing LSD1 and methylmark co-occurrence to the regulatory regions of the β-oxidation complex I-V genes *(Ndfua6, Sdha, Uqcrc1, Cox6a1,* and *AtpSb,* respectively). A view of the signals obtained by ChIP-seq for LSD1 and histone methylation profiles at the analyzed binding sites is shown.
   (F). Relative transcript levels of *Ndfua6, Sdha, Uqcrc1, Cox6a1,* and *Atp5b* in day 0 and day 7 3T3-L1_LSD1 and Ctrl cells.
   (G). Sequence logos showing the consensus sequences of the enriched NRF1 motif identified by motif analysis of LSD1 binding sites in 3T3-L1 cells. NRF1 motifs common at promoters of genes were identified by comparing LSD1 peaks to randomly selected genomic regions.
   (H)-(I). Mitochondrial energy metabolism assessed in day 0 and day 7 3T3-L1_LSD1 and Ctrl cells. Cells were stained with JC-1 followed by flow cytometric analyses. (H) Red fluorescent JC-1 aggregates (FL2) and green fluorescent monomers (FL1) were measured. Hatched histograms indicate the unstained control samples. (I) Each histogram shows the representative result of triplicate samples. At day 7, cells have been sorted into 4 subsets according to their size and to their granulosity. The histogram shows the average of the 4 subsets. Each histogram shows the representative result of triplicate samples.
   (J). Relative transcript levels of *Nrf1, Prdm16, Pgc-1*α*,* and *Ucp1* in day 0 and day 7 3T3-L1_LSD1, treated either siRNA control or directed against Nrf1.
   (K). Western blot analysis of Nrf1, Prdm16, Ucp1, and Fabp4 protein levels in day 0 and day 7 3T3-L1_LSD1, treated either siRNA control or directed against Nrf1. β-Actin has been used as a loading control.
   (L). Relative transcript levels of *Prdm16, Pgc-1*α*,* and *Ucp1* in day 0 and day 7 3T3-L1_LSD1, treated either siRNA control or directed against Prdm16.
   (M). Western blot analysis of Prdm16, Ucp1, and Fabp4 protein levels in day 0 and day 7 3T3-L1_LSD1, treated either siRNA control or directed against Prdm16. β-Actin has been used as a loading control.
      For (A) to (B), (F) and (I) to (M) n=6; standard deviation represents + SEM, *: p<0.05, **: p<0.01, ***: p<0.001.

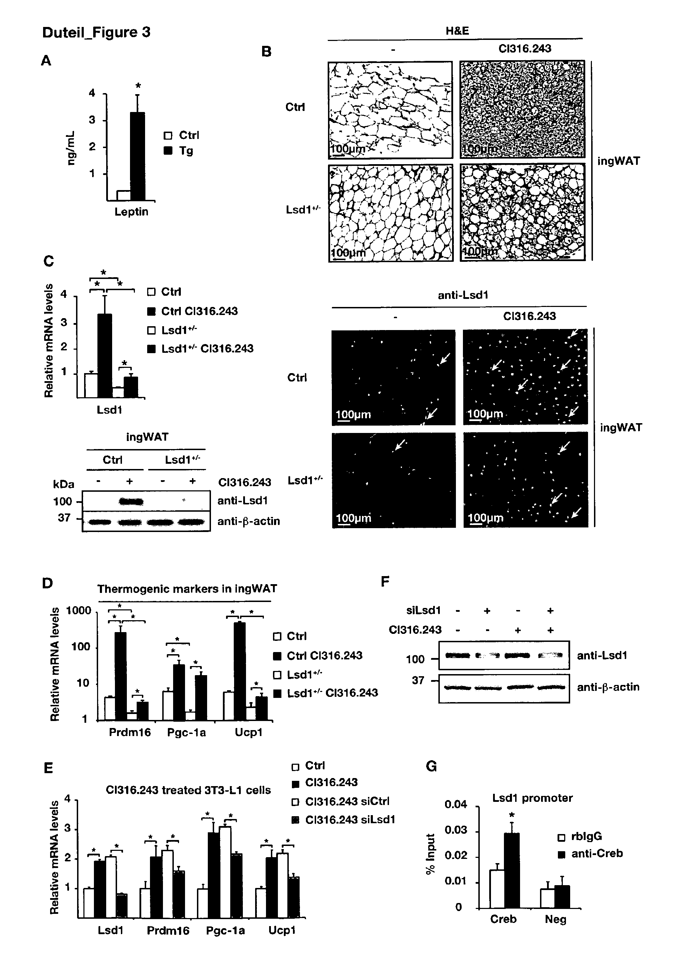

**Figure 3****. β3-adrenergic-signalling regulates Lsd1 levels to prevent obesity-related disorders.**
   (A). Serum leptin levels of Ctrl and Tg mice.
   (B). Representative hematoxylin and eosin staining and immunohistochemical analysis of Lsd1 (yellow) in ingWAT of control (Ctrl) and heterozygous Lsd1 knock-out (Lsd1^{+/-}) mice treated with CI316,243 or vehicle. Rabbit IgG has been used as a negative control.
   (C). Lsd1 levels are increased upon C1316,243 treatment. (up left panel) Relative *Lsd1* transcript levels in ingWAT of Ctrl and Lsd1^{+/-} mice treated with C1316,243 or vehicle. (low left panel) Western blot analysis of Lsd1 protein levels in ingWAT of control (Lsd1^{+/+}) and heterozygous Lsd1 knock-out (Lsd1⁺¹⁻) mice treated with CI316,243 or vehicle. (right) Representative hematoxylin and eosin staining and immunohistochemical analysis of Lsd1 (yellow) in ingWAT of control (Ctrl) and heterozygous Lsd1 knock-out (Lsd1 ^{+/-}) mice treated with CI316,243 or vehicle. Rabbit IgG has been used as a negative control.
   (D). Relative *Prdm16, Pgc-1*α*,* and *Ucp1* transcript levels in ingWAT of Ctrl and Lsd1^{+/-} mice treated with C1316,243 or vehicle.
   (E). Relative transcript levels of *Lsd1, Prdm16, Pgc-1*α*,* and *Ucp1* in vehicle or C1316,243 treated differentiated (day 7) 3T3-L1 cells, transfected with either a siRNA control or directed against Lsd1.
   (F). Western blot analysis of Lsd1 protein levels in vehicle or C1316,243 treated differentiated (day 7) 3T3-L1 cells, transfected with either a siRNA control or directed against Lsd1. β-Actin has been used as a loading control.
   (G). Quantitative PCR detection of the DNA segments encompassing a putative Creb response element located on the Lsd1 promoter after chromatin immunoprecipitation from vehicle or C1316,243 treated 3T3-L1 pre-adipocytes with antibody directed against Creb or with the corresponding rabbit IgG. Data are expressed as a percentage relative to the input DNA.
      For (A) n=10; (B) to (D) n=5; for (E) to (G) n=6; standard deviation represents + SEM, *: p<0.05, **: p<0.01, ***: p<0.001.

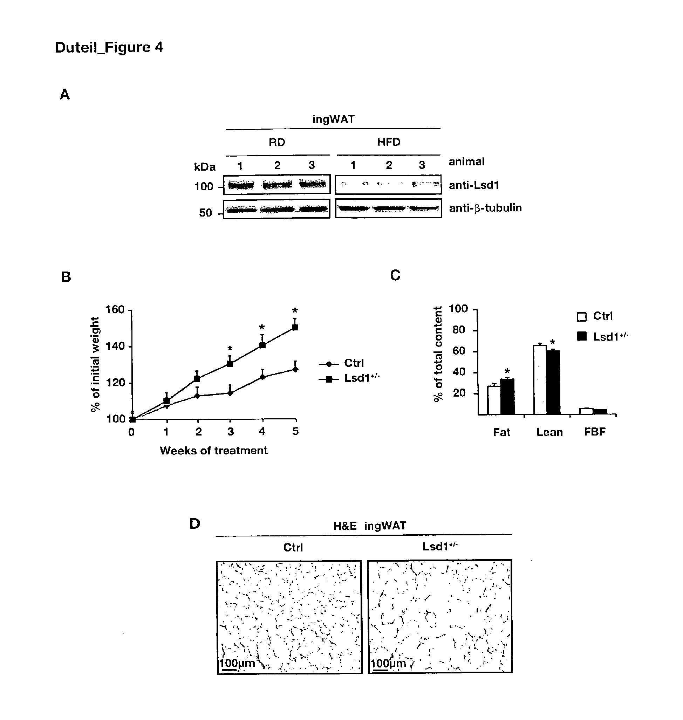

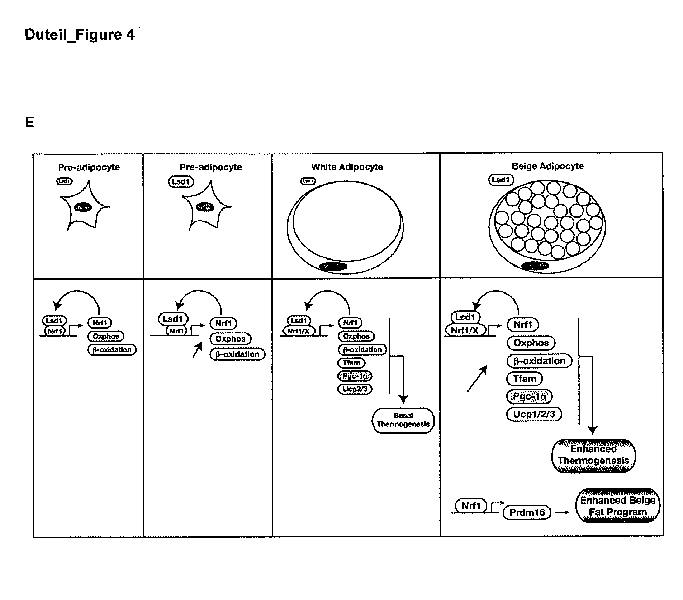

**Figure 4****. Lsd1^{+/-} mice are prone to obesity and type 2 diabetes.**
   (A). Lsd1 levels are decreased upon high fat diet treatment. Western blot analysis of Lsd1 protein levels in ingWAT of wild type mice fed with a high fat or a regular chow diet.
   (B). Body weight of Ctrl and Lsd1^{+/-} mice fed a high caloric diet. The arrow indicates the start of the high fat diet.
   (C). Lean and fat content of Ctrl and Lsd1^{+/-} mice fed a high fat diet analyzed by QNMR.
   (D). Representative hematoxylin and eosin staining of ingWAT fat pad of Ctrl and Lsd1^{+/-} mice fed a high fat diet.
   (E). Schematic illustration of the putative role of Lsd1 in white adipocytes.
      For (A) to (D) n=6; standard deviation represents + SEM, *: p<0.05, **: p<0.01, ***: p<0.001.

### Supplementary tables

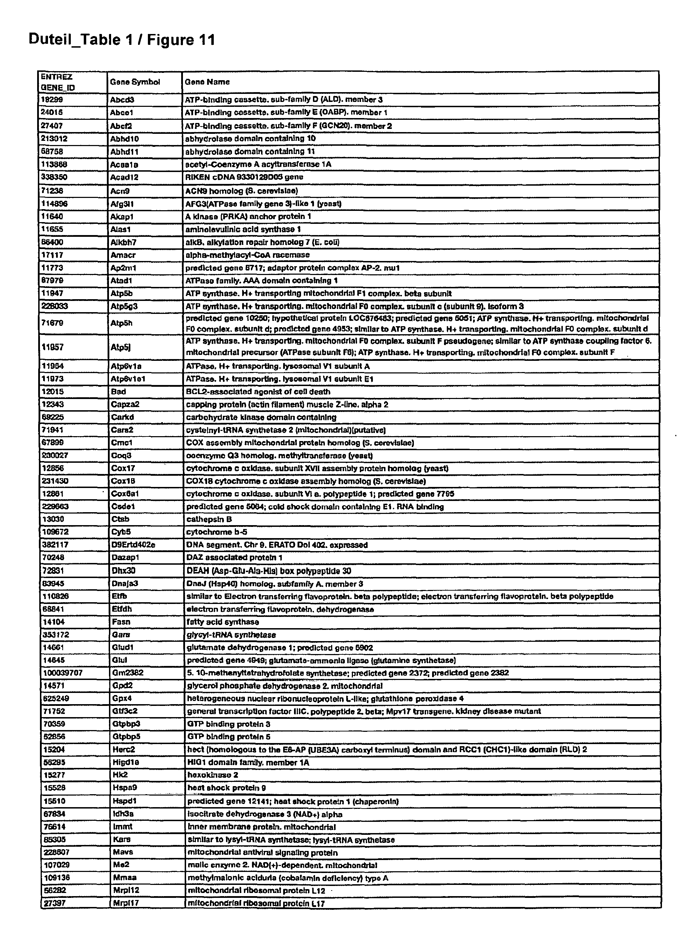

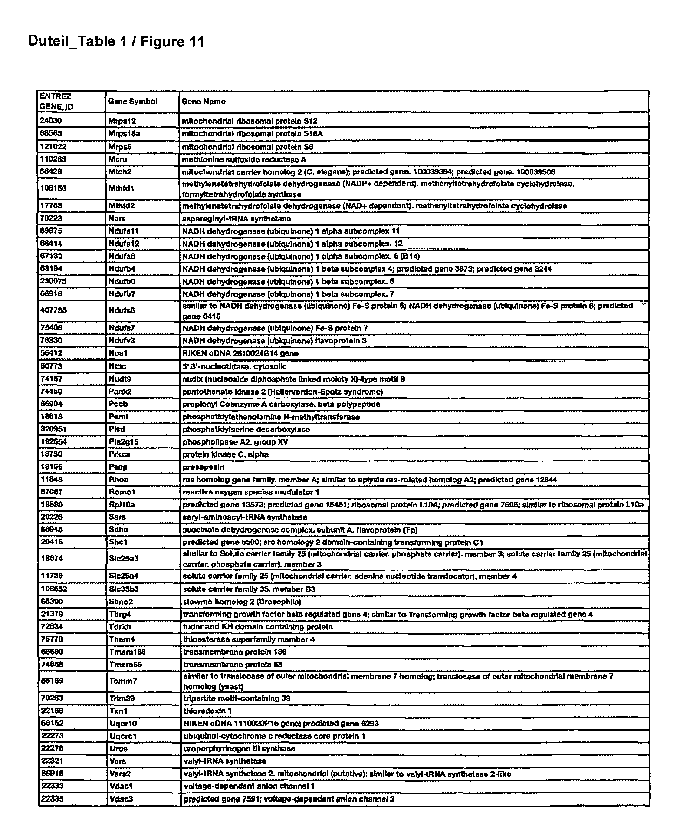

**Figure 11****: Table1 (Relative to** **Figure 2****).** Representative fraction of Lsd1 and Nrf1 co-target genes DAVID analysis showing genes involved in oxidative phosphorylation at D0. Genes encoding for mitochondria transport chain are highlighted in yellow.

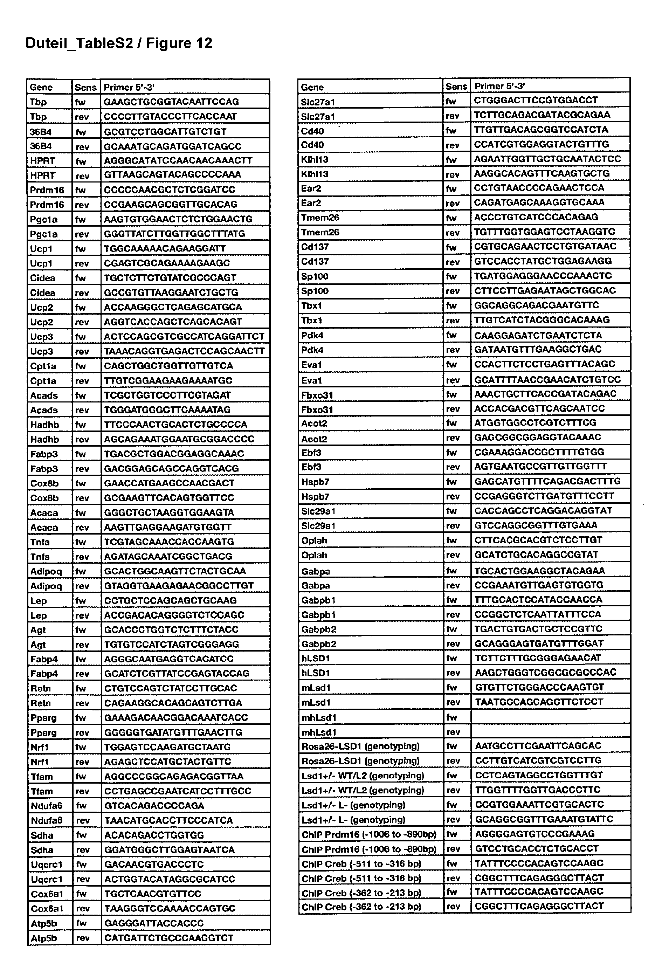

**Figure 12****: Table2. List of the oligonucleotides used for qPCR and PCR analyses.**

### Supplementary figures

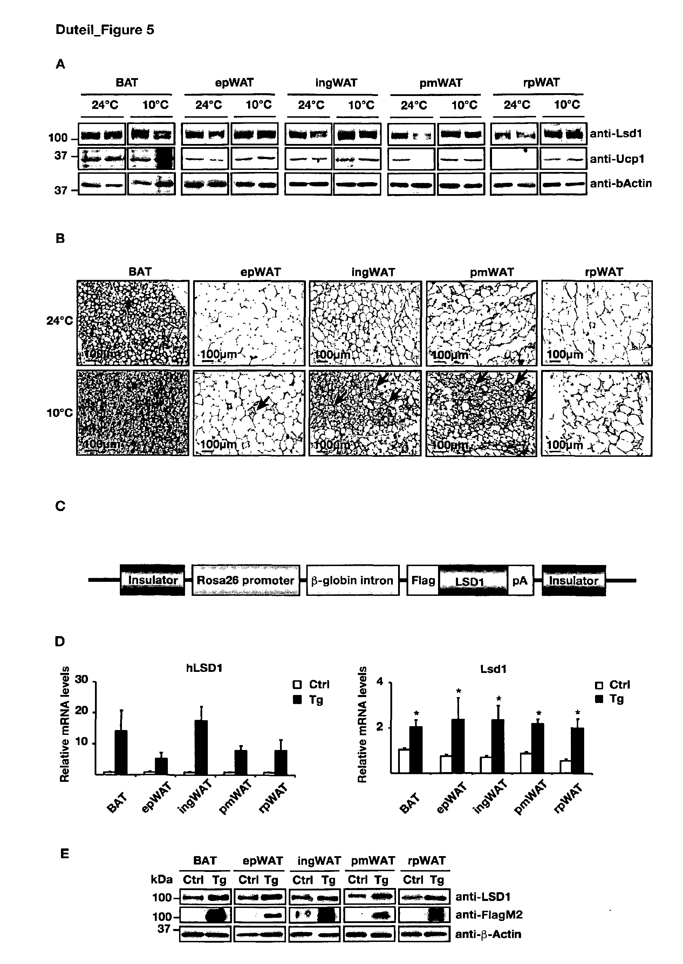

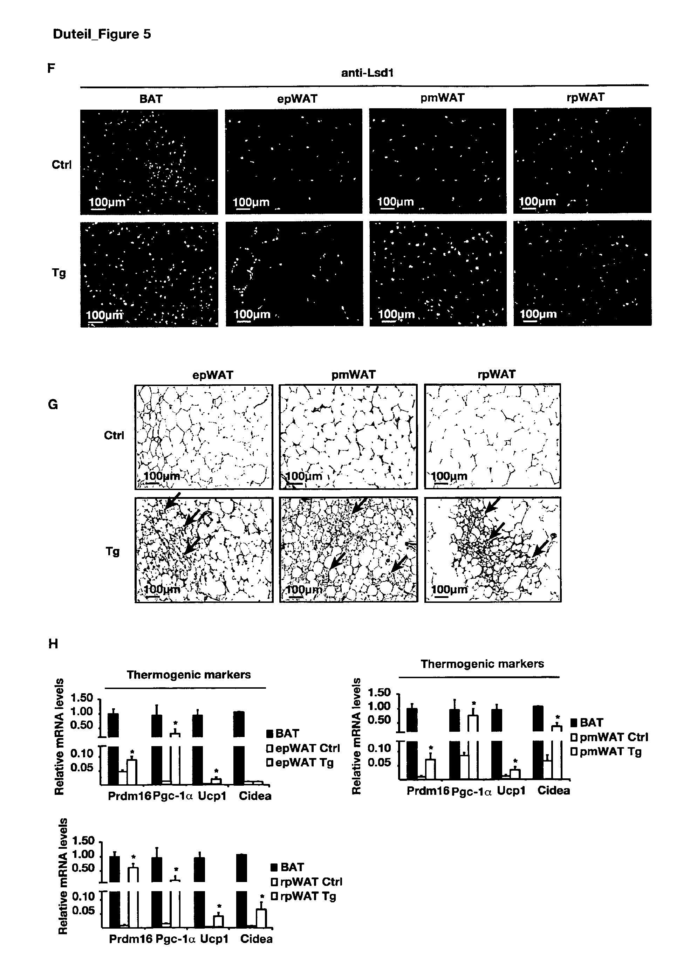

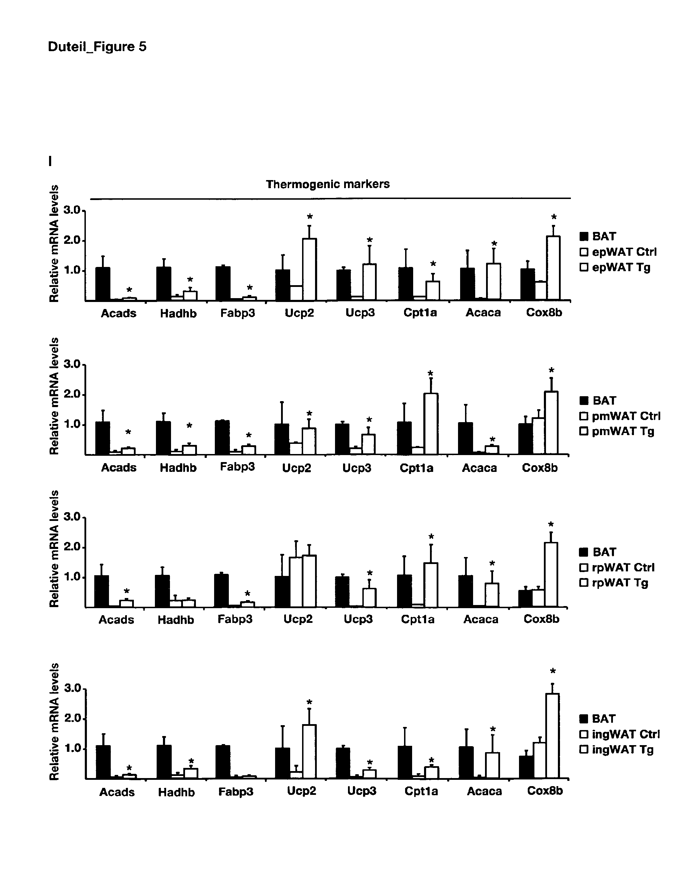

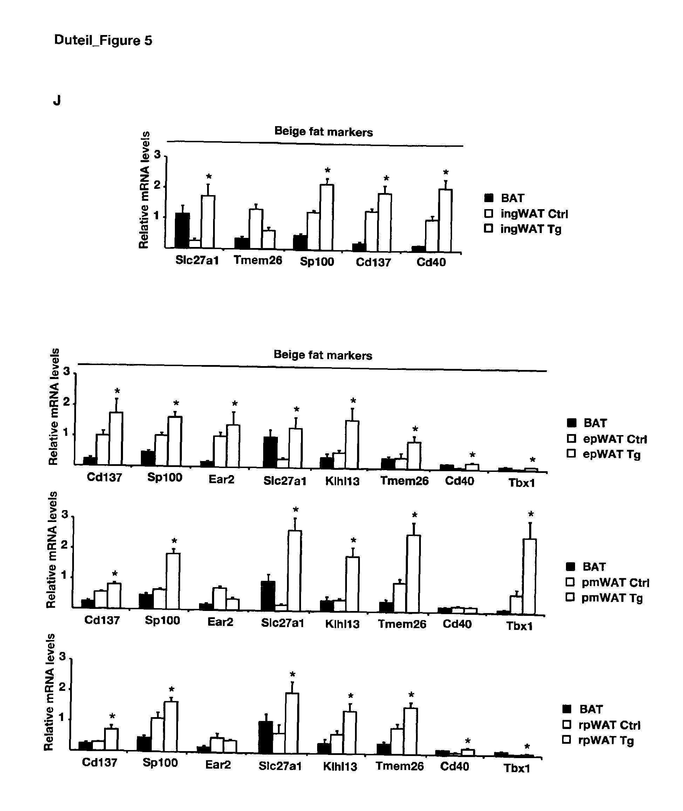

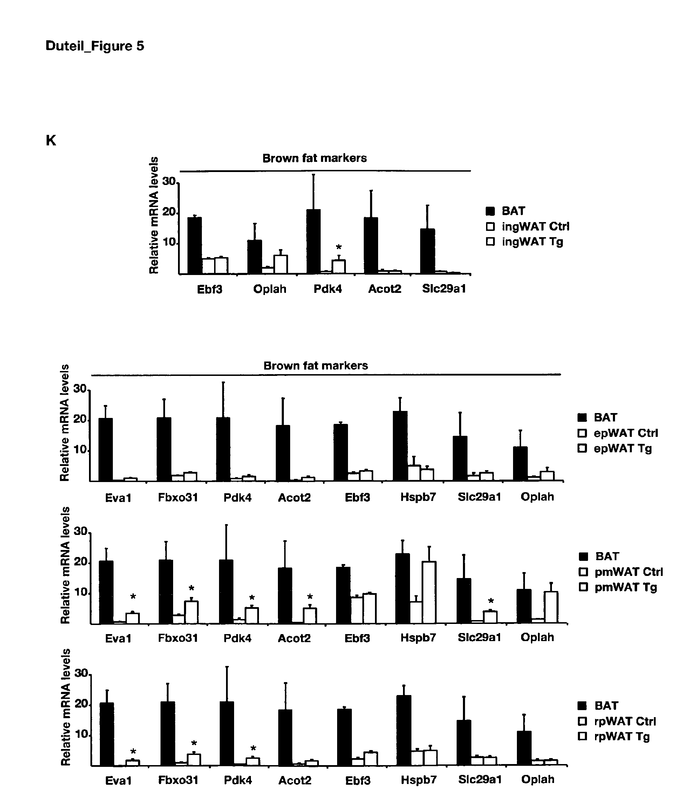

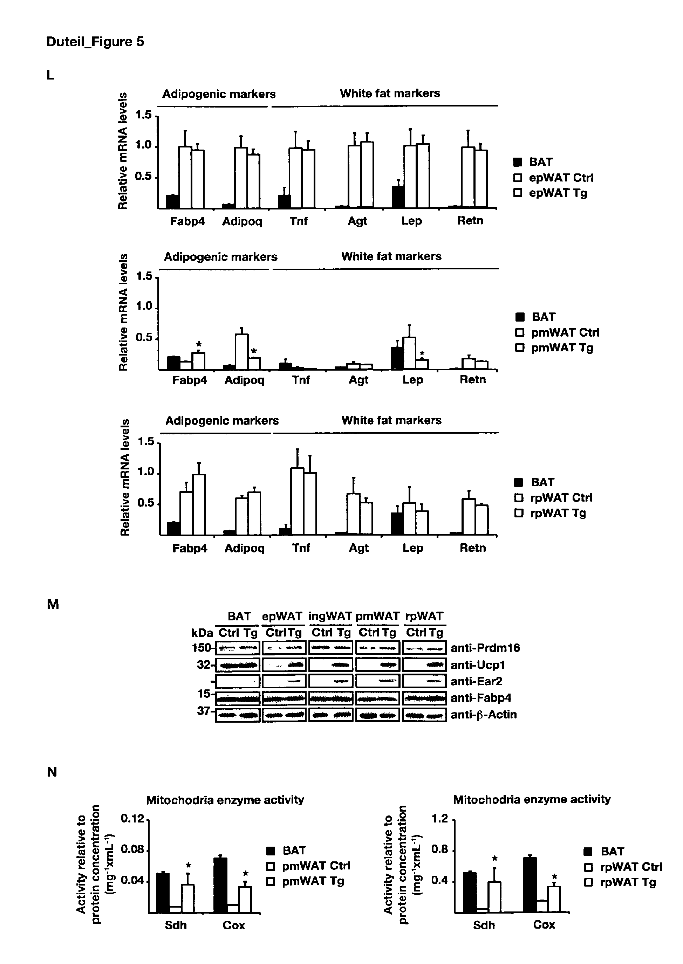

**Figure 5** **(relative to** **Figure 1****). Overexpression of LSD1 in transgenic mice promotes brown fat formation in mouse white adipose tissue.**
   (A). Western blot analysis (left panel) and quantification (right panel) of Lsd1 and Ucp1 protein levels in intrascapular brown fat (iBAT), epididymal (epWAT), inguinal white adipose tissue (ingWAT), perimuscular (pmWAT), and retroperitoneal (rpWAT) white fat pads of C57/Bl6 mice maintain during 10 days at either 24 or 10 °C. β-Tubulin has been used as a loading control.
   (B). Representative hematoxylin and eosin staining and immunohistochemical analysis of ingWAT of C57/Bl6 mice maintain during 10 days at either 24 or 10 °C.
   (C). Schematic representation of the Rosa26-LSD1 transgene.
   (D). qRT-PCR analyses of human (h)LSD1 (left panel) and both human and mouse Lsd1 levels (right panel) in iBAT, epWAT, ingWAT, pmWAT and rpWAT white fat pads of Ctrl and Tg mice to determine the presence of the transgene and the overexpression rate.
   (E). Western blot analyses of LSD1 levels in iBAT, epWAT, ingWAT, pmWAT and rpWAT of Ctrl and Tg mice. FlagM2 was used to show the presence of the transgene, and β-actin was used as a loading control.
   (F). lmmunohistochemical analysis of Lsd1 expression (green) in iBAT, epWAT, ingWAT, pmWAT and rpWAT of control (Ctrl) and LSD1 overexpressing (Tg) mice. Rabbit IgG has been used as a negative control.
   (G). Representative hematoxylin and eosin staining of epWAT, pmWAT and rpWAT of Ctrl and Tg mice.
   (H-I). Relative transcript levels of (H) *Prdm16, Pgc-1*α*, Ucp1,* and Cidea in epWAT, pmWAT and rpWAT of Ctrl and Tg mice, and (I) *Acads, Hadhb, Fabp3, Ucp2, Ucp3, Cpt1*α*,* Acaca, and *Cox8b* in epWAT, ingWAT, pmWAT and rpWAT of Ctrl and Tg mice. IBAT of Ctrl mice was used as a positive control for brown fat markers expression.
   (J). Relative transcript levels of the beige fat markers *Cd137, Sp100, Ear2, Slc27a1, K*/*h*/*13, Tmem26, Cd40,* and *Tbx1,* in epWAT, ingWAT, pmWAT and rpWAT of Ctrl and Tg mice. Interscapular brown adipose tissue (iBAT) of Ctrl mice was used as a control.
   (K). Relative transcript levels of the brown fat markers *Eva1, Fbxo31, Pdk4, Acot2, Ebf3, Hspb7, SIc29a1,* and *Oplah* in epWAT, ingWAT, pmWAT and rpWAT of Ctrl and Tg mice. Interscapular brown adipose tissue (iBAT) of Ctrl mice was used as a positive control for brown fat markers expression.
   (L). Relative transcript levels of adipogenic markers *Fabp4* and *Adipoq,* and of white fat markers *Tnf, Agt, Lep,* and *Retn* in epWAT, ingWAT, pmWAT and rpWAT of Ctrl and Tg mice. Interscapular brown adipose tissue (iBAT) of Ctrl mice was used as a control.
   (M). Western blot analysis of Prdm16, Ucp1, Ear2 and Fabp4 protein levels in Ctrl and Tg mice. β-Actin has been used as a loading control.
   (N). Biochemical determination of Sdh and Cox activities in pmWAT and rpWAT of Ctrl and Tg mice. Interscapular brown adipose tissue (iBAT) of Ctrl mice was used as a positive control.
      For (A) and (B), (D) to (N) n=10; standard deviation represents + SEM, *: p<0.05, **: p<0.01, ***: p<0.001.

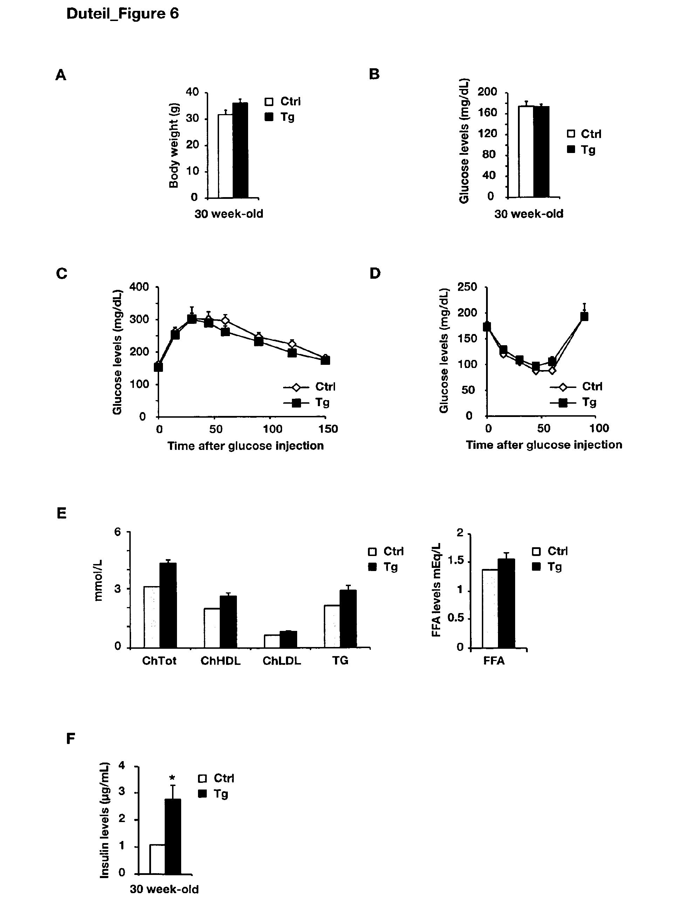

**Figure 6** **(relative to** **Figure 1****). Overexpression of LSD1 in transgenic mice does not affect metabolic profile upon regular diet.**
   (A). Body weight of 30-week-old Ctrl and Tg mice.
   (B). Serum glucose levels in 30-week-old Ctrl and Tg mice.
   (C)-(D). Intraperitoneal glucose tolerance test (IPGTT) (C) and intraperitoneal insulin sensitive test (IPIST) (D) in 30-week-old Ctrl and Tg mice.
   (E). Serum cholesterol (total: T. Chol., HDL: HDL Chol. and LDL: LDL Chol.), triglyceride (TG) and free fatty acid (FFA) levels in 30-week-old Ctrl and Tg mice.
   (F). Serum insulin levels in 30-week-old Ctrl and Tg mice.
      For (A) to (F), n=10; standard deviation represents + SEM, *: p<0.05, **: p<0.01, ***: p<0.001.

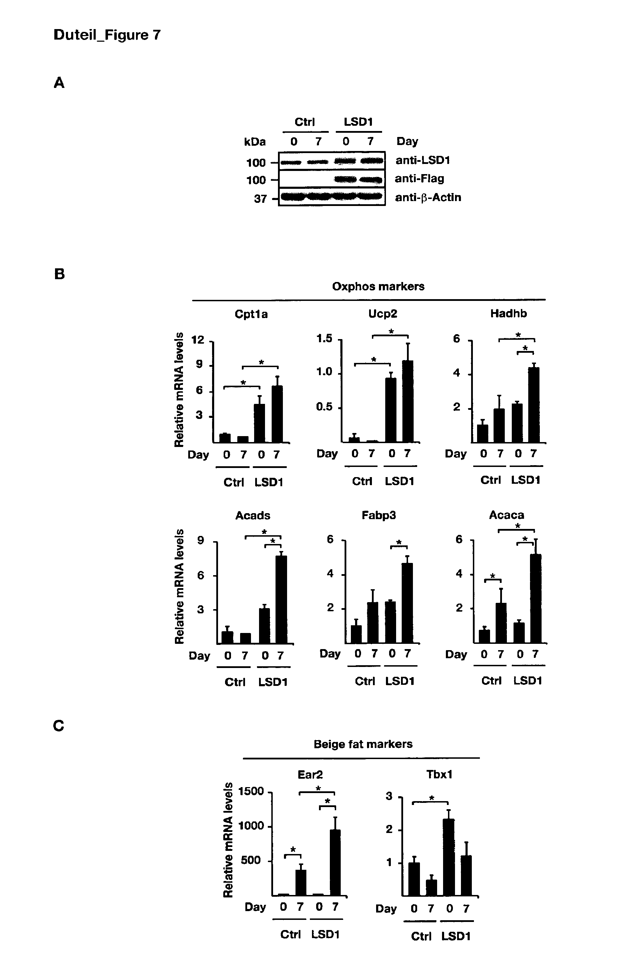

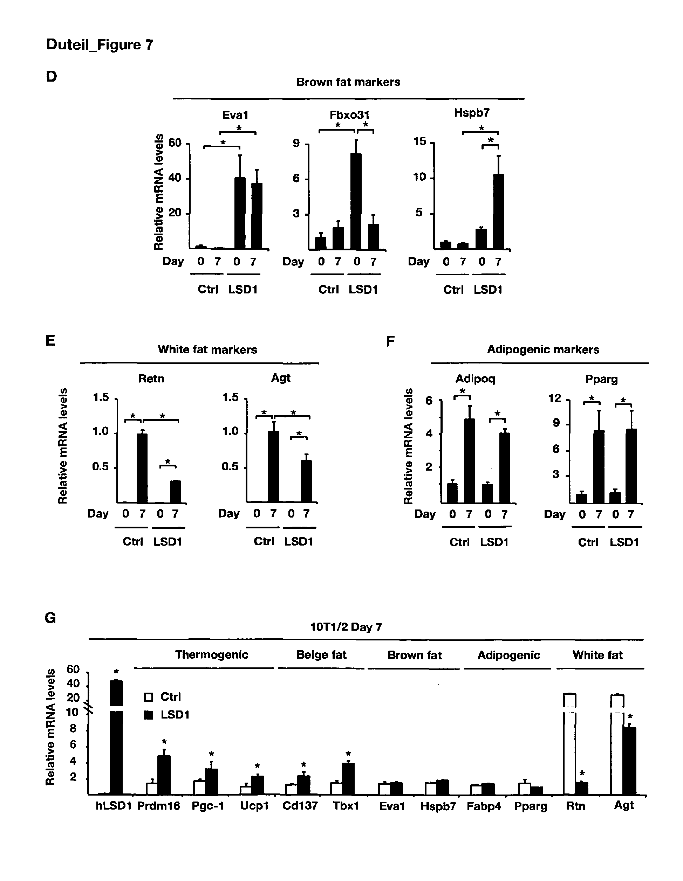

**Figure 7** **(relative to** **Figure 2****). Lsd1 promotes brown fat program in 3T3-L1 and 10T1/2 cells.**
   (A). Western blot analysis of LSD1 and FIagM2 protein levels in day 0 and day 7 3T3-L1_LSD1 and Ctrl cells. β-Actin has been used as a loading control.
   (B). Relative transcript levels of the Oxphos makers *Cpt1a, Ucp2, Hadhb, Acads, Ucp3, Fabp3, Cox8b, Cidea,* and Acaca in day 0 and day 7 3T3-L1_LSD1 and 3T3-L1(Ctrl) cells.
   (C). Relative transcript levels of the beige fat makers *Ear2* and *Tbx1* in day 0 and day 7 3T3-L1_LSD1 and Ctrl cells.
   (D). Relative transcript levels of the brown fat makers *Eva1, Fbxo31,* and *Hspb7* in day 0 and day 7 3T3-L1_LSD1 and Ctrl cells.
   (E). Relative transcript levels of the white fat markers *Retn* and *Agt* in day 0 and day 7 3T3-L1_LSD1 and Ctrl cells.
   (F). Relative transcript levels of the adipogenic markers *Adipoq* and *Pparg* in day 0 and day 7 3T3-L1_LSD1 and Ctrl cells.
   (G). Relative transcript levels of *hLSD1,* and the Oxphos markers *Prdm16, Pgc-1a, Ucp1,* and *Cpt1a,* of the beige fat makers *Cd137* and *Tbx1,* of the brown fat makers *Eva1,* and *Hspb7,* of the adipogenic marker *Fabp4,* and of the white fat markers *Retn* and *Agt* in day 7 10T1/2_LSD1 and Ctrl cells.
      For (A) to (G) n=6; standard deviation represents + SEM, *: p<0.05, **: p<0.01, ***: p<0.001.

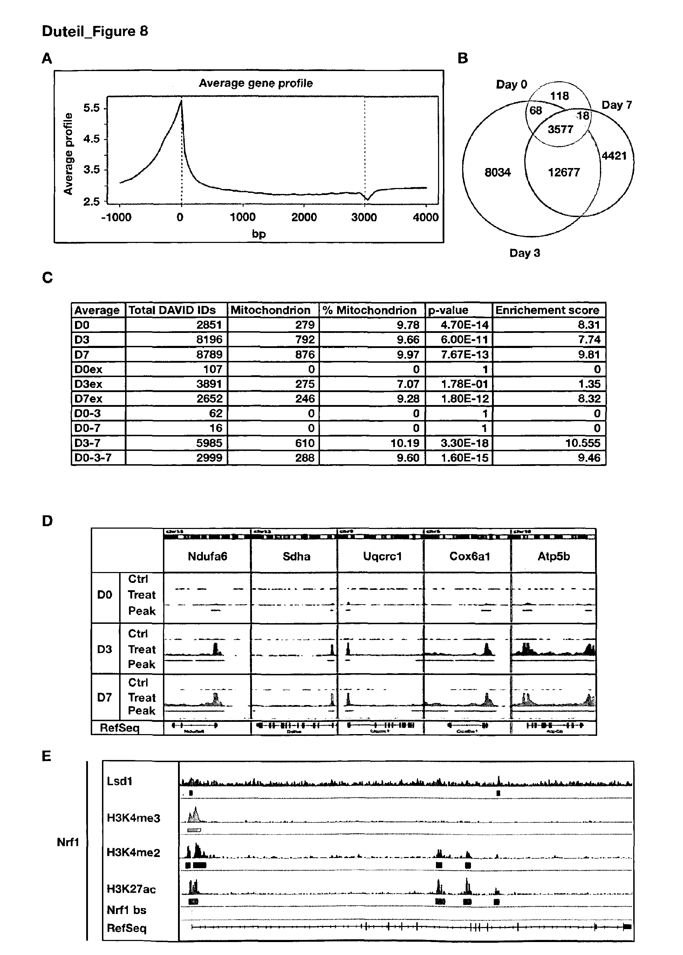

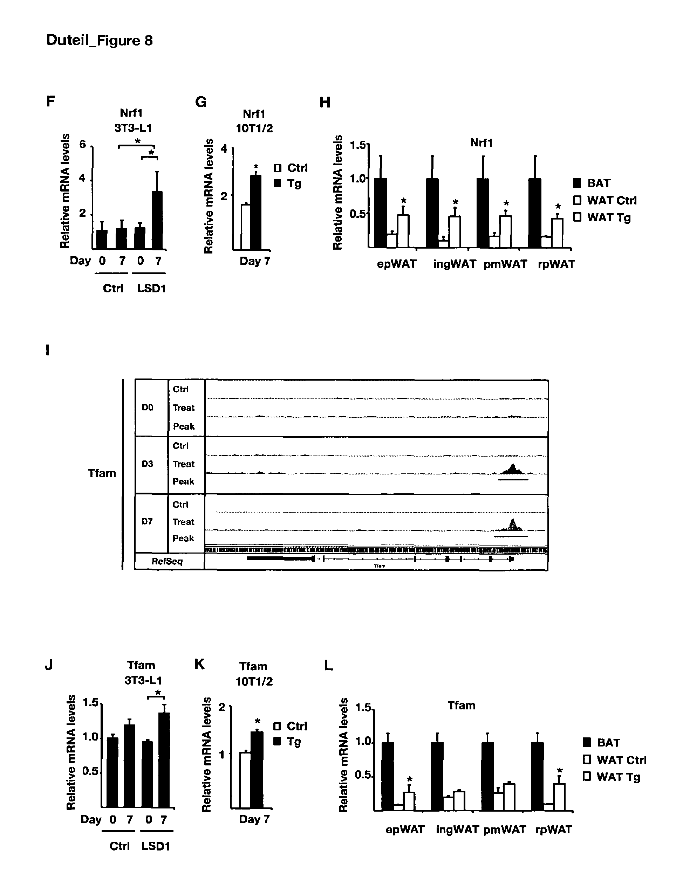

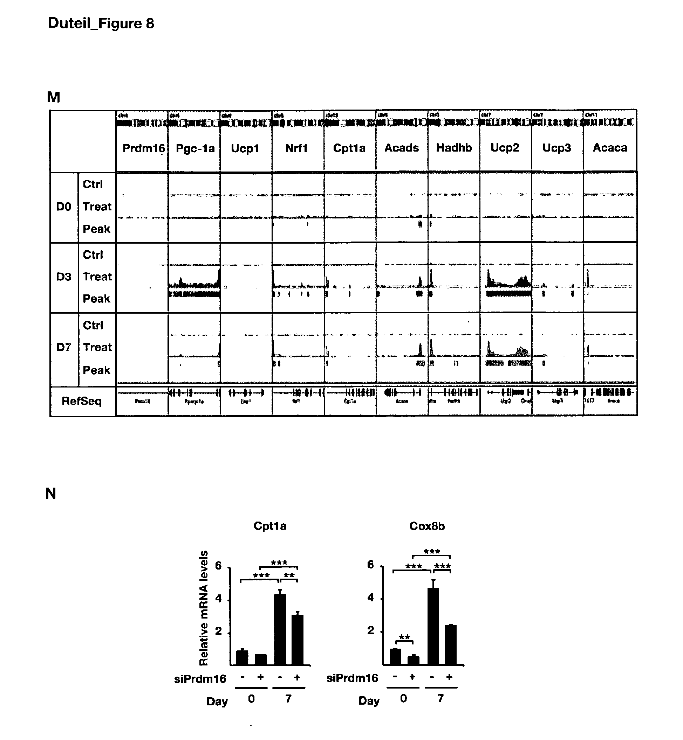

**Figure 8** **(relative to** **Figure 2****). Lsd1 promotes brown fat program through Prdm16 in 3T3-L1 cells.**
   (A). LSD1 profile across an average gene body.
   (B). Venn diagram showing overlap of the ChIP-Seq of Lsd1 regions in 3T3-L1 cells at D0, D3 and D7.
   (C). Table representing the occurrence of the mitochondrion gene clusters at D0, D3, D7, the genes obtained at D0 that do not overlap with the genes obtained at D3 and 7 (D0ex), the genes obtained at D3 that do not overlap with the genes obtained at D0 and 7 (D3ex), the genes obtained at D7 that do not overlap with the genes obtained at D0 and 3 (D7ex), and the genes that overlap between D0 and D3 (D0-3), D0 and D7 (D0-7), D3 and D7 (D3-7), and D0, D3 and D7 (D0-3-7).
   (D). Representative genes occupied by Lsd1 peaks in the promoter region. ChlP-Seq showing LSD1 occurrence to the regulatory regions of the ß-oxidation complex I-V genes *(Ndfua6, Sdha, Uqcrc1, Cox6a1,* and *Atp5b,* respectively). A view of the signals obtained by ChIP-seq for LSD1 and histone methylation profiles at the analyzed binding sites is shown.
   (E). Browser images of the *Nrf1* gene depicting coordinate binding of Lsd1 together with the associated H3K4me3, H3K4me2, and H3K27ac signatures, as well as Nrf1 binding sites.
   (F-H). Relative transcript levels of *Nrf1* in day 0 and day 7 3T3-L1_LSD1 and 3T3-L1(Ctrl) cells (F), in day 7 10T1/2(LSD1) and 10T1/2(Ctrl) cells (G), and in epWAT, ingWAT, pmWAT and rpWAT of Ctrl and Tg mice. iBAT is used as a positive control for expression of brown fat markers (H).
   (I). Browser images of the *Tfam* gene depicting binding of Lsd1 at D0, D3 and D7.
   (J-L). Relative transcript levels of *Tfam* in day 0 and day 7 3T3-L1_LSD1 and 3T3-L1(Ctrl) cells (J), in day 7 10T1/2(LSD1) and 10T1/2(Ctrl) cells (K), and in epWAT, ingWAT, pmWAT and rpWAT of Ctrl and Tg mice. iBAT is used as a positive control for expression of brown fat markers (L). (M). Representative thermogenic genes occupied by LSD1 in the promoter region at D0, D3 and D7.
   (N). Relative transcript levels of *Cpt1a,* and *Cox8b* in day 0 and day 7 3T3-L1_LSD1 cells, treated either siRNA control or directed against Prdm16.
      For (H) and (L) n=10; (F), (G), (J), (K) and (N) n=6; standard deviation represents + SEM, *: p<0.05, **: p<0.01, ***: p<0.001.

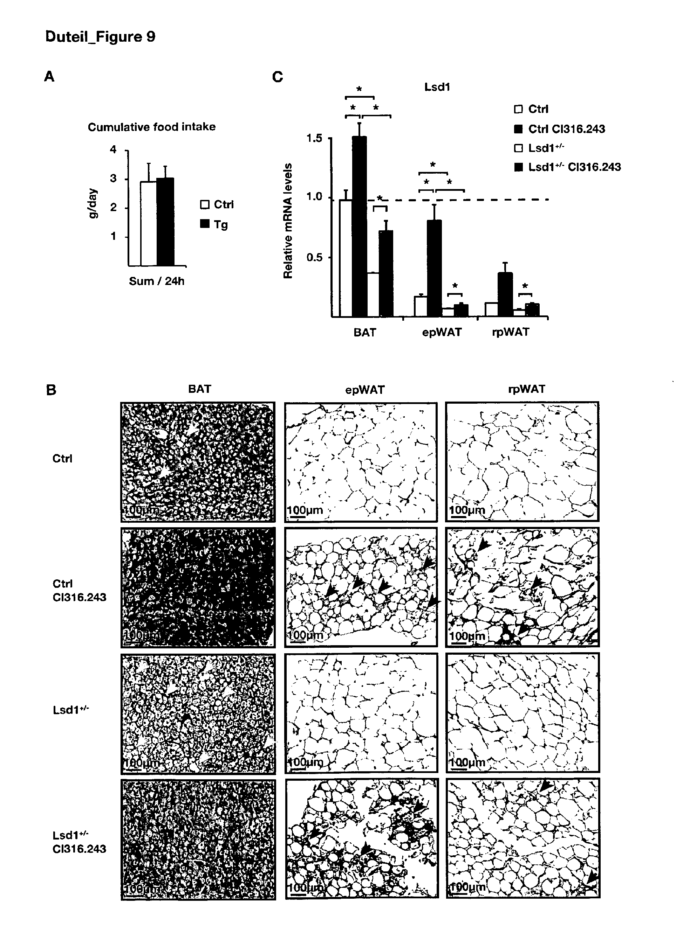

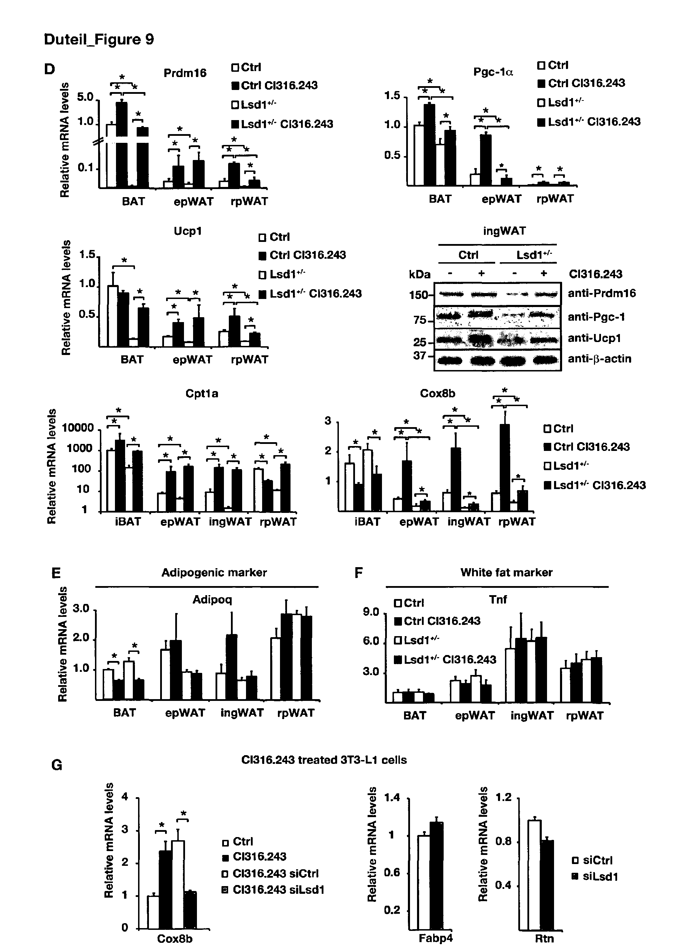

**Figure 9** **(relative to** **Figure 3****). β3-adrenergic-signalling regulates Lsd1 levels to prevent obesity-related disorders.**
   (A). Food consumption of control (Ctrl) and Tg mice fed a regular diet.
   (B). Representative hematoxylin and eosin stained iBAT, epWAT, and rpWAT white fat pads of control (Lsd1^{+/+}) and heterozygous Lsd1 knock-out (Lsd1^{+/-}) mice treated with CI316,243 or vehicle.
   (C). Relative *Lsd1* transcript levels in iBAT, epWAT, and rpWAT of control (Lsd1^{+/+}) and heterozygous Lsd1 knock-out (Lsd1^{+/-}) mice treated with CI316,243 or vehicle.
   (D). Analysis of brown fat marker expression levels upon CI316,243 treatment. Relative *Prdm 16, Pgc-1* and *Ucp1* transcript levels in iBAT, epWAT, and rpWAT, and Western blot analyses of Prdm16, Pgc-1 and Ucp1 protein levels in ingWAT of control (Lsd1^{+/+}) and heterozygous Lsd1 knock-out (Lsd1^{+/-}) mice treated with CI316,243 or vehicle.
   (E-F). Relative transcript levels of the adipogenic and white fat markers *Adipoq* (E) and *Tnf,* (F) respectively, in epWAT, ingWAT, rpWAT and iBAT of Ctrl and Lsd1^{+/-} mice treated with CI316,243 (Ctrl CI316,243 and Lsd1^{+/-} CI316,243, respectively) or vehicle (Ctrl and Lsd1^{+/-}, respectively).
   (F). Relative transcript levels of the white fat marker *Tnf* in epWAT, ingWAT, rpWAT and iBAT of Ctrl and Lsd1^{+/-} mice treated with CI316,243 (Ctrl CI316,243 and Lsd1^{+/-} CI316,243, respectively) or vehicle (Ctrl and Lsd1^{+/-}, respectively).
   (G). Relative transcript levels of *Cox8b, Fabp4, and Rtn* in CI316,243 treated differentiated (day 7) 3T3-L1 cells, transfected with either a siRNA control or directed against Lsd1.
   (A), n=10; (B) to (F), n=5, and (G), n=6; standard deviation represents + SEM, *: p<0.05, **: p<0.01, ***: p<0.001.

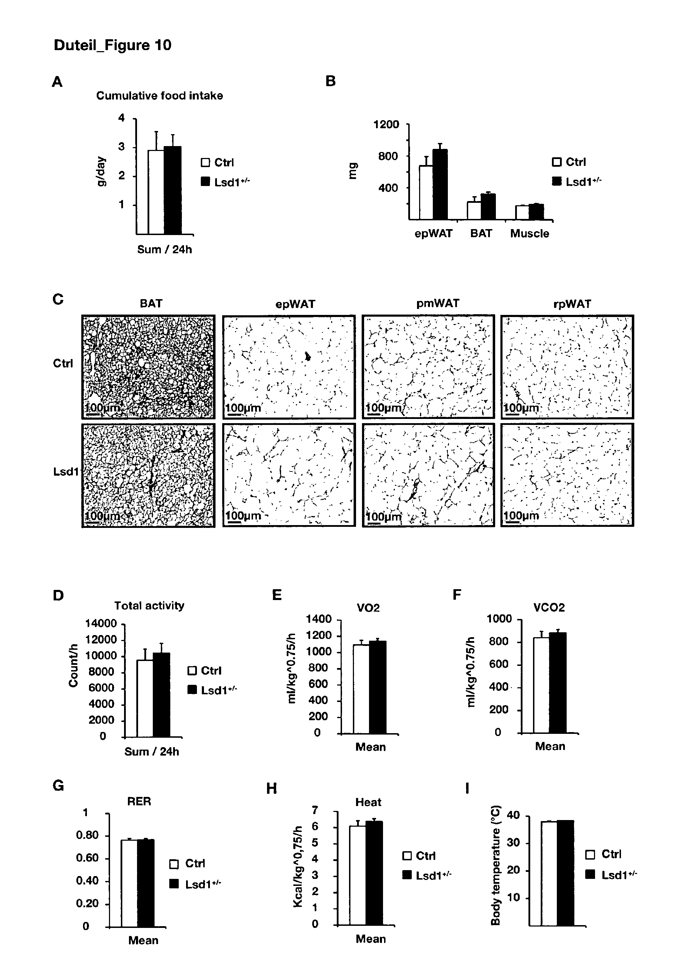

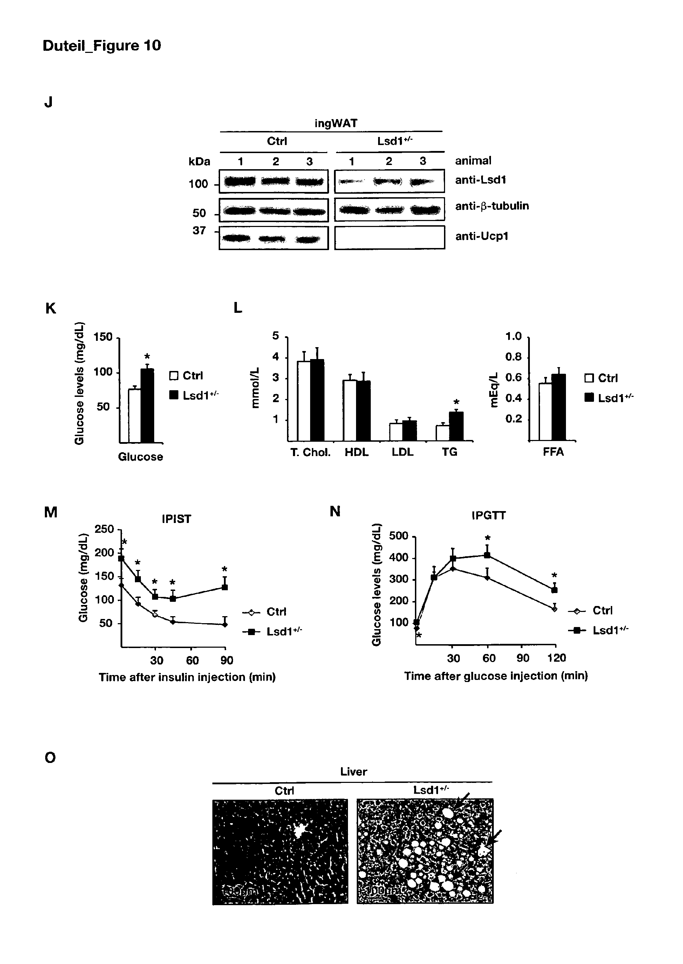

**Figure 10** **(relative to** **Figure 4****). Lsd1+/- mice are prone to obesity and type 2 diabetes upon high fat diet**
   (A). Food consumption of control (Ctrl) and heterozygous Lsd1 knock-out (Lsd1^{+/-}) mice fed a high fat diet.
   (B). Weight of iBAT and epWAT of Ctrl and Lsd1^{+/-} mice fed a high fat diet.
   (C). Representative hematoxylin and eosin staining of iBAT, epWAT, pmWAT and rpWAT white fat pads of Ctrl and Lsd1^{+/-} mice fed a high caloric diet.
   (D). Spontaneous activity of Ctrl and Lsd1^{+/-} mice fed a high fat diet.
   (E-H). VO₂ (E) and VCO₂ (F) determined in Ctrl and Lsd1^{+/-} mice fed a high fat diet, and evaluation of the RQ (G) in Ctrl and Lsd1^{+/-} mice fed a high fat diet and deduced heat production (H).
   (I). Rectal temperature of Ctrl and Tg mice fed a high caloric diet.
   (J). Western blot analysis showing Lsd1 and Ucp1 levels in Ctrl and Lsd1^{+/-} mice fed a high fat diet. β-Tibulin has been used as a loading control.
   (K). Serum glucose levels of Ctrl and Lsd1^{+/-} mice fed a high fat diet.
   (L). Serum cholesterol (total: T. Chol., HDL: HDL Chol. and LDL: LDL Chol.), triglyceride (TG) and free fatty acid (FFA) levels of Ctrl and Lsd1^{+/-} mice fed a high fat diet.
   (M)-(N). Intraperitoneal insulin sensitive test (IPIST) (M) and intraperitoneal glucose tolerance test (IPGTT) (N) of Ctrl and Lsd1^{+/-} mice fed a high fat diet.
   (O). Representative hematoxylin and eosin staining of liver from Ctrl and Lsd1^{+/-} mice fed a high caloric diet. Mutant mice are prone to liver steatosis.
      For (A) to (O), n=6; standard deviation represents + SEM, *: p<0.05, **: p<0.01, ***: p<0.001.

### EXAMPLES

### Results

### Lsd1 is induced in white fat pads after cold exposure

To characterize the physiological functions of the epigenetic regulator Lsd1 in fat metabolism, we performed a detailed analysis of Lsd1 expression in different depots of adipose tissue from mice housed at standard temperature (24 °C) and found that Lsd1 is expressed in both white and brown fat (Figures 1A, 5A, 5D and 5E). We wondered whether expression of Lsd1 in fat might be controlled by environmental cues, and thus might be affected during adaptation to cold stress. Therefore, two groups of littermates were maintained for 10 days at standard (24 °C) or cold temperature (10 °C). Importantly, concomitantly with the appearance of multilocular beige cells (Figure 5B) and induced Ucp1 expression (Figure 1A and 5A) (Seale et al., 2008; Vegiopoulos et al., 2010; Wu et al., 2012) protein levels of Lsd1 are substantially enriched in the analyzed white adipose tissues (WAT) after cold exposure (Figure 1A and 5A), suggesting that Lsd1 might be involved in cold response in WAT.

### Expression of LSD1 in transgenic mice promotes beige fat formation in white adipose tissue

To determine whether Lsd1 is a potential mediator of beige cells formation in WAT, we generated transgenic (Tg) mice expressing human LSD1 (hLSD1) under the control of the Rosa26 promoter (Figure 5C). Quantitative reverse transcription qRT-PCR, Western blot, and immunofluorescence analyses confirmed the expression of hLSD1 in WAT and interscapular brown adipose (BAT) tissue, respectivel (Figures 1B and 5D to 5F). Tg mice are viable and fertile, and show no gross abnormalities. However, detailed histological analyses revealed the presence of islets of multilocular brown adipocyte-like cells in white fat pads of Tg mice, namely in epididymal- (epWAT), inguinal- (ingWAT), perimuscular-(pmWAT), and retroperitoneal WAT (rpWAT), whereas the morphology of BAT was not altered (Figure 1C and 5G). Consistent with a beige-ing of fat pads our qRT-PCR analyses revealed significantly increased expression of *Prdm16, Pgc-1*α*, Ucp1, cell death-inducing DFFA-like effector a* (*Cidea*), and other thermogenic genes in WAT of Tg mice (Figures 1B, 5H, and 5I). Since classical brown fat derives from a Myf5-positive cellular lineage (Seale et al., 2008), we screened the islets of multilocular adipocyte-like cells for the presence of Myf5-positive cells. In contrast to classical BAT, Myf5 is not expressed in the adipocyte-like fat islets in the Tg mice (Figure 1C), suggesting that these fat islets emerge from a non-Myf5 cellular lineage and thus might represent beige fat. Consequently, we compared the expression pattern of BAT, WAT, and beige selective marker genes in control and Tg mice. We evaluated the expression of beige fat selective marker genes such as *Ear2, Cd137,* and *Tbx1* (Wu et al., 2012) that distinguish beige fat from both brown fat as well as white fats cells (Figure 1D and 5J). Remarkably, expression of beige fat selective marker genes is significantly upregulated in white fat pads of LSD1 Tg mice. In contrast, levels of brown fat selective markers such as *Eva1, Fbxo31* and *Hspb7* (Figures 1D and 5K) (Wu et al., 2012), adipogenic markers such as *fatty acid binding protein* 4 (*Fabp4*) and *adiponectin* (*Adipoq*), and white fat selective markers such as *tumor necrosis factor* (*Tnf*), *angiotensinogen* (*Agt*), *leptin* (*Lep*), and *resistin* (*Rtn*) are not significantly altered compared to control (Figures 1D and 5L). Thus, the LSD1-induced expression profile closely mirrors the recently described gene expression pattern of beige fat cells (Wu et al., 2012). Consistently, immunofluorescence analyses and Western blot studies confirmed the expression of Prdm16, Ucp1, and Ear2 in Lsd1-induced beige fat islets (Figures 1E and 5M). Taken together, this data demonstrate that LSD1 is a key regulator of the beige adipocyte gene expression program in vivo.

Next, we analyzed whether the beige fat islets in WAT of Tg mice represent functional thermogenic fat. To characterize the mitochondrial oxidative capability in brown, white, and beige fat, activities of key enzymes of oxidative phosphorylation (oxphos) were analyzed. The activity of Succinate dehydrogenase (Sdh, mitochondria complex II) and Cytochrome C oxidases (Cox, mitochondria complex IV) is significantly increased in beige fat islets in pmWAT and rpWAT (Figure 5N) of Tg mice. Together, these results show that increased expression of LSD1 in vivo results in the formation beige fat islets in WAT that express genes characteristic of thermogenically active fat.

### Metabolic capacities of Tg mice are similar to those of control mice upon regular diet.

Despite the presence of metabolically active brown fat in white fat pads, under a regular diet Tg mice do not display any notable difference in body weight compared with their control littermates (Figure 6A). Furthermore, serum glucose levels are similar between Tg and control mice at 30 weeks of age (Figure 6B), and intraperitoneal glucose tolerance test and insulin sensitive test also show no difference between control and Tg mice (Figure 6C and 6D, respectively). Moreover, serum cholesterol, triglyceride, and free fatty acids levels are similar between Tg and control mice (Figure 6E). Interestingly, insulin levels are 2.5-fold higher in Tg than in control mice (Figure 6F).

### Lsd1 promotes the expression of beige fat markers in adipocytes in vitro

Next, we asked whether elevated expression of hLSD1 in 3T3-L1 cells results in appearance of beige fat markers and recapitulates beige fat biogenesis in vitro. Therefore, we transduced 3T3-L1 cells with lentivirus driving expression of either wild-type hLSD1 (3T3-L1_LSD1) (Metzger et al., 2010) (Figure 7A). QRT-PCR analyses show substantially elevated expression levels of thermogenic genes including *Prdm16, Pgc-1*α*,* and *Ucp1* in differentiated 3T3-L1_LSD1 cells compared to control (Ctrl) (Figure 2A and 7B). Western blot analyses confirmed that Prdm16, Pgc-1α, and Ucp1 protein levels are highly concordant with RNA expression in 3T3-L1_LSD1 cells (Figure 2B). Consistent with our observations in Tg mice, transcript levels of the beige fat markers *Cd137, Ear2,* and *Tbx1* are increased in 3T3-L1_LSD1 cells (Figure 2A). Furthermore, 3T3-L1_LSD1 and control cells show decreased transcript levels of white adipocyte markers (Figure 7E) and similar transcript levels of general adipogenic markers such as Adipoq and Pparg (Figure 7F). These results were confirmed in differentiated C3H-10T1/2_LSD1 adipocytes (Figure 7G). Together, our data demonstrate that LSD1 is a cell-autonomous regulator of the beige fat differentiation program in vitro.

### Lsd1 controls mitochondrial activity and induction of the beige fat program through Nrf1 and Prdm16

To identify direct Lsd1 targets that might induce the beige fat program, we performed chromatin immunoprecipitation (ChlP) followed by massively parallel sequencing (ChlP-seq) with Lsd1 antibody in 3T3-L1 cells at days 0 (D0), 3 (D3), and 7 (D7) of differentiation. At D0, a total of 3900 high-confidence LSD1 binding sites were identified (using a stringent statistical false discovery rate [FDR] of less than 1 %). Interestingly, the genomic distribution of Lsd1 is enriched at promoters (Figure 2C). Indeed, the majority of the sites (83 %) are located at the promoter region (defined as ± 2000 bp around the TSS) while the remaining peaks are found at exons or 3'UTR regions (1 %), intergenic regions (10 %), and introns (6 %). The Lsd1 profile across an average Refseq gene shows high density around the transcription start site (TSS) and drops dramatically upon entering the gene body (Figure 8A). Furthermore, 3575 (91%) of the Lsd1 peaks overlap with H3K4me3 mark (Mikkelsen et al., 2010), which typically marks promoter regions of transcribed genes (Santos-Rosa et al., 2002), thus further corroborating the preferential association of Lsd1 with promoters in 3T3-L1 cells (Figure 2D). At D3 and D7, a total of 24356 and 20693 high-confidence Lsd1 binding sites were identified, respectively (Figure 8B). Interestingly, about 92 % of D0 peaks were also present at days D3 and D7 showing that this pattern of Lsd1 controlled gene is conserved along the differentiation and that Lsd1 is recruited to new sites recruited during differentiation at D3 and D7.

To characterize in more detail the group of genes controlled by Lsd1 in adipocytes, we performed a Database for Annotation, Visualization and Integrated Discovery (DAVID) analysis at D0, D3, and D7 (Table1). DAVID provides a comprehensive set of functional annotation tools to understand biological meaning behind gene lists. Interestingly, we found that Lsd1 was massively present at genes coding for of mitochondrial proteins at D0 (10 %), D3 (25 %), and D7 (25 %). Among this gene set, we identified numerous genes of the mitochondria electron transport chain. The substantial overlap of LSD1 with histone marks H3K4me3, H3K4me2, and H3K27ac, linked to active gene expression, is exemplarily displayed for Ndufa6, Sdha, Uqcrc1, Cox6a1, and Atp5b, each gene representative for the five different complexes of the mitochondrial electron transport chain (Figure 2E and 8D). In line with this data set, LSD1 controls the transcript levels of the five mitochondria complex components as shown by qRT-PCR analysis (Figure 2F). Taken together, these data ascribe a key role for LSD1 in regulating gene sets essential for energy production.

To unravel whether Lsd1 enrichment at promoters correlates with the presence of specific regulatory elements, we explored for the presence of enriched motifs via the HOMER motif finder algorithm (Heinz et al., 2010). Interestingly, binding sites for the transcription factor Nrf1 were ranked among the top-scoring motifs associated with promoters (Figure 2G). Nrf1 is considered a key player in mitochondrial biogenesis and growth and is implicated in the control of nuclear genes required for respiration, mitochondrial DNA transcription, and replication (Scarpulla, 2008).

We then performed a DAVID analysis to identify common Lsd1 and Nrf1 target genes at D0 (Table1). Interestingly, the analysis uncovered not only colocalization of Lsd1 and Nrf1 binding sites at the Nrf1 promoter, suggesting a positive auto-regulation of Nrf1 gene expression, but also that Lsd1 and Nrf1 were present at a vast number of gene promoters controlling β-oxidation (Table1) and about 25 % of all mitochondria electron transport chain genes (Table1, in yellow). The substantial overlap of high-confidence LSD1 and Nrf1 is exemplarily displayed for Ndufa6, Sdha, Uqcrc1, Cox6a1, and Atp5b (Figure 2E).

Importantly, Lsd1 binding regions overlap with active histone marks H3K4me3, H3K4me2, and H3K27ac, suggesting that Lsd1 might regulate Nrf1 expression (Figure 8E). We verified this hypothesis by comparing the expression levels of Nrf1 in control and 3T3-L1_LSD1, 10T1/2_LSD1 cells, as well as in fat tissues of Tg mice (Figures 8F-H). Nrf1 was increased in differentiated 3T3-L1 and 10T1/2 cells as well as in beige fat in Tg mice, suggesting that Lsd1 might positively regulate oxphos-related genes expression via Nrf1. Furthermore, Lsd1 is recruited to thermogenic genes such as *Pgc-1*α*, Cpt1a, Ucp2, Ucp3,* and the Nrf1 target gene mitochondria transcription factor A (Tfam) at D3 and D7 (Figure 8M). Concomitantly with Lsd1 promoter binding transcription of Tfam is increased in differentiated adipocytes in vitro and beige fat in vivo (Figures 8J to 8L). Since Nrf1, Tfam, Pgc-1α are known to play major roles in mitochondria replication and activity, we hypothesized that increased Lsd1 levels might enhance mitochondrial functions. To corroborate this further, 3T3-L1_LSD1 and control cells were stained with JC-1, a dye that shows green fluorescence upon binding to the mitochondrial inner membrane (FL1; mitochondrial mass) and forms red-fluorescent aggregates depending on the membrane potential (FL2; respiratory activity). Flow cytometric analyses of JC-1-stained cells revealed that 3T3-L1_LSD1 cells exhibit significantly increased mitochondrial membrane potentials with an elevated FL2/FL1 ratio representing increased respiratory activity relative to the mitochondrial surface area (Figure 2H and 2I).

To evaluate further, whether promotion of the thermogenic program by LSD1 is driven through induction of Nrf1, we used short interfering RNA (siRNA)-mediated knockdown (KD) in 3T3-L1_LSD1 cells (Figures 2J and 2K). Knockdown of Nrf1 significantly decreased expression levels of *Nrf1, Prdm16, Pgc-1*α*,* and *Ucp1* in differentiated adipocytes (Figures 2J and 2K). Since KD of Prdm16 also decreased expression levels of the *Prdm16, Pgc-1*α*, Ucp1, Cpt1a,* and *Cox8b* in 3T3-L1_LSD1 cells (Figures 2L, 2M, and 8N) the data suggest that the LSD1-controlled thermogenic program is mediated by Nrf1 and Prdm16.

### Lsd1 mediates β3-adrenergic-signalling to promote the beige fat program

White fat cells secrete numerous hormones including leptin, which decreases appetite and induces energy expenditure (Halaas et al., 1995; Plum et al., 2007). Leptin levels are strongly increased in LSD1 Tg mice (Figure 3A). Upon a regular diet, food consumption is not altered in Tg mice compared to their control littermates (Figure 9A). Leptin has been reported to enhance metabolism through the β3-adrenergic pathway (Chuang et al., 2010) and treatment of mice with the β3-selective adrenergic agonist CL316,243 promotes beige fat formation within white fat depots (Himms-Hagen et al., 1994), which is reminiscent of the phenotype of LSD1 Tg mice. Since the exact mechanism by which CL316,243 promotes beige fat formation has remained elusive (Seale et al., 2007), we hypothesized that it may be mediated via upregulation of Lsd1 expression. Upon treatment of mice with CL316,243 for three days, we observed formation of clusters of multilocular beige cells in white fat pads (Figure 3B and 9B), which is in agreement with published results. Importantly, both transcript and protein levels of Lsd1 are strongly increased by CL316,243 in mice (Figures 3C and 9C). This observation supports the hypothesis that CL316,243 induced beige fat accumulation in white fat depots might be mediated by upregulation of Lsd1 expression. Accordingly, this idea implies that CL316,243 may not efficiently induce beige fat islets in WAT in Lsd1^{+/-} mice. To validate this hypothesis *in vivo* we chose Lsd1^{+/-} heterozygous mice since the development of Lsd1 null mice is interrupted at day E6.5 (Wang et al., 2009a; Wang et al., 2007). Treatment of Lsd1^{+/-} mice with the β3-selective adrenergic agonist generated fewer clusters of beige fat cells in WAT compared to control mice (Figure 3B and 9B). Of note, CL316,243 treatment of Lsd1^{+/-} mice restores *Lsd1* mRNA and protein levels to those of untreated wild-type control mice (Figure 3C, 9C). In accordance with previous reports, qRT-PCR and Western blot analyses show that treatment of wilt-type mice with CL316,243 significantly increases the transcript and protein levels of *Prdm16, Pgc-1*α*,* and *Ucp1* (Figure 3D and 9D) in WAT. Interestingly, upon treatment, *Prdm16, Pgc-1*α and *Ucp1* transcript and protein levels of Lsd1^{+/-} mice reach those of untreated control mice whereas white fat and adipogenic markers are not significantly affected (Figure 9E and 9F). These in vivo results strongly indicate that induction of the beige fat cells program via the β3-adrenergic signaling pathway is, at least in part, mediated by Lsd1.

### β3-adrenergic-signalling induces Lsd1 expression via Creb in adipocytes

To identify the mechanisms responsible for Lsd1 induction by the β3-adrenergic cascade, we treated 3T3-L1 adipocytes with CL316,243. Consistent with our observations in mice, levels of *Lsd1, Prdm16, Pgc-1*α*,* and, *Ucp1* increased after treatment (Figures 3E and 3F). To evaluate whether promotion of the beige fat program upon CL316,243 treatment is driven by induction of Lsd1, we used siRNA-mediated knockdown in 3T3-L1 cells (Figures 3E and 3F). As expected, Lsd1 knockdown decreased expression levels of *Prdm16, Pgc-1*α, and *Ucp1* (Figures 3E, 3F and 9G), demonstrating that Lsd1 propagates gene expression in response to β3-adrenergic signaling. Since β3-adrenergic signaling is transduced by the transcription factor Creb (Fu et al., 2005), we unraveled a potential Creb-response element (GTGATCATTAATTAAGTTGTA) at position -380 bp in the promoter of Lsd1 and Creb is recruited to the Lsd1 promoter after treatment with CI316,243 as demonstrated by ChlP (Figure 3G). Together, our data show that Lsd1 propagates the β3-adrenergic signaling pathway to promote beige fat formation.

### Lsd1+/- mice are prone to obesity and type 2 diabetes due to decreased oxidative capacities in adipose tissue

Since LSD1 expression drives the activation of the beige fat program and results in increased energy expenditure to counteract diet-induced obesity, we speculated that Lsd1 levels might be altered by a high caloric diet uptake. Indeed, as shown in Figure 4A, Lsd1 protein levels were dramatically decreased during a high fat diet. We then reasoned that ablation of Lsd1 in mice would lead to decreased oxidative capacities. As expected, *Lsd1* mRNA and protein levels are reduced by approximately 50 % in WAT and BAT of Lsd1^{+/-} mice (Figures 3A, 3B, and, 9A). As shown in Figure 9B, BAT of Lsd1^{+/-} mice is characterized by the presence of enlarged lipid droplets, while no difference is observed in the morphology of white fat pads of control and Lsd1^{+/-} mice. Decreased Lsd1 levels in Lsd1^{+/-} mice correlate with a significant reduction in transcript and protein levels of *Prdm16, Pgc-1*α*,* and *Ucp1* (Figures 3C and 9C). Conversely, adipogenic and white fat-selective markers are overall not affected in Lsd1^{+/-} mice (Figures 9E and 9F). Taken together, these data show impaired expression of genes regulation oxidative capacities in Lsd1^{+/-} mice suggesting that reduced expression of LSD1 might prone mice to obesity. Consequently, when challenged with a high fat diet, Lsd1^{+/-} mice gained substantially more weight than their control littermates (Figure 4B) despite a similar food intake (Figure 10A). Quantitative Nuclear Magnetic Resonance Spectroscopy (qNMR) analyses revealed that body fat content was 16 % higher in Lsd1^{+/-} mice than in control littermates (Figure 4C). In agreement with these results, fat mass was higher in Lsd1^{+/-} mice (Figure 10B), and histological analyses revealed that their adipocytes were more hypertrophic (Figure 4D and Figure 10C). Increased body fat mass was not due to decreased mobility since total activity was not altered (Figure 10D). Oxygen consumption (VO₂) was determined to characterize energy expenditure (Figure 10E). Upon HFD, neither VO₂ nor CO₂ production (VCO₂) were altered (Figure 10E and 10F). Consequently, the respiratory quotient (RQ) was not modified and corresponds to fatty acid consumption in both Ctrl and Lsd1^{+/-} mice (Figures 10G). In addition, calorimetric parameters deduced from these analyses indicated no difference in heat production between Lsd1^{+/-} and control mice (Figure 10H). In agreement with these results, body temperature was similar in Lsd1^{+/-} mice and their age-matched control littermates (Figure 10I). However, consistent with the decreased Lsd1 levels, Ucp1 protein levels were decreased in Lsd1^{+/-} mice (Figure 10J). Blood glucose levels were higher in Lsd1^{+/-} than in control mice upon high-caloric-diet feeding (Figure 10K), as well as blood cholesterol and triglyceride levels (Figures 10L). In parallel, insulin sensitivity and glucose uptake were reduced in Lsd1^{+/-} mice (Figures 10M and 10N), indicating that Lsd1^{+/-} mice are prone to type-2 diabetes. Furthermore, H&E staining revealed that the liver steatosis of Lsd1^{+/-} mice is dramatically increased upon high fat diet (Figure 10O). In summary, the in vivo analyses of Lsd1 transgenic- and heterozygous mice demonstrate that Lsd1 coordinates the formation and the oxidative capacities of beige fat, thus providing protection from obesity-related disorders.

### Materials and Methods

### Mice

Mice were maintained in a temperature and humidity controlled animal facility, with a 12 hours light/dark cycle and free access to water and a standard rodent chow (2800 kcal/kg, Usine d'Alimentation Rationelle, Villemoisson-sur-Orge, France). The high fat diet (HFD) study was carried out with a chow containing 4.056 kcal/kg (fat: 1.600 kcal/kg and sucrose: 1.600 kcal/kg; research Diets, New Brunswick, New Jersey). HFD was given to mice at 7 weeks of age. Breeding and maintenance of mice were performed according to institutional guidelines. For cold stress experiment, mice were maintained at 10 °C, with a 12 hours light/dark cycle and free access to water and a standard rodent chow. Animals were killed by cervical dislocation and tissues were immediately collected, weighted, and frozen in liquid nitrogen or processed for biochemical and histological analysis. In general, we adhered to the nomenclature for dissected depots described by Murano et al.. Specific definitions of dissected depots are as follows: epididymidal WAT (epWAT), prominent bilateral intra-abdominal visceral depots in male mice attached to the epididymides; inguinal, bilateral superficial inguinal WAT (ingWAT) depots between the skin and muscle fascia just anterior to the lower segment of the hind limbs; perimuscular WAT (pmWAT), depots located on the upper part of the quadriceps muscle; retroperitoneal WAT (rpWAT), bilateral depots in abdominal cavity behind the peritoneum on the dorsal side of the kidney; interscapular BAT (iBAT), most prominent depot of classic brown adipocytes in rodents, found between the scapulae.

### Generation of Rosa26-LSD1 transgenic (Tg) mice

To generate transgenic mice, the complete human LSD1 cDNA was cloned 3' to the Rosa26 promoter/enhancer and the human growth hormone polyadenylation site was inserted 3' to the cDNA (Figure 5C). Mice were genotyped by PCR amplification of genomic DNA extracted from tail biopsies using the DirectPCR extraction kit (Viagen, cat # 102-T), with primers for detection of the transgene Rosa26-LSD1 (Table2). As expected, LSD1 RNA and protein are present in all fat pads analyzed (Figure 5D and 5E).

### Generation of Lsd1^{+/-} mice

Generation of Lsd1^{+/-} mice was described previously (Zhu et al., 2012). Briefly, to ubiquitously ablate Lsd1 in mice, Lsd1p/p mice bearing floxed Lsd1 alleles (in which LoxP sites flank the Lsd1 exon 1 containing the ATG) were bred with Rosa26-Cre(Tg/0) transgenic mice that ubiquitously express the Cre recombinase. Mice were genotyped by PCR amplification of genomic DNA extracted from tail biopsies using the DirectPCR extraction kit (Viagen, cat # 102-T), with primers for detection of the floxed Lsd1 and the deleted allele (Table2).

### Mouse treatment

CL316,243 (Sigma-Aldrich) was injected intraperitoneally into mice at 1 mg/kg.

### Food Consumption

Mice were individually housed. Food pellets (150 g) were delivered and weighed after 1 week. Weekly food consumption was calculated by subtracting the final from the initial pellet weight.

### Serum analysis

Blood was collected from retro orbital sinus after a 6 hr fast that started at the beginning of the light cycle, and serum glucose, insulin, leptin, adiponectin, cholesterol, triglyceride and free fatty acid levels were analyzed as described (Picard et al., 2002).

### Glucose tolerance and insulin sensitivity tests

### Intraperitoneal glucose tolerance test (IPGTT)

IPGTT was performed on 6 h fasted regular diet fed mice and on 16 h fasted high fat diet fed mice. After measurement of the basal glucose level on blood collected from the tail vein (time 0), mice were intraperitoneally injected with a 20 % glucose solution in sterile saline (0.9 % NaCl) at a dose of 2 g glucose/kg body weight. Blood was collected from the tail vein after 15, 30, 45, 60, 90, 120, 150 and 180 min for glucose determination.

### Insulin sensitivity test (IPIST)

Six hour fasted regular diet fed mice and on 16 h fasted high fat diet fed mice were intraperitoneally injected with porcine insulin (0.5 U/kg; Sigma). Blood was collected at 15, 30, 60, and 90 min, and glucose analyzed as describe above.

### Temperature measurement

Body temperature was measured with a rectal probe linked to a digital thermometer (Bioseb). For the cold test, mice were stored at 4 °C in individual cages for 4 h. Their body temperature was measured every hour.

### Body Lean and Fat Content

Body lean and fat content were recorded in anaesthetized mice QNMR (PIXIMUS, GE Medical Systems, Buc, France) according to the manufacturer's instructions.

### Energy expenditure

Oxygen consumption and carbon dioxide production were measured with a Labmaster system (TSE, www.TSE-Systems.com) at 26 minutes intervals for 24 h. VO2 and VCO2 values were normalized to body mass. The respiratory quotient (RQ) corresponds to VCO2/VO2. Heat (Kcal/kg0.75h) was calculated using the formula: Heat (H) = (Calorific Value x VO2 x 0.001) / body weight x 0.75; (Caloric Value = 3.815 + 1.232 x RQ). Body temperature was measured with a rectal probe linked to a digital thermometer (Bioseb).

### Histological and histochemical analysis

Epididymal, inguinal, perimuscular and retroperitoneal WAT, and interscapular BAT were fixed in 10 % buffered formalin, dehydrated in ethanol, embedded in paraffin, and cut at 5 µm. Sections were deparaffinized, rehydrated, and stained with hematoxylin and eosin, washed in running tap water, decolorized in acid alcohol (1 mL HCl 37 % in 100 mL of 70 % EtOH) for 2 seconds, washed in running tap water, dehydrated, cleared and mounted. For immunohistochemistry, 5 µm cryosections were deparaffinized, rehydrated, boiled in antigen unmasking solution (H-3300, Vector) for 10 min, cooled at room temperature and then washed with PBS, 0.1 % Triton-X100 3 times for 5 min, incubated in 5 % Fetal Bovine Serum (10270-106, Gibco) in PBS, 0.1 % Triton-X100 for 1 h and overnight at 4°C with anti-LSD1 (3544, 1/1000), anti-Myf5 (C20, sc-302, 1/500), anti-Prdm16 (abcam, ab118573, 1/500), anti-Pgc-1 (H300, sc-13067, 1/500) and anti-Ucp1 (abcam, ab10983, 1/200) antibodies. Slides were then incubated with a secondary antibody Alexa546 (Ref, 1/400) and mounted in aqueous medium with DAPI (SIGMA, D-9542, 1/1000). Between each step, sections were washed with PBS, 0.1 % Triton-X100.

### RNA Preparation and Analysis

RNA was isolated with TRlzol Reagent (Invitrogen). Five micrograms of RNA was converted to cDNA with SuperScript II reverse transcriptase (Invitrogen, Life Technologies) and hexamers according to the supplier's protocol. Quantitative RT-PCR was performed by using the Abgene SYBR Green PCR kit (Invitrogen) according to the supplier's protocol. 36B4, HPRT and TBP were used as an internal controls. Primer sequences are given in Table2.

### Protein preparation and analysis

Fat tissues were grounded in RIPA buffer [50 mM Tris pH 7.5, 1 % Nonident P40, 0.5 % Sodium Deoxycholate, 0.1 % SDS, 150 mM NaCl, 5 mM EDTA and protease inhibitor cocktail (45 g/mL, 11 873 580 001, Roche)] with a potter at 4°C. Homogenates (100 g of protein) were electrophoresed on 10 % polyacrylamid gels. Proteins were electroblotted to Hybond nitrocellulose membranes (Amersham Biosciences) and immunodetected using primary antibodies directed against anti-LSD1 (Metzger et al., 2005), anti-Prdm16 (abcam, ab118573, 1/500), anti-Pgc-1 (H300, sc-13067, 1/1000) and anti-Ucp1 (abcam, ab10983, 1/1000), Fabp4 (sc-18661, 1/2000), β-tubulin (Sigma, T6074, 1/10000), and β-actin (Sigma, A1978, 1/10000). Secondary antibodies conjugated to horseradish peroxidase (Amersham Biosciences) were detected using an enhanced chemiluminescence detection system (Pierce, Rockford, IL, 1/10000).

### Chromatin immunoprecipitation (ChlP) and ChlP sequencing (ChlPseq)

ChlP experiments were performed as described (Metzger et al., 2008). Immunoprecipitation was performed with specific antibodies: anti-H3K9me2 (07-441 lot 30309) (Upstate Biotechnology), anti-H3K4me2 (cs-035-100 lot A391001) (Diagenode), anti-H3 (abcam, ab1791 lot 172452), anti-LSD1 (Metzger et al., 2005), and anti-Creb (abcam, ab31387) on protein A-Sepharose 4B (GE-Healthcare). After elution, DNA volume was adjusted from 50 µL to 200 µL. For qPCR, 2µL out of 200 µL DNA extract was used. qPCR primers for Prdm16 and Lsd1 are described in Table1. ChlPseq analysis was performed according to (Mikkelsen et al., 2010). Briefly, sequence reads from each ChlP-Seq library were sequenced on an Illumina platform (Illumina, USA, San Diego). 49 bp sequences were generated and mapped to the mm9 reference genome by bowtie 0.12.7 (Langmead et al., 2009) with standard parameters. These raw sequencing data were further analyzed using the peak finding algorithm MACS 1.4.1 (Zhang et al., 2008) using sequences from input as control. All peaks with FDR less than 1 % were included. The uniquely mapping locations were used to generate the genome-wide intensity profiles, which were visualized using the IGV genome browser (Thorvaldsdottir et al., 2012). Homer (Shin et al., 2009) was used to annotate peaks, to calculate overlaps between different bed files, and for the motif analysis. To draw the average gene profile CEAS was used (Shin et al., 2009). The genes obtained from the homer analysis were further used for a DAVID analysis (Huang et al., 2010).

### Biochemical analysis.

Tissues obtained from control and Tg mice were immediately frozen in liquid nitrogen. We determined the activities of the respiratory chain enzyme complexes SDH (complex II) and COX (complex IV) as described (Puccio et al., 2001).

### Ultrastructure analyses

For ultrastructural analyses, fat samples were fixed by immersion in 2.5 % glutaraldehyde and 2.5 % paraformaldehyde in cacodylate buffer (0.1 M, pH 7.4) and washed in cacodylate buffer for 30 minutes and kept at 4 °C. Post-fixation was performed with 1 % osmium tetraoxide in 0.1 M cacodylate buffer for 1 h at 4 °C and dehydration through graded alcohol (50, 70, 90 and 100 %) and propylene oxide for 30 minutes each. Samples were embedded in Epon 812. Ultrathin sections were cut at 70 nm and contrasted with uranyl acetate and lead citrate, and examined at 70 kv with a Morgagni 268D electron microscope. Images were captured digitally by a Mega View III camera (Soft Imaging System). Mitochondria number and cross section areas were determined with the MetaMorph software.

### Plasmids

The following plasmids were used: pLenti4_Flag_HA_Puro, pLenti4_Flag_HA_Puro_hAOF2, pLenti4_FlagHA_Puro_hAOF2_K661A_W751A_Y761S, pSlik_Neo, pSlik_Neo_Flag_HA_hLSD1, pSlik_Neo_hLSD1_K661A_W751A_Y761S. Plasmids were obtained by Gateway LRII cloning in the pLenti4 and the pSlik vectors, respectively. Cloning details can be obtained upon request.

### Cell culture

### Cell maintenance

293FT cells were cultured in Dulbecco's modified Eagle's medium (DMEM) supplemented with 10 % fetal bovine serum (FBS, Gibco), non-essential amino-acids and Glutamine (Gibco). Mouse 3T3-L1 and 10T1/2 preadipocytes were maintained in DMEM or Eagle's minimal essential medium (EMEM), respectively, supplemented with 10 % FBS and Glutamine. Differentiation of 3T3-L1 and 10T1/2 to adipocytes was induced by treatment of confluent cells (designated day 0) with an adipogenic mixture consisting of 10 µg/mL insulin, 1 µM dexamethasone, 10 µM rosiglitazone and 500 µM isobutylmethylxanthine in the presence of 10% FCS. The differentiation medium was replaced 3 days later (day 3) with medium supplemented with 10% FBS and 10 µg/mL insulin for 2 days. Afterwards, the cells were cultured in same medium for 2 more days (day 7). For brown fat inducement, cells were treated with CL316,243 (Sigma-Aldrich) at 1 µM for 4 h at 37 °C.

### Oil Red-O staining

Oil Red-O staining was performed as described by René Koopman et al. Histochem. Cell Biol. (2001). Briefly, cells were fixed in 4 % PFA for 1 h at room temperature and stained with an Oil Red-O staining solution (stock solution: 0.5 % Oil Red-O, 60 % triethyl phosphate (Fluka) in H₂O; working solution: 60 % of stock solution in water). For OD measurements, stained cells were incubated for 10 min in a 4 % NP-40 solution (in isopropanol), and absorbance was measured at 529 nm.

### Cell transfection

10T1/2 stable transfection assays were carried out in 6-well plates. Briefly, at 50-70 % confluence cell medium was changed for growth medium (GM: DMEM, 10 % FCS, non-essential amino-acids and glutamine) for 3 h. Cells were then transfected with Fugene HD and 750ng of DNA in growth medium without FCS for 5 h at 37 °C. FCS was then adjusted at 10 %. Cells were selected with 0.5 µM of puromycine.

### Cell infection

For viral production, 293FT cells were transfected using Polyethylenimine (Pei), with the transgene vector (see Plasmids), the Gag/pol, and the pVSVG vectors. After viral production, the cell supernatant was collected and the virus was concentrated by ultracentrifugation. 3T3-L1 cells were then infected and selected with 1 µM of puromycine.

### RNA interference

A total of 3 x 10⁶ 3T3-L1 cells were transfected with 1 µM siRNA against Lsd1 (stealth 675, Invitrogen), Prdm16 (stealth 513, Invitrogen), Nrf1 (stealth 255, Invitrogen) or negative control using DharmaFECT 1 (Thermo Scientific).

siRNA oligonucleotide sequences were as follows:
Lsd1 siRNA-sense: 5'-CCCAAAGAUCCAGCUGACGUUUGAA-3'
Control-Lsd1 siRNA-sense: 5'-UUCUUAGCAAGACUGGUCUCUAGGG-3'
Prdm16 siRNA sense: 5'-CAUCCGUGUAGCGUGUUCCUGUGAU-3'
Control-Prdm16 siRNA sense: 5'-AUCGGAACGGAAACGCUCCACAAUG-3'
Nrf1 siRNA sense: 5'-UAUGGUAGCCAUGUGUUCAGUUUGG-3'
Control-Nrf1 siRNA sense: 5'-UAUUUGGAUGUACCUGUGGACUUGG-3'

### Cellular metabolism

For the determination of mitochondrial metabolism, 3T3-L1 cells were stained with fluorescence dye JC-1, followed by flow cytometric analysis. Following trypsinization, cells were exposed to 20 µg/mL of JC-1 in the culture medium for 15 min at 37 °C and were suspended in PBS for fluorescence-activated cell sorting (FACS) analysis. Green and red fluorescent signals detected mitochondrial mass (FL1) and mitochondrial membrane potential (FL2), respectively, using a FACS Canto cytometer (Becton Dickinson).

### Data Analysis

Data are represented as mean ± standard error of the mean (SEM). Differences analyzed by a two-tailed Student's t tests were considered statistically significant at p < 0.05 and are indicated by an asterisk in the figures.

### References

Auffret, J., Viengchareun, S., Carre, N., Denis, R.G., Magnan, C., Marie, P.Y., Muscat, A., Feve, B., Lombes, M., and Binart, N. (2012). Beige differentiation of adipose depots in mice lacking prolactin receptor protects against high-fat-diet-induced obesity. FASEB J 26, 3728-3737.
Baar, K. (2004). Involvement of PPAR gamma co-activator-1, nuclear respiratory factors 1 and 2, and PPAR alpha in the adaptive response to endurance exercise. Proc Nutr Soc 63, 269-273.
Cam, H., Balciunaite, E., Blais, A., Spektor, A., Scarpulla, R.C., Young, R., Kluger, Y., and Dynlacht, B.D. (2004). A common set of gene regulatory networks links metabolism and growth inhibition. Mol Cell 16, 399-411.
Cho, Y.W., Hong, S., Jin, Q., Wang, L., Lee, J.E., Gavrilova, O., and Ge, K. (2009). Histone methylation regulator PTIP is required for PPARgamma and C/EBPalpha expression and adipogenesis. Cell Metab 10, 27-39.
Chuang, J.C., Krishnan, V., Yu, H.G., Mason, B., Cui, H., Wilkinson, M.B., Zigman, J.M., Elmquist, J.K., Nestler, E.J., and Lutter, M. (2010). A beta3-adrenergic-leptin-melanocortin circuit regulates behavioral and metabolic changes induced by chronic stress. Biol Psychiatry 67, 1075-1082.
Darlington, G.J., Ross, S.E., and MacDougald, O.A. (1998). The role of C/EBP genes in adipocyte differentiation. J Biol Chem 273, 30057-30060.
Fu, L., Patel, M.S., Bradley, A., Wagner, E.F., and Karsenty, G. (2005). The molecular clock mediates leptin-regulated bone formation. Cell 122, 803-815.
Garcia-Bassets, I., Kwon, Y.S., Telese, F., Prefontaine, G.G., Hutt, K.R., Cheng, C.S., Ju, B.G., Ohgi, K.A., Wang, J., Escoubet-Lozach, L., et al. (2007). Histone methylation-dependent mechanisms impose ligand dependency for gene activation by nuclear receptors. Cell 128, 505-518.
Granneman, J.G., Li, P., Zhu, Z., and Lu, Y. (2005). Metabolic and cellular plasticity in white adipose tissue I: effects of beta3-adrenergic receptor activation. Am J Physiol Endocrinol Metab 289, E608-616.
Halaas, J.L., Gajiwala, K.S., Maffei, M., Cohen, S.L., Chait, B.T., Rabinowitz, D., Lallone, R.L., Burley, S.K., and Friedman, J.M. (1995). Weight-reducing effects of the plasma protein encoded by the obese gene. Science 269, 543-546.
Heinz, S., Benner, C., Spann, N., Bertolino, E., Lin, Y.C., Laslo, P., Cheng, J.X., Murre, C., Singh, H., and Glass, C.K. (2010). Simple combinations of lineage-determining transcription factors prime cis-regulatory elements required for macrophage and B cell identities. Mol Cell 38, 576-589.
Himms-Hagen, J. (1990). Brown adipose tissue thermogenesis: interdisciplinary studies. FASEB J 4, 2890-2898.
Himms-Hagen, J., Cui, J., Danforth, E., Jr., Taatjes, D.J., Lang, S.S., Waters, B.L., and Claus, T.H. (1994). Effect of CL-316,243, a thermogenic beta 3-agonist, on energy balance and brown and white adipose tissues in rats. Am J Physiol 266, R1371-1382.
Himms-Hagen, J., Melnyk, A., Zingaretti, M.C., Ceresi, E., Barbatelli, G., and Cinti, S. (2000). Multilocular fat cells in WAT of CL-316243-treated rats derive directly from white adipocytes. Am J Physiol Cell Physiol 279, C670-681.
Hino, S., Sakamoto, A., Nagaoka, K., Anan, K., Wang, Y., Mimasu, S., Umehara, T., Yokoyama, S., Kosai, K., and Nakao, M. (2012). FAD-dependent lysine-specific demethylase-1 regulates cellular energy expenditure. Nat Commun 3, 758.
Hirotsu, Y., Hataya, N., Katsuoka, F., and Yamamoto, M. (2012). NF-E2-related factor 1 (Nrf1) serves as a novel regulator of hepatic lipid metabolism through regulation of the Lipin1 and PGC-1 beta genes. Mol Cell Biol 32, 2760-2770.
Huang, J., Wang, H., Xie, X., Gao, H., and Guo, G. (2010). Developmental changes in DNA methylation of pollen mother cells of David lily during meiotic prophase I. Mol Biol (Mosk) 44, 853-858.
Langin, D. (2010). Recruitment of brown fat and conversion of white into brown adipocytes: strategies to fight the metabolic complications of obesity? Biochim Biophys Acta 1801, 372-376.
Langmead, B., Trapnell, C., Pop, M., and Salzberg, S.L. (2009). Ultrafast and memory-efficient alignment of short DNA sequences to the human genome. Genome Biol 10, R25. Lee, M.G., Wynder, C., Cooch, N., and Shiekhattar, R. (2005). An essential role for CoREST in nucleosomal histone 3 lysine 4 demethylation. Nature 437, 432-435.
Loncar, D. (1991). Convertible adipose tissue in mice. Cell Tissue Res 266, 149-161.
Mazzucotelli, A., Viguerie, N., Tiraby, C., Annicotte, J.S., Mairal, A., Klimcakova, E., Lepin, E., Delmar, P., Dejean, S., Tavernier, G., et al. (2007). The transcriptional coactivator peroxisome proliferator activated receptor (PPAR)gamma coactivator-1 alpha and the nuclear receptor PPAR alpha control the expression of glycerol kinase and metabolism genes independently of PPAR gamma activation in human white adipocytes. Diabetes 56, 2467-2475.
Metzger, E., Imhof, A., Patel, D., Kahl, P., Hoffmeyer, K., Friedrichs, N., Muller, J.M., Greschik, H., Kirfel, J., Ji, S., et al. (2010). Phosphorylation of histone H3T6 by PKCbeta(I) controls demethylation at histone H3K4. Nature 464, 792-796.
Metzger, E., Wissmann, M., Yin, N., Muller, J.M., Schneider, R., Peters, A.H., Gunther, T., Buettner, R., and Schule, R. (2005). LSD1 demethylates repressive histone marks to promote androgen-receptor-dependent transcription. Nature 437, 436-439.
Metzger, E., Yin, N., Wissmann, M., Kunowska, N., Fischer, K., Friedrichs, N., Patnaik, D., Higgins, J.M., Potier, N., Scheidtmann, K.H., et al. (2008). Phosphorylation of histone H3 at threonine 11 establishes a novel chromatin mark for transcriptional regulation. Nat Cell Biol 10, 53-60.
Mikkelsen, T.S., Xu, Z., Zhang, X., Wang, L., Gimble, J.M., Lander, E.S., and Rosen, E.D. (2010). Comparative epigenomic analysis of murine and human adipogenesis. Cell 143, 156-169.
Musri, M.M., Carmona, M.C., Hanzu, F.A., Kaliman, P., Gomis, R., and Parrizas, M. (2010). Histone demethylase LSD1 regulates adipogenesis. J Biol Chem 285, 30034-30041.
Plum, L., Rother, E., Munzberg, H., Wunderlich, F.T., Morgan, D.A., Hampel, B., Shanabrough, M., Janoschek, R., Konner, A.C., Alber, J., et al. (2007). Enhanced leptin-stimulated Pi3k activation in the CNS promotes white adipose tissue transdifferentiation. Cell Metab 6, 431-445.
Puccio, H., Simon, D., Cossee, M., Criqui-Filipe, P., Tiziano, F., Melki, J., Hindelang, C., Matyas, R., Rustin, P., and Koenig, M. (2001). Mouse models for Friedreich ataxia exhibit cardiomyopathy, sensory nerve defect and Fe-S enzyme deficiency followed by intramitochondrial iron deposits. Nat Genet 27, 181-186.
Puigserver, P., and Spiegelman, B.M. (2003). Peroxisome proliferator-activated receptor-gamma coactivator 1 alpha (PGC-1 alpha): transcriptional coactivator and metabolic regulator. Endocr Rev 24, 78-90.
Puigserver, P., Wu, Z., Park, C.W., Graves, R., Wright, M., and Spiegelman, B.M. (1998). A cold-inducible coactivator of nuclear receptors linked to adaptive thermogenesis. Cell 92, 829-839.
Ricquier, D., and Bouillaud, F. (2000). Mitochondrial uncoupling proteins: from mitochondria to the regulation of energy balance. J Physiol 529 Pt 1, 3-10.
Santos-Rosa, H., Schneider, R., Bannister, A.J., Sherriff, J., Bernstein, B.E., Emre, N.C., Schreiber, S.L., Mellor, J., and Kouzarides, T. (2002). Active genes are tri-methylated at K4 of histone H3. Nature 419, 407-411.
Scarpulla, R.C. (2008). Transcriptional paradigms in mammalian mitochondrial biogenesis and function. Physiol Rev 88, 611-638.
Seale, P., Bjork, B., Yang, W., Kajimura, S., Chin, S., Kuang, S., Scime, A., Devarakonda, S., Conroe, H.M., Erdjument-Bromage, H., et al. (2008). PRDM16 controls a brown fat/skeletal muscle switch. Nature 454, 961-967.
Seale, P., Conroe, H.M., Estall, J., Kajimura, S., Frontini, A., Ishibashi, J., Cohen, P., Cinti, S., and Spiegelman, B.M. (2011). Prdm16 determines the thermogenic program of subcutaneous white adipose tissue in mice. J Clin Invest 121, 96-105.
Seale, P., Kajimura, S., Yang, W., Chin, S., Rohas, L.M., Uldry, M., Tavernier, G., Langin, D., and Spiegelman, B.M. (2007). Transcriptional control of brown fat determination by PRDM16. Cell Metab 6, 38-54.
Seebacher, F., and Glanville, E.J. (2010). Low levels of physical activity increase metabolic responsiveness to cold in a rat (Rattus fuscipes). PLoS One 5, e13022.
Shi, Y., Lan, F., Matson, C., Mulligan, P., Whetstine, J.R., Cole, P.A., and Casero, R.A. (2004). Histone demethylation mediated by the nuclear amine oxidase homolog LSD1. Cell 119, 941-953.
Shin, H., Liu, T., Manrai, A.K., and Liu, X.S. (2009). CEAS: cis-regulatory element annotation system. Bioinformatics 25, 2605-2606.
Thorvaldsdottir, H., Robinson, J.T., and Mesirov, J.P. (2012). Integrative Genomics Viewer (IGV): high-performance genomics data visualization and exploration. Brief Bioinform.
Tiraby, C., Tavernier, G., Lefort, C., Larrouy, D., Bouillaud, F., Ricquier, D., and Langin, D. (2003). Acquirement of brown fat cell features by human white adipocytes. J Biol Chem 278, 33370-33376.
van Tienen, F.H., Lindsey, P.J., van der Kallen, C.J., and Smeets, H.J. (2010). Prolonged Nrf1 overexpression triggers adipocyte inflammation and insulin resistance. J Cell Biochem 111, 1575-1585.
Vegiopoulos, A., Muller-Decker, K., Strzoda, D., Schmitt, I., Chichelnitskiy, E., Ostertag, A., Berriel Diaz, M., Rozman, J., Hrabe de Angelis, M., Nusing, R.M., et al. (2010). Cyclooxygenase-2 controls energy homeostasis in mice by de novo recruitment of brown adipocytes. Science 328, 1158-1161.
Wang, J., Hevi, S., Kurash, J.K., Lei, H., Gay, F., Bajko, J., Su, H., Sun, W., Chang, H., Xu, G., et al. (2009a). The lysine demethylase LSD1 (KDM1) is required for maintenance of global DNA methylation. Nat Genet 41, 125-129.
Wang, J., Scully, K., Zhu, X., Cai, L., Zhang, J., Prefontaine, G.G., Krones, A., Ohgi, K.A., Zhu, P., Garcia-Bassets, I., et al. (2007). Opposing LSD1 complexes function in developmental gene activation and repression programmes. Nature 446, 882-887.
Wang, L., Jin, Q., Lee, J.E., Su, I.H., and Ge, K. (2010). Histone H3K27 methyltransferase Ezh2 represses Wnt genes to facilitate adipogenesis. Proc Natl Acad Sci U S A 107, 7317-7322.
Wang, Y., Zhang, H., Chen, Y., Sun, Y., Yang, F., Yu, W., Liang, J., Sun, L., Yang, X., Shi, L., et al. (2009b). LSD1 is a subunit of the NuRD complex and targets the metastasis programs in breast cancer. Cell 138, 660-672.
Wu, J., Bostrom, P., Sparks, L.M., Ye, L., Choi, J.H., Giang, A.H., Khandekar, M., Virtanen, K.A., Nuutila, P., Schaart, G., et al. (2012). Beige adipocytes are a distinct type of thermogenic fat cell in mouse and human. Cell 150, 366-376.
Wu, Z., Puigserver, P., Andersson, U., Zhang, C., Adelmant, G., Mootha, V., Troy, A., Cinti, S., Lowell, B., Scarpulla, R.C., et al. (1999). Mechanisms controlling mitochondrial biogenesis and respiration through the thermogenic coactivator PGC-1. Cell 98, 115-124. Young, P., Arch, J.R., and Ashwell, M. (1984). Brown adipose tissue in the parametrial fat pad of the mouse. FEBS Lett 167, 10-14.
Zhang, Y., Liu, T., Meyer, C.A., Eeckhoute, J., Johnson, D.S., Bernstein, B.E., Nusbaum, C., Myers, R.M., Brown, M., Li, W., et al. (2008). Model-based analysis of ChlP-Seq (MACS). Genome Biol 9, R137.

## Claims

1. The use of a genetically modified non-human animal as an animal model for type 2 diabetes, obesity or obesity-related disorders, wherein the amount of Lysine-specific Demethylase 1 (LSD1) in at least one tissue or at least one cell type of said animal is reduced.

2. The use of claim 1, wherein the genetically modified non-human animal has a nucleic acid inserted in its genome, wherein the presence of the inserted nucleic acid in the genome of the animal results in reduced expression of LSD1.

3. The use of claim 1 or 2, wherein said genetic modification is a disruption of an allele of the endogenous gene encoding LSD1.

4. The use of any one of the preceding claims, wherein said animal is a non-human transgenic animal which is heterozygous for the disruption of the gene encoding LSD1.

5. The use of any one of the preceding claims, wherein the amount of LSD1 is reduced in adipose tissue, liver or muscle of said animal.

6. The use of any one of the preceding claims, wherein said animal develops obesity after high fat diet.

7. The use of any one of the preceding claims, wherein said animal has at least one symptom of type 2 diabetes.

8. The use of any one of the preceding claims, wherein the animal is a rodent, preferably a mouse.

9. A method for identifying a compound useful in the treatment and/or prevention of type 2 diabetes, obesity or an obesity-related disorder, comprising (a) administering a test compound to a transgenic animal as defined in any one of claims 1 to 8, and (b) determining the effect of the test compound on the initiation, maintenance, or progression of at least one disease-related parameter in said transgenic animal, thereby identifying a compound that inhibits type 2 diabetes, obesity or an obesity-related disorder.

10. The method of claim 9, wherein said disease-related parameter is selected from the group consisting of the mass of white adipose tissue, the total mass of adipose tissue, glucose intolerance, and expression of a adipose tissue differentiation marker.

11. A compound for use in the treatment and/or prevention of type 2 diabetes, obesity or an obesity-related disorder, wherein said compound is capable of modulating LSD1 protein, the LSD1 gene or a target gene of Lsd1.

12. The compound for use according to claim 11, wherein the target gene of Lsd1 is selected from the group consisting of *Nrf1, Prdm16* and *Pgc-1*α*.*

13. A method for identifying a compound useful in the treatment and/or prevention of type 2 diabetes, obesity or an obesity-related disorder, comprising (i) contacting a cell with a test compound; (ii) determining expression level of the LSD1 gene and/or of a target gene of LSD1; and (iii) selecting the compound if the test compound is capable of increasing the expression of the LSD1 gene and/or of the target gene of LSD1, as compared to a control cell which has not been contacted with the test compound.

14. The method of claim 13, wherein the cell is an adipocyte or a preadipocyte, preferably a mouse 3T3-L1 cell or a 10T1/2 cell.

15. The use according to any one of claims 1 to 8, the method of claim 9 or 10, the LSD1 inhibitor for use according to claim 11 or 12, or the method of claim 13 or 14, wherein said obesity-related disorder is selected from the group consisting of insulin resistance, hypertension, hyperuricemia, fatty liver, non-alcoholic fatty liver disease, polycystic ovarian syndrome, acanthosis nigricans, hyperphagia, endocrine abnormalities, triglyceride storage disease, Bardet-Biedl syndrome, and Lawrence-Moon syndrome.


SEQUENCE LISTING

<110>  Universitaetsklinikum Freiburg

<120>  Animal model

<130>  LSD1

<160>  7

<170>  PatentIn version 3.5

<210>  1
<211>  2559
<212>  DNA
<213>  homo sapiens

<400>  1
atgttatctg ggaagaaggc ggcagccgcg gcggcggcgg ctgcagcggc agcaaccggg      60

acggaggctg gccctgggac agcaggcggc tccgagaacg ggtctgaggt ggccgcgcag     120

cccgcgggcc tgtcgggccc agccgaggtc gggccggggg cggtggggga gcgcacaccc     180

cgcaagaaag agcctccgcg ggcctcgccc cccgggggcc tggcggaacc gccggggtcc     240

gcagggcctc aggccggccc tactgtcgtg cctgggtctg cgacccccat ggaaactgga     300

atagcagaga ctccggaggg gcgtcggacc agccggcgca agcgggcgaa ggtagagtac     360

agagagatgg atgaaagctt ggccaacctc tcagaagatg agtattattc agaagaagag     420

agaaatgcca aagcagagaa ggaaaagaag cttcccccac caccccctca agccccacct     480

gaggaagaaa atgaaagtga gcctgaagaa ccatcgggtg tggagggcgc agctttccag     540

agccgacttc ctcatgaccg gatgacttct caagaagcag cctgttttcc agatattatc     600

agtggaccac aacagaccca gaaggttttt cttttcatta gaaaccgcac actgcagttg     660

tggttggata atccaaagat tcagctgaca tttgaggcta ctctccaaca attagaagca     720

ccttataaca gtgatactgt gcttgtccac cgagttcaca gttatttaga gcgtcatggt     780

cttatcaact tcggcatcta taagaggata aaacccctac caactaaaaa gacaggaaag     840

gtaattatta taggctctgg ggtctcaggc ttggcagcag ctcgacagtt acaaagtttt     900

ggaatggatg tcacactttt ggaagccagg gatcgtgtgg gtggacgagt tgccacattt     960

cgcaaaggaa actatgtagc tgatcttgga gccatggtgg taacaggtct tggagggaat    1020

cctatggctg tggtcagcaa acaagtaaat atggaactgg ccaagatcaa gcaaaaatgc    1080

ccactttatg aagccaacgg acaagctgtt cctaaagaga aagatgaaat ggtagagcaa    1140

gagtttaacc ggttgctaga agctacatct taccttagtc atcaactaga cttcaatgtc    1200

ctcaataata agcctgtgtc ccttggccag gcattggaag ttgtcattca gttacaagag    1260

aagcatgtca aagatgagca gattgaacat tggaagaaga tagtgaaaac tcaggaagaa    1320

ttgaaagaac ttcttaataa gatggtaaat ttgaaagaga aaattaaaga actccatcag    1380

caatacaaag aagcatctga agtaaagcca cccagagata ttactgccga gttcttagtg    1440

aaaagcaaac acagggatct gaccgcccta tgcaaggaat atgatgaatt agctgaaaca 1500

caaggaaagc tagaagaaaa acttcaggag ttggaagcga atcccccaag tgatgtatat 1560

ctctcatcaa gagacagaca aatacttgat tggcattttg caaatcttga atttgctaat 1620

gccacacctc tctcaactct ctcccttaag cactgggatc aggatgatga ctttgagttc 1680

actggcagcc acctgacagt aaggaatggc tactcgtgtg tgcctgtggc tttagcagaa 1740

ggcctagaca ttaaactgaa tacagcagtg cgacaggttc gctacacggc ttcaggatgt 1800

gaagtgatag ctgtgaatac ccgctccacg agtcaaacct ttatttataa atgcgacgca 1860

gttctctgta cccttcccct gggtgtgctg aagcagcagc caccagccgt tcagtttgtg 1920

ccacctctcc ctgagtggaa aacatctgca gtccaaagga tgggatttgg caaccttaac 1980

aaggtggtgt tgtgttttga tcgggtgttc tgggatccaa gtgtcaattt gttcgggcat 2040

gttggcagta cgactgccag caggggtgag ctcttcctct tctggaacct ctataaagct 2100

ccaatactgt tggcactagt ggcaggagaa gctgctggta tcatggaaaa cataagtgac 2160

gatgtgattg ttggccgatg cctggccatt ctcaaaggga tttttggtag cagtgcagta 2220

cctcagccca aagaaactgt ggtgtctcgt tggcgtgctg atccctgggc tcggggctct 2280

tattcctatg ttgctgcagg atcatctgga aatgactatg atttaatggc tcagccaatc 2340

actcctggcc cctcgattcc aggtgcccca cagccgattc cacgactctt ctttgcggga 2400

gaacatacga tccgtaacta cccagccaca gtgcatggtg ctctgctgag tgggctgcga 2460

gaagcgggaa gaattgcaga ccagtttttg ggggccatgt atacgctgcc tcgccaggcc 2520

acaccaggtg ttcctgcaca gcagtcccca agcatgtga 2559

<210> 2
<211> 3053
<212> DNA
<213> homo sapiens

<220>
<221> CDS
<222> (150)..(2708)

<400> 2
ggcgcggcgg gagcgcgctt ggcgcgtgcg tacgcgacgg cggttggcgg cgcgcgggca 60

gcgtgaagcg aggcgaggca aggcttttcg gacccacgga gcgacagagc gagcggcccc 120

tacggccgtc ggcggcccgg cggcccgag atg tta tct ggg aag aag gcg gca 173
Met Leu Ser Gly Lys Lys Ala Ala
1               5

gcc gcg gcg gcg gcg gct gca gcg gca gca acc ggg acg gag gct ggc 221
Ala Ala Ala Ala Ala Ala Ala Ala Ala Ala Thr Gly Thr Glu Ala Gly
    10                  15                  20

cct ggg aca gca ggc ggc tcc gag aac ggg tct gag gtg gcc gcg cag 269
Pro Gly Thr Ala Gly Gly Ser Glu Asn Gly Ser Glu Val Ala Ala Gln
25                  30                  35                  40

ccc gcg ggc ctg tcg ggc cca gcc gag gtc ggg ccg ggg gcg gtg ggg 317
Pro Ala Gly Leu Ser Gly Pro Ala Glu Val Gly Pro Gly Ala Val Gly
                45                  50                  55

gag cgc aca ccc cgc aag aaa gag cct ccg cgg gcc tcg ccc ccc ggg 365
Glu Arg Thr Pro Arg Lys Lys Glu Pro Pro Arg Ala Ser Pro Pro Gly
            60                  65                  70

ggc ctg gcg gaa ccg ccg ggg tcc gca ggg cct cag gcc ggc cct act 413
Gly Leu Ala Glu Pro Pro Gly Ser Ala Gly Pro Gln Ala Gly Pro Thr
        75                  80                  85

gtc gtg cct ggg tct gcg acc ccc atg gaa act gga ata gca gag act 461
Val Val Pro Gly Ser Ala Thr Pro Met Glu Thr Gly Ile Ala Glu Thr
    90                  95                  100

ccg gag ggg cgt cgg acc agc cgg cgc aag cgg gcg aag gta gag tac 509
Pro Glu Gly Arg Arg Thr Ser Arg Arg Lys Arg Ala Lys Val Glu Tyr
105                 110                 115                 120

aga gag atg gat gaa agc ttg gcc aac ctc tca gaa gat gag tat tat 557
Arg Glu Met Asp Glu Ser Leu Ala Asn Leu Ser Glu Asp Glu Tyr Tyr
                125                 130                 135

tca gaa gaa gag aga aat gcc aaa gca gag aag gaa aag aag ctt ccc 605
Ser Glu Glu Glu Arg Asn Ala Lys Ala Glu Lys Glu Lys Lys Leu Pro
            140                 145                 150

cca cca ccc cct caa gcc cca cct gag gaa gaa aat gaa agt gag cct 653
Pro Pro Pro Pro Gln Ala Pro Pro Glu Glu Glu Asn Glu Ser Glu Pro
        155                 160                 165

gaa gaa cca tcg ggt gtg gag ggc gca gct ttc cag agc cga ctt cct 701
Glu Glu Pro Ser Gly Val Glu Gly Ala Ala Phe Gln Ser Arg Leu Pro
    170                 175                 180

cat gac cgg atg act tct caa gaa gca gcc tgt ttt cca gat att atc 749
His Asp Arg Met Thr Ser Gln Glu Ala Ala Cys Phe Pro Asp Ile Ile
185                 190                 195                 200

agt gga cca caa cag acc cag aag gtt ttt ctt ttc att aga aac cgc 797
Ser Gly Pro Gln Gln Thr Gln Lys Val Phe Leu Phe Ile Arg Asn Arg
                205                 210                 215

aca ctg cag ttg tgg ttg gat aat cca aag att cag ctg aca ttt gag 845
Thr Leu Gln Leu Trp Leu Asp Asn Pro Lys Ile Gln Leu Thr Phe Glu
            220                 225                 230

gct act ctc caa caa tta gaa gca cct tat aac agt gat act gtg ctt 893
Ala Thr Leu Gln Gln Leu Glu Ala Pro Tyr Asn Ser Asp Thr Val Leu
        235                 240                 245

gtc cac cga gtt cac agt tat tta gag cgt cat ggt ctt atc aac ttc 941
Val His Arg Val His Ser Tyr Leu Glu Arg His Gly Leu Ile Asn Phe
    250                 255                 260

ggc atc tat aag agg ata aaa ccc cta cca act aaa aag aca gga aag 989
Gly Ile Tyr Lys Arg Ile Lys Pro Leu Pro Thr Lys Lys Thr Gly Lys
265                 270                 275                 280

gta att att ata ggc tct ggg gtc tca ggc ttg gca gca gct cga cag 1037
Val Ile Ile Ile Gly Ser Gly Val Ser Gly Leu Ala Ala Ala Arg Gln
                285                 290                 295

tta caa agt ttt gga atg gat gtc aca ctt ttg gaa gcc agg gat cgt 1085
Leu Gln Ser Phe Gly Met Asp Val Thr Leu Leu Glu Ala Arg Asp Arg

                    300                 305                 310

gtg ggt gga cga gtt gcc aca ttt cgc aaa gga aac tat gta gct gat      1133
Val Gly Gly Arg Val Ala Thr Phe Arg Lys Gly Asn Tyr Val Ala Asp
        315                 320                 325

ctt gga gcc atg gtg gta aca ggt ctt gga ggg aat cct atg gct gtg      1181
Leu Gly Ala Met Val Val Thr Gly Leu Gly Gly Asn Pro Met Ala Val
    330                 335                 340

gtc agc aaa caa gta aat atg gaa ctg gcc aag atc aag caa aaa tgc      1229
Val Ser Lys Gln Val Asn Met Glu Leu Ala Lys Ile Lys Gln Lys Cys
345                 350                 355                 360

cca ctt tat gaa gcc aac gga caa gct gtt cct aaa gag aaa gat gaa      1277
Pro Leu Tyr Glu Ala Asn Gly Gln Ala Val Pro Lys Glu Lys Asp Glu
                365                 370                 375

atg gta gag caa gag ttt aac cgg ttg cta gaa gct aca tct tac ctt      1325
Met Val Glu Gln Glu Phe Asn Arg Leu Leu Glu Ala Thr Ser Tyr Leu
            380                 385                 390

agt cat caa cta gac ttc aat gtc ctc aat aat aag cct gtg tcc ctt      1373
Ser His Gln Leu Asp Phe Asn Val Leu Asn Asn Lys Pro Val Ser Leu
        395                 400                 405

ggc cag gca ttg gaa gtt gtc att cag tta caa gag aag cat gtc aaa      1421
Gly Gln Ala Leu Glu Val Val Ile Gln Leu Gln Glu Lys His Val Lys
    410                 415                 420

gat gag cag att gaa cat tgg aag aag ata gtg aaa act cag gaa gaa      1469
Asp Glu Gln Ile Glu His Trp Lys Lys Ile Val Lys Thr Gln Glu Glu
425                 430                 435                 440

ttg aaa gaa ctt ctt aat aag atg gta aat ttg aaa gag aaa att aaa      1517
Leu Lys Glu Leu Leu Asn Lys Met Val Asn Leu Lys Glu Lys Ile Lys
                445                 450                 455

gaa ctc cat cag caa tac aaa gaa gca tct gaa gta aag cca ccc aga      1565
Glu Leu His Gln Gln Tyr Lys Glu Ala Ser Glu Val Lys Pro Pro Arg
            460                 465                 470

gat att act gcc gag ttc tta gtg aaa agc aaa cac agg gat ctg acc      1613
Asp Ile Thr Ala Glu Phe Leu Val Lys Ser Lys His Arg Asp Leu Thr
        475                 480                 485

gcc cta tgc aag gaa tat gat gaa tta gct gaa aca caa gga aag cta      1661
Ala Leu Cys Lys Glu Tyr Asp Glu Leu Ala Glu Thr Gln Gly Lys Leu
    490                 495                 500

gaa gaa aaa ctt cag gag ttg gaa gcg aat ccc cca agt gat gta tat      1709
Glu Glu Lys Leu Gln Glu Leu Glu Ala Asn Pro Pro Ser Asp Val Tyr
505                 510                 515                 520

ctc tca tca aga gac aga caa ata ctt gat tgg cat ttt gca aat ctt      1757
Leu Ser Ser Arg Asp Arg Gln Ile Leu Asp Trp His Phe Ala Asn Leu
                525                 530                 535

gaa ttt gct aat gcc aca cct ctc tca act ctc tcc ctt aag cac tgg      1805
Glu Phe Ala Asn Ala Thr Pro Leu Ser Thr Leu Ser Leu Lys His Trp
            540                 545                 550

gat cag gat gat gac ttt gag ttc act ggc agc cac ctg aca gta agg      1853
Asp Gln Asp Asp Asp Phe Glu Phe Thr Gly Ser His Leu Thr Val Arg
        555                 560                 565

aat ggc tac tcg tgt gtg cct gtg gct tta gca gaa ggc cta gac att 1901
Asn Gly Tyr Ser Cys Val Pro Val Ala Leu Ala Glu Gly Leu Asp Ile
    570                 575                 580

aaa ctg aat aca gca gtg cga cag gtt cgc tac acg gct tca gga tgt 1949
Lys Leu Asn Thr Ala Val Arg Gln Val Arg Tyr Thr Ala Ser Gly Cys
585                 590                 595                 600

gaa gtg ata gct gtg aat acc cgc tcc acg agt caa acc ttt att tat 1997
Glu Val Ile Ala Val Asn Thr Arg Ser Thr Ser Gln Thr Phe Ile Tyr
                605                 610                 615

aaa tgc gac gca gtt ctc tgt acc ctt ccc ctg ggt gtg ctg aag cag 2045
Lys Cys Asp Ala Val Leu Cys Thr Leu Pro Leu Gly Val Leu Lys Gln
            620                 625                 630

cag cca cca gcc gtt cag ttt gtg cca cct ctc cct gag tgg aaa aca 2093
Gln Pro Pro Ala Val Gln Phe Val Pro Pro Leu Pro Glu Trp Lys Thr
        635                 640                 645

tct gca gtc caa agg atg gga ttt ggc aac ctt aac aag gtg gtg ttg 2141
Ser Ala Val Gln Arg Met Gly Phe Gly Asn Leu Asn Lys Val Val Leu
    650                 655                 660

tgt ttt gat cgg gtg ttc tgg gat cca agt gtc aat ttg ttc ggg cat 2189
Cys Phe Asp Arg Val Phe Trp Asp Pro Ser Val Asn Leu Phe Gly His
665                 670                 675                 680

gtt ggc agt acg act gcc agc agg ggt gag ctc ttc ctc ttc tgg aac 2237
Val Gly Ser Thr Thr Ala Ser Arg Gly Glu Leu Phe Leu Phe Trp Asn
                685                 690                 695

ctc tat aaa gct cca ata ctg ttg gca cta gtg gca gga gaa gct gct 2285
Leu Tyr Lys Ala Pro Ile Leu Leu Ala Leu Val Ala Gly Glu Ala Ala
            700                 705                 710

ggt atc atg gaa aac ata agt gac gat gtg att gtt ggc cga tgc ctg 2333
Gly Ile Met Glu Asn Ile Ser Asp Asp Val Ile Val Gly Arg Cys Leu
        715                 720                 725

gcc att ctc aaa ggg att ttt ggt agc agt gca gta cct cag ccc aaa 2381
Ala Ile Leu Lys Gly Ile Phe Gly Ser Ser Ala Val Pro Gln Pro Lys
    730                 735                 740

gaa act gtg gtg tct cgt tgg cgt gct gat ccc tgg gct cgg ggc tct 2429
Glu Thr Val Val Ser Arg Trp Arg Ala Asp Pro Trp Ala Arg Gly Ser
745                 750                 755                 760

tat tcc tat gtt gct gca gga tca tct gga aat gac tat gat tta atg 2477
Tyr Ser Tyr Val Ala Ala Gly Ser Ser Gly Asn Asp Tyr Asp Leu Met
                765                 770                 775

gct cag cca atc act cct ggc ccc tcg att cca ggt gcc cca cag ccg 2525
Ala Gln Pro Ile Thr Pro Gly Pro Ser Ile Pro Gly Ala Pro Gln Pro
            780                 785                 790

att cca cga ctc ttc ttt gcg gga gaa cat acg atc cgt aac tac cca 2573
Ile Pro Arg Leu Phe Phe Ala Gly Glu His Thr Ile Arg Asn Tyr Pro
        795                 800                 805

gcc aca gtg cat ggt gct ctg ctg agt ggg ctg cga gaa gcg gga aga 2621
Ala Thr Val His Gly Ala Leu Leu Ser Gly Leu Arg Glu Ala Gly Arg
    810                 815                 820

att gca gac cag ttt ttg ggg gcc atg tat acg ctg cct cgc cag gcc 2669
Ile Ala Asp Gln Phe Leu Gly Ala Met Tyr Thr Leu Pro Arg Gln Ala

825                 830                 835                 840

aca cca ggt gtt cct gca cag cag tcc cca agc atg tga gacagatgca      2718
Thr Pro Gly Val Pro Ala Gln Gln Ser Pro Ser Met
                845                 850

ttctaaggga agaggcccat gtgcctgttt ctgccatgta aggaaggctc ttctagcaat      2778

actagatccc actgagaaaa tccaccctgg catctgggct cctgatcagc tgatggagct      2838

cctgatttga caaaggagct tgcctccttt gaatgaccta gagcacaggg aggaacttgt      2898

ccattagttt ggaattgtgt tcttcgtaaa gactgaggca agcaagtgct gtgaaataac      2958

atcatcttag tcccttggtg tgtggggttt ttgttttttt tttatatttt gagaataaaa      3018

cttcatataa aattggcaaa aaaaaaaaaa aaaaa                                 3053

<210>  3
<211>  852
<212>  PRT
<213>  homo sapiens

<400>  3

Met Leu Ser Gly Lys Lys Ala Ala Ala Ala Ala Ala Ala Ala Ala Ala
1               5                   10                  15

Ala Ala Thr Gly Thr Glu Ala Gly Pro Gly Thr Ala Gly Gly Ser Glu
            20                  25                  30

Asn Gly Ser Glu Val Ala Ala Gln Pro Ala Gly Leu Ser Gly Pro Ala
        35                  40                  45

Glu Val Gly Pro Gly Ala Val Gly Glu Arg Thr Pro Arg Lys Lys Glu
    50                  55                  60

Pro Pro Arg Ala Ser Pro Pro Gly Gly Leu Ala Glu Pro Pro Gly Ser
65                  70                  75                  80

Ala Gly Pro Gln Ala Gly Pro Thr Val Val Pro Gly Ser Ala Thr Pro
                85                  90                  95

Met Glu Thr Gly Ile Ala Glu Thr Pro Glu Gly Arg Arg Thr Ser Arg
            100                 105                 110

Arg Lys Arg Ala Lys Val Glu Tyr Arg Glu Met Asp Glu Ser Leu Ala
        115                 120                 125

Asn Leu Ser Glu Asp Glu Tyr Tyr Ser Glu Glu Glu Arg Asn Ala Lys
    130                 135                 140

Ala Glu Lys Glu Lys Lys Leu Pro Pro Pro Pro Pro Gln Ala Pro Pro
145                 150                 155                 160

Glu Glu Glu Asn Glu Ser Glu Pro Glu Glu Pro Ser Gly Val Glu Gly
                165                 170                 175

Ala Ala Phe Gln Ser Arg Leu Pro His Asp Arg Met Thr Ser Gln Glu
            180                 185                 190

Ala Ala Cys Phe Pro Asp Ile Ile Ser Gly Pro Gln Gln Thr Gln Lys
        195                 200                 205

Val Phe Leu Phe Ile Arg Asn Arg Thr Leu Gln Leu Trp Leu Asp Asn
    210                 215                 220

Pro Lys Ile Gln Leu Thr Phe Glu Ala Thr Leu Gln Gln Leu Glu Ala
225                 230                 235                 240

Pro Tyr Asn Ser Asp Thr Val Leu Val His Arg Val His Ser Tyr Leu
                245                 250                 255

Glu Arg His Gly Leu Ile Asn Phe Gly Ile Tyr Lys Arg Ile Lys Pro
            260                 265                 270

Leu Pro Thr Lys Lys Thr Gly Lys Val Ile Ile Ile Gly Ser Gly Val
        275                 280                 285

Ser Gly Leu Ala Ala Ala Arg Gln Leu Gln Ser Phe Gly Met Asp Val
    290                 295                 300

Thr Leu Leu Glu Ala Arg Asp Arg Val Gly Gly Arg Val Ala Thr Phe
305                 310                 315                 320

Arg Lys Gly Asn Tyr Val Ala Asp Leu Gly Ala Met Val Val Thr Gly
                325                 330                 335

Leu Gly Gly Asn Pro Met Ala Val Val Ser Lys Gln Val Asn Met Glu
            340                 345                 350

Leu Ala Lys Ile Lys Gln Lys Cys Pro Leu Tyr Glu Ala Asn Gly Gln
        355                 360                 365

Ala Val Pro Lys Glu Lys Asp Glu Met Val Glu Gln Glu Phe Asn Arg
    370                 375                 380

Leu Leu Glu Ala Thr Ser Tyr Leu Ser His Gln Leu Asp Phe Asn Val
385                 390                 395                 400

Leu Asn Asn Lys Pro Val Ser Leu Gly Gln Ala Leu Glu Val Val Ile
                405                 410                 415

Gln Leu Gln Glu Lys His Val Lys Asp Glu Gln Ile Glu His Trp Lys
            420                 425                 430

Lys Ile Val Lys Thr Gln Glu Glu Leu Lys Glu Leu Leu Asn Lys Met
        435                 440                 445

Val Asn Leu Lys Glu Lys Ile Lys Glu Leu His Gln Gln Tyr Lys Glu
    450                 455                 460

Ala Ser Glu Val Lys Pro Pro Arg Asp Ile Thr Ala Glu Phe Leu Val
465                 470                 475                 480

Lys Ser Lys His Arg Asp Leu Thr Ala Leu Cys Lys Glu Tyr Asp Glu
                485                 490                 495

Leu Ala Glu Thr Gln Gly Lys Leu Glu Glu Lys Leu Gln Glu Leu Glu
            500                 505                 510

Ala Asn Pro Pro Ser Asp Val Tyr Leu Ser Ser Arg Asp Arg Gln Ile
        515                 520                 525

Leu Asp Trp His Phe Ala Asn Leu Glu Phe Ala Asn Ala Thr Pro Leu
    530                 535                 540

Ser Thr Leu Ser Leu Lys His Trp Asp Gln Asp Asp Asp Phe Glu Phe
545                 550                 555                 560

Thr Gly Ser His Leu Thr Val Arg Asn Gly Tyr Ser Cys Val Pro Val
                565                 570                 575

Ala Leu Ala Glu Gly Leu Asp Ile Lys Leu Asn Thr Ala Val Arg Gln
            580                 585                 590

Val Arg Tyr Thr Ala Ser Gly Cys Glu Val Ile Ala Val Asn Thr Arg
        595                 600                 605

Ser Thr Ser Gln Thr Phe Ile Tyr Lys Cys Asp Ala Val Leu Cys Thr
    610                 615                 620

Leu Pro Leu Gly Val Leu Lys Gln Gln Pro Pro Ala Val Gln Phe Val
625                 630                 635                 640

Pro Pro Leu Pro Glu Trp Lys Thr Ser Ala Val Gln Arg Met Gly Phe
                645                 650                 655

Gly Asn Leu Asn Lys Val Val Leu Cys Phe Asp Arg Val Phe Trp Asp
            660                 665                 670

Pro Ser Val Asn Leu Phe Gly His Val Gly Ser Thr Thr Ala Ser Arg
        675                 680                 685

Gly Glu Leu Phe Leu Phe Trp Asn Leu Tyr Lys Ala Pro Ile Leu Leu
    690                 695                 700

Ala Leu Val Ala Gly Glu Ala Ala Gly Ile Met Glu Asn Ile Ser Asp
705                 710                 715                 720

Asp Val Ile Val Gly Arg Cys Leu Ala Ile Leu Lys Gly Ile Phe Gly
                725                 730                 735

Ser Ser Ala Val Pro Gln Pro Lys Glu Thr Val Val Ser Arg Trp Arg
            740                 745                 750

Ala Asp Pro Trp Ala Arg Gly Ser Tyr Ser Tyr Val Ala Ala Gly Ser
        755                 760                 765

Ser Gly Asn Asp Tyr Asp Leu Met Ala Gln Pro Ile Thr Pro Gly Pro
    770                 775                 780

Ser Ile Pro Gly Ala Pro Gln Pro Ile Pro Arg Leu Phe Phe Ala Gly
785                 790                 795                 800

Glu His Thr Ile Arg Asn Tyr Pro Ala Thr Val His Gly Ala Leu Leu
                805                 810                 815

Ser Gly Leu Arg Glu Ala Gly Arg Ile Ala Asp Gln Phe Leu Gly Ala
            820                 825                 830

Met Tyr Thr Leu Pro Arg Gln Ala Thr Pro Gly Val Pro Ala Gln Gln
        835                 840                 845

Ser Pro Ser Met
    850

<210>  4
<211>  3030
<212>  DNA
<213>  mus musculus

<220>
<221>  CDS
<222>  (139)..(2700)

<400>  4
gggcgcgtgc gcacgcgggg gtgtttggct tcgcacggag cgtgagaggt gcggggcgga      60

gaggcgcgag gcggctgcgg acccacggag cggcagaccg atcggcccct gcggcccgcg     120

gcggccaggc ggcccgag atg ttg tct ggg aag aag gcg gcg gcg gcg gca       171
                    Met Leu Ser Gly Lys Lys Ala Ala Ala Ala Ala
                    1               5                   10

gcg gca gcg gcg gcg gcg gcg gct gct ggg acc gag gcc ggg tcc ggg       219
Ala Ala Ala Ala Ala Ala Ala Ala Ala Gly Thr Glu Ala Gly Ser Gly
            15                  20                  25

gcg gcg ggc ggt gcc gag aac ggc tct gag gtg gcc gcg ccg ccc gcg 267
Ala Ala Gly Gly Ala Glu Asn Gly Ser Glu Val Ala Ala Pro Pro Ala
        30                  35                  40

ggc ctg acg ggc ccc acc gac atg gct acg ggg gcg gcg ggc gag cgc 315
Gly Leu Thr Gly Pro Thr Asp Met Ala Thr Gly Ala Ala Gly Glu Arg
    45                  50                  55

act ccc cga aag aag gag cct ccg cgg gcc tcg ccg ccc ggg ggc cta 363
Thr Pro Arg Lys Lys Glu Pro Pro Arg Ala Ser Pro Pro Gly Gly Leu
60                  65                  70                  75

gcc gag ccg ccg ggg tct gct ggg ccc cag gcg ggg ccc aca gcc ggg 411
Ala Glu Pro Pro Gly Ser Ala Gly Pro Gln Ala Gly Pro Thr Ala Gly
                80                  85                  90

ccc ggc tcc gcg acg ccc atg gag acc gga ata gcc gag acc ccg gag 459
Pro Gly Ser Ala Thr Pro Met Glu Thr Gly Ile Ala Glu Thr Pro Glu
            95                  100                 105

ggc cga cgg acc agc cgg cgc aag cgg gcc aag gta gaa tac aga gaa 507
Gly Arg Arg Thr Ser Arg Arg Lys Arg Ala Lys Val Glu Tyr Arg Glu
        110                 115                 120

atg gat gaa agc ttg gcc aac ctc tca gaa gat gaa tat tat tcg gaa 555
Met Asp Glu Ser Leu Ala Asn Leu Ser Glu Asp Glu Tyr Tyr Ser Glu
    125                 130                 135

gaa gaa aga aat gct aaa gca gag aag gaa aag aag ctt ccc cca cca 603
Glu Glu Arg Asn Ala Lys Ala Glu Lys Glu Lys Lys Leu Pro Pro Pro
140                 145                 150                 155

cct cct caa gcc cca cct gag gaa gaa aat gaa agt gag ccg gaa gag 651
Pro Pro Gln Ala Pro Pro Glu Glu Glu Asn Glu Ser Glu Pro Glu Glu
                160                 165                 170

ccg tct ggt gtg gag ggt gca gct ttt caa agc cga ctt ccc cat gac 699
Pro Ser Gly Val Glu Gly Ala Ala Phe Gln Ser Arg Leu Pro His Asp
            175                 180                 185

cga atg acc tct cag gaa gca gcc tgt ttc cca gac atc atc agt ggg 747
Arg Met Thr Ser Gln Glu Ala Ala Cys Phe Pro Asp Ile Ile Ser Gly
        190                 195                 200

cct cag cag aca cag aag gtt ttt ctg ttc atc agg aat cgc aca ttg 795
Pro Gln Gln Thr Gln Lys Val Phe Leu Phe Ile Arg Asn Arg Thr Leu
    205                 210                 215

cag tta tgg ctg gac aac cca aag atc cag ctg acg ttt gaa gcc act 843
Gln Leu Trp Leu Asp Asn Pro Lys Ile Gln Leu Thr Phe Glu Ala Thr
220                 225                 230                 235

ctc cag cag ctg gaa gcg cct tac aac agc gat act gtg ctt gtc cac 891
Leu Gln Gln Leu Glu Ala Pro Tyr Asn Ser Asp Thr Val Leu Val His
                240                 245                 250

cga gtt cac agt tac tta gag cgc cat ggt ctt atc aac ttc ggc atc 939
Arg Val His Ser Tyr Leu Glu Arg His Gly Leu Ile Asn Phe Gly Ile
            255                 260                 265

tac aag agg ata aaa ccc tta cca att aaa aag aca gga aag gtg att 987
Tyr Lys Arg Ile Lys Pro Leu Pro Ile Lys Lys Thr Gly Lys Val Ile
        270                 275                 280

att ata ggt tca ggt gtt tct ggc ttg gca gca gct cga cag cta cag 1035

Ile Ile Gly Ser Gly Val Ser Gly Leu Ala Ala Ala Arg Gln Leu Gln
    285                 290                 295

agt ttt ggg atg gat gtc aca ctt ctg gaa gcc agg gat cga gta ggt      1083
Ser Phe Gly Met Asp Val Thr Leu Leu Glu Ala Arg Asp Arg Val Gly
300                 305                 310                 315

gga cga gtt gct aca ttt cga aaa gga aac tat gta gct gat ctt ggc      1131
Gly Arg Val Ala Thr Phe Arg Lys Gly Asn Tyr Val Ala Asp Leu Gly
                320                 325                 330

gcc atg gtt gta aca ggt ctt gga ggg aat ccc atg gct gtc gtc agc      1179
Ala Met Val Val Thr Gly Leu Gly Gly Asn Pro Met Ala Val Val Ser
            335                 340                 345

aaa caa gta aat atg gaa ctg gcc aag atc aag caa aaa tgc cca ctt      1227
Lys Gln Val Asn Met Glu Leu Ala Lys Ile Lys Gln Lys Cys Pro Leu
        350                 355                 360

tat gaa gcc aat gga caa gct gtt cca aaa gaa aaa gat gaa atg gta      1275
Tyr Glu Ala Asn Gly Gln Ala Val Pro Lys Glu Lys Asp Glu Met Val
    365                 370                 375

gaa caa gaa ttt aac cgg ttg cta gaa gcc act tct tac ctt agt cac      1323
Glu Gln Glu Phe Asn Arg Leu Leu Glu Ala Thr Ser Tyr Leu Ser His
380                 385                 390                 395

cag tta gac ttc aac gtc ctc aat aat aaa cct gta tcc ctt ggc cag      1371
Gln Leu Asp Phe Asn Val Leu Asn Asn Lys Pro Val Ser Leu Gly Gln
                400                 405                 410

gca ttg gag gtt gtc att cag ctg caa gaa aag cat gtc aaa gat gag      1419
Ala Leu Glu Val Val Ile Gln Leu Gln Glu Lys His Val Lys Asp Glu
            415                 420                 425

cag att gaa cat tgg aag aag ata gtg aaa act cag gag gag ttg aaa      1467
Gln Ile Glu His Trp Lys Lys Ile Val Lys Thr Gln Glu Glu Leu Lys
        430                 435                 440

gag ctt ctt aat aag atg gta aat ttg aag gag aaa att aaa gag ctc      1515
Glu Leu Leu Asn Lys Met Val Asn Leu Lys Glu Lys Ile Lys Glu Leu
    445                 450                 455

cat cag caa tac aaa gaa gct tca gaa gtg aag ccg ccc aga gat atc      1563
His Gln Gln Tyr Lys Glu Ala Ser Glu Val Lys Pro Pro Arg Asp Ile
460                 465                 470                 475

aca gcc gag ttc ctg gtg aag agc aag cac agg gac ctg act gcc ctc      1611
Thr Ala Glu Phe Leu Val Lys Ser Lys His Arg Asp Leu Thr Ala Leu
                480                 485                 490

tgc aag gaa tat gat gaa tta gct gaa aca caa gga aag cta gaa gaa      1659
Cys Lys Glu Tyr Asp Glu Leu Ala Glu Thr Gln Gly Lys Leu Glu Glu
            495                 500                 505

aaa ctt caa gaa ttg gaa gcc aat ccc cca agt gat gta tac ctc tca      1707
Lys Leu Gln Glu Leu Glu Ala Asn Pro Pro Ser Asp Val Tyr Leu Ser
        510                 515                 520

tca aga gac aga caa ata ctt gac tgg cat ttt gca aat ctt gaa ttt      1755
Ser Arg Asp Arg Gln Ile Leu Asp Trp His Phe Ala Asn Leu Glu Phe
    525                 530                 535

gcc aac gcc aca cct ctc tct acc ctc tct ctt aaa cat tgg gat cag      1803
Ala Asn Ala Thr Pro Leu Ser Thr Leu Ser Leu Lys His Trp Asp Gln
540                 545                 550                 555

gat gat gac ttt gag ttt act gga agc cac ctg aca gta agg aat ggc 1851
Asp Asp Asp Phe Glu Phe Thr Gly Ser His Leu Thr Val Arg Asn Gly
                560                 565                 570

tac tca tgt gtg cct gtg gct tta gct gaa ggc ttg gac att aaa ctg 1899
Tyr Ser Cys Val Pro Val Ala Leu Ala Glu Gly Leu Asp Ile Lys Leu
            575                 580                 585

aac aca gca gtg cgg cag gtt cgc tac aca gcc tca gga tgt gaa gtg 1947
Asn Thr Ala Val Arg Gln Val Arg Tyr Thr Ala Ser Gly Cys Glu Val
        590                 595                 600

att gct gtg aac aca cgt tcc aca agt caa acc ttt att tat aag tgt 1995
Ile Ala Val Asn Thr Arg Ser Thr Ser Gln Thr Phe Ile Tyr Lys Cys
    605                 610                 615

gat gca gtt ctc tgt aca ctt cct ttg gga gtg ttg aag cag cag cca 2043
Asp Ala Val Leu Cys Thr Leu Pro Leu Gly Val Leu Lys Gln Gln Pro
620                 625                 630                 635

cca gct gtt cag ttt gtg cca cct ctt cct gag tgg aaa aca tct gca 2091
Pro Ala Val Gln Phe Val Pro Pro Leu Pro Glu Trp Lys Thr Ser Ala
                640                 645                 650

gtc caa agg atg gga ttt ggc aac ctt aac aag gtg gtg tta tgc ttt 2139
Val Gln Arg Met Gly Phe Gly Asn Leu Asn Lys Val Val Leu Cys Phe
            655                 660                 665

gac cgt gtg ttc tgg gac cca agt gtc aat ttg ttt ggg cac gtt ggc 2187
Asp Arg Val Phe Trp Asp Pro Ser Val Asn Leu Phe Gly His Val Gly
        670                 675                 680

agt aca act gct agc agg ggt gag ctc ttc ctc ttc tgg aac cta tat 2235
Ser Thr Thr Ala Ser Arg Gly Glu Leu Phe Leu Phe Trp Asn Leu Tyr
    685                 690                 695

aaa gct cca ata cta ttg gcc ctg gta gca gga gaa gct gct ggc att 2283
Lys Ala Pro Ile Leu Leu Ala Leu Val Ala Gly Glu Ala Ala Gly Ile
700                 705                 710                 715

atg gag aac att agt gat gat gtg att gtc ggc cgg tgc ctg gcc att 2331
Met Glu Asn Ile Ser Asp Asp Val Ile Val Gly Arg Cys Leu Ala Ile
                720                 725                 730

ctc aaa ggg att ttt ggc agc agt gca gtc cca cag ccc aag gaa act 2379
Leu Lys Gly Ile Phe Gly Ser Ser Ala Val Pro Gln Pro Lys Glu Thr
            735                 740                 745

gtg gta tct cgt tgg cgt gct gat ccg tgg gcc cgg ggc tcc tat tct 2427
Val Val Ser Arg Trp Arg Ala Asp Pro Trp Ala Arg Gly Ser Tyr Ser
        750                 755                 760

tat gtg gct gca gga tcc tct gga aat gac tat gat tta atg gct cag 2475
Tyr Val Ala Ala Gly Ser Ser Gly Asn Asp Tyr Asp Leu Met Ala Gln
    765                 770                 775

ccg atc act cct ggc ccc tca att cca ggt gcc cca cag cca atc cca 2523
Pro Ile Thr Pro Gly Pro Ser Ile Pro Gly Ala Pro Gln Pro Ile Pro
780                 785                 790                 795

aga ctc ttc ttt gct gga gaa cac aca atc cgg aac tac cca gct aca 2571
Arg Leu Phe Phe Ala Gly Glu His Thr Ile Arg Asn Tyr Pro Ala Thr
                800                 805                 810

gtc cat ggt gct ctg ttg agt ggg ctt cga gaa gca gga agg att gcc 2619

Val His Gly Ala Leu Leu Ser Gly Leu Arg Glu Ala Gly Arg Ile Ala
            815                 820                 825

gac cag ttt ttg gga gcc atg tac act ttg cct cgt cag gcc aca cca 2667
Asp Gln Phe Leu Gly Ala Met Tyr Thr Leu Pro Arg Gln Ala Thr Pro
        830                 835                 840

ggt gtc cct gca cag cag tcc cca agt atg tga gacagatggt tctgaacaga 2720
Gly Val Pro Ala Gln Gln Ser Pro Ser Met
    845                 850

gagatccaac ggcatgtcat ctgccacgta agcaagctct tctagcaata ctagatccta 2780

ctgagaaact ccatgtcatc agctactggg actcctagtt tgacagcaga ggctggctcc 2840

tttggctgac agcaacttac ccattgattt ggaagtacag ctccataaag actgctcgag 2900

aagcaagtgg tgtgagataa cctcttagtc tatggtgttt gtttgttttt gttttttttt 2960

aatatatttt gagaataaaa ctttaaaata attttatatg aaaatttatt tttaaaaaaa 3020

aaaaaaaaaa 3030

<210> 5
<211> 853
<212> PRT
<213> mus musculus

<400> 5

Met Leu Ser Gly Lys Lys Ala Ala Ala Ala Ala Ala Ala Ala Ala Ala
1               5                   10                  15

Ala Ala Ala Ala Gly Thr Glu Ala Gly Ser Gly Ala Ala Gly Gly Ala
            20                  25                  30

Glu Asn Gly Ser Glu Val Ala Ala Pro Pro Ala Gly Leu Thr Gly Pro
        35                  40                  45

Thr Asp Met Ala Thr Gly Ala Ala Gly Glu Arg Thr Pro Arg Lys Lys
    50                  55                  60

Glu Pro Pro Arg Ala Ser Pro Pro Gly Gly Leu Ala Glu Pro Pro Gly
65                  70                  75                  80

Ser Ala Gly Pro Gln Ala Gly Pro Thr Ala Gly Pro Gly Ser Ala Thr
                85                  90                  95

Pro Met Glu Thr Gly Ile Ala Glu Thr Pro Glu Gly Arg Arg Thr Ser
            100                 105                 110

Arg Arg Lys Arg Ala Lys Val Glu Tyr Arg Glu Met Asp Glu Ser Leu
        115                 120                 125

Ala Asn Leu Ser Glu Asp Glu Tyr Tyr Ser Glu Glu Glu Arg Asn Ala
    130                 135                 140

Lys Ala Glu Lys Glu Lys Lys Leu Pro Pro Pro Pro Pro Gln Ala Pro
145                 150                 155                 160

Pro Glu Glu Glu Asn Glu Ser Glu Pro Glu Glu Pro Ser Gly Val Glu
                165                 170                 175

Gly Ala Ala Phe Gln Ser Arg Leu Pro His Asp Arg Met Thr Ser Gln
            180                 185                 190

Glu Ala Ala Cys Phe Pro Asp Ile Ile Ser Gly Pro Gln Gln Thr Gln
        195                 200                 205

Lys Val Phe Leu Phe Ile Arg Asn Arg Thr Leu Gln Leu Trp Leu Asp
    210                 215                 220

Asn Pro Lys Ile Gln Leu Thr Phe Glu Ala Thr Leu Gln Gln Leu Glu
225                 230                 235                 240

Ala Pro Tyr Asn Ser Asp Thr Val Leu Val His Arg Val His Ser Tyr
                245                 250                 255

Leu Glu Arg His Gly Leu Ile Asn Phe Gly Ile Tyr Lys Arg Ile Lys
            260                 265                 270

Pro Leu Pro Ile Lys Lys Thr Gly Lys Val Ile Ile Ile Gly Ser Gly
        275                 280                 285

Val Ser Gly Leu Ala Ala Ala Arg Gln Leu Gln Ser Phe Gly Met Asp
    290                 295                 300

Val Thr Leu Leu Glu Ala Arg Asp Arg Val Gly Gly Arg Val Ala Thr
305                 310                 315                 320

Phe Arg Lys Gly Asn Tyr Val Ala Asp Leu Gly Ala Met Val Val Thr
                325                 330                 335

Gly Leu Gly Gly Asn Pro Met Ala Val Val Ser Lys Gln Val Asn Met
            340                 345                 350

Glu Leu Ala Lys Ile Lys Gln Lys Cys Pro Leu Tyr Glu Ala Asn Gly
        355                 360                 365

Gln Ala Val Pro Lys Glu Lys Asp Glu Met Val Glu Gln Glu Phe Asn
    370                 375                 380

Arg Leu Leu Glu Ala Thr Ser Tyr Leu Ser His Gln Leu Asp Phe Asn
385                 390                 395                 400

Val Leu Asn Asn Lys Pro Val Ser Leu Gly Gln Ala Leu Glu Val Val

                405                 410                 415

Ile Gln Leu Gln Glu Lys His Val Lys Asp Glu Gln Ile Glu His Trp
            420                 425                 430

Lys Lys Ile Val Lys Thr Gln Glu Glu Leu Lys Glu Leu Leu Asn Lys
        435                 440                 445

Met Val Asn Leu Lys Glu Lys Ile Lys Glu Leu His Gln Gln Tyr Lys
    450                 455                 460

Glu Ala Ser Glu Val Lys Pro Pro Arg Asp Ile Thr Ala Glu Phe Leu
465                 470                 475                 480

Val Lys Ser Lys His Arg Asp Leu Thr Ala Leu Cys Lys Glu Tyr Asp
                485                 490                 495

Glu Leu Ala Glu Thr Gln Gly Lys Leu Glu Glu Lys Leu Gln Glu Leu
            500                 505                 510

Glu Ala Asn Pro Pro Ser Asp Val Tyr Leu Ser Ser Arg Asp Arg Gln
        515                 520                 525

Ile Leu Asp Trp His Phe Ala Asn Leu Glu Phe Ala Asn Ala Thr Pro
    530                 535                 540

Leu Ser Thr Leu Ser Leu Lys His Trp Asp Gln Asp Asp Asp Phe Glu
545                 550                 555                 560

Phe Thr Gly Ser His Leu Thr Val Arg Asn Gly Tyr Ser Cys Val Pro
                565                 570                 575

Val Ala Leu Ala Glu Gly Leu Asp Ile Lys Leu Asn Thr Ala Val Arg
            580                 585                 590

Gln Val Arg Tyr Thr Ala Ser Gly Cys Glu Val Ile Ala Val Asn Thr
        595                 600                 605

Arg Ser Thr Ser Gln Thr Phe Ile Tyr Lys Cys Asp Ala Val Leu Cys
    610                 615                 620

Thr Leu Pro Leu Gly Val Leu Lys Gln Gln Pro Pro Ala Val Gln Phe
625                 630                 635                 640

Val Pro Pro Leu Pro Glu Trp Lys Thr Ser Ala Val Gln Arg Met Gly
                645                 650                 655

Phe Gly Asn Leu Asn Lys Val Val Leu Cys Phe Asp Arg Val Phe Trp
            660                 665                 670

Asp Pro Ser Val Asn Leu Phe Gly His Val Gly Ser Thr Thr Ala Ser
        675                 680                 685

Arg Gly Glu Leu Phe Leu Phe Trp Asn Leu Tyr Lys Ala Pro Ile Leu
    690                 695                 700

Leu Ala Leu Val Ala Gly Glu Ala Ala Gly Ile Met Glu Asn Ile Ser
705                 710                 715                 720

Asp Asp Val Ile Val Gly Arg Cys Leu Ala Ile Leu Lys Gly Ile Phe
                725                 730                 735

Gly Ser Ser Ala Val Pro Gln Pro Lys Glu Thr Val Val Ser Arg Trp
            740                 745                 750

Arg Ala Asp Pro Trp Ala Arg Gly Ser Tyr Ser Tyr Val Ala Ala Gly
        755                 760                 765

Ser Ser Gly Asn Asp Tyr Asp Leu Met Ala Gln Pro Ile Thr Pro Gly
    770                 775                 780

Pro Ser Ile Pro Gly Ala Pro Gln Pro Ile Pro Arg Leu Phe Phe Ala
785                 790                 795                 800

Gly Glu His Thr Ile Arg Asn Tyr Pro Ala Thr Val His Gly Ala Leu
                805                 810                 815

Leu Ser Gly Leu Arg Glu Ala Gly Arg Ile Ala Asp Gln Phe Leu Gly
            820                 825                 830

Ala Met Tyr Thr Leu Pro Arg Gln Ala Thr Pro Gly Val Pro Ala Gln
        835                 840                 845

Gln Ser Pro Ser Met
    850

<210>  6
<211>  8717
<212>  DNA
<213>  artificial sequence

<220>
<223>  pBS-ROSA26-AOF2

<220>
<221>  promoter
<222>  (625)..(645)
<223>  T7 promoter

<220>
<221>  misc_feature
<222>  (1018)..(1381)

<223>  HS4 insulator

<220>
<221>  promoter
<222>  (1414)..(2225)
<223>  ROSA26 promoter

<220>
<221>  Intron
<222>  (2311)..(2878)
<223>  rabbit beta-globin intron

<220>
<221>  misc_feature
<222>  (3079)..(3106)
<223>  sequence encoding FLAG tag

<220>
<221>  CDS
<222>  (3079)..(5667)

<220>
<221>  misc_feature
<222>  (3112)..(5667)
<223>  seqence encoding human LSD1

<220>
<221>  polyA_site
<222>  (5686)..(5819)
<223>  SV40 poly A site

<220>
<221>  misc_feature
<222>  (5896)..(6434)
<223>  HS4 insulator

<220>
<221>  misc_feature
<222>  (6914)..(7581)
<223>  pUC origin

<220>
<221>  misc_feature
<222>  (8589)..(7729)
<223>  sequence encoding beta-lactamase

<400>  6
ctaaattgta agcgttaata ttttgttaaa attcgcgtta aatttttgtt aaatcagctc      60

attttttaac caataggccg aaatcggcaa aatcccttat aaatcaaaag aatagaccga      120

gatagggttg agtgttgttc cagtttggaa caagagtcca ctattaaaga acgtggactc      180

caacgtcaaa gggcgaaaaa ccgtctatca gggcgatggc ccactacgtg aaccatcacc      240

ctaatcaagt tttttggggt cgaggtgccg taaagcacta aatcggaacc ctaaagggag      300

cccccgattt agagcttgac ggggaaagcc ggcgaacgtg gcgagaaagg aagggaagaa      360

agcgaaagga gcgggcgcta gggcgctggc aagtgtagcg gtcacgctgc gcgtaaccac      420

cacacccgcc gcgcttaatg cgccgctaca gggcgcgtcc cattcgccat tcaggctgcg      480

caactgttgg gaagggcgat cggtgcgggc ctcttcgcta ttacgccagc tggcgaaagg      540

gggatgtgct gcaaggcgat taagttgggt aacgccaggg ttttcccagt cacgacgttg      600

taaaacgacg gccagtgagc gcgcgtaata cgactcacta tagggcgaat tgggtaccgg 660
gccccccctc gaggtcgacg gtatcgataa gcttgattcg agctctgtac atgtccgcgg 720
tcgcgacgta cgcgtatcga tggcgccagc tgcaggcggc cgccatatgc atcctaggcc 780
tattaatatt ccggagtata cgtagccggc taacgttaac aaccggtacc gagttggcgc 840
gcctgggagc tcacggggac agcccccccc caaagccccc agggatgtaa ttacgtccct 900
cccccgctag ggggcagcag cgagccgccc ggggctccgc tccggtccgg cgctcccccc 960
gcatccccga gccggcagcg tgcggggaca gcccgggcac ggggaaggtg gcacgggatc 1020
gctttcctct gaacgcttct cgctgctctt tgagcctgca gacacctggg gggatacggg 1080
gaaaaagctt taggctgaaa gagagattta gaatgacggg cgcgcctggg agctcacggg 1140
gacagccccc ccccaaagcc cccagggatg taattacgtc cctcccccgc tagggggcag 1200
cagcgagccg cccggggctc cgctccggtc cggcgctccc cccgcatccc cgagccggca 1260
gcgtgcgggg acagcccggg cacggggaag gtggcacggg atcgctttcc tctgaacgct 1320
tctcgctgct ctttgagcct gcagacacct ggggggatac ggggaaaaag ctgggcgcgc 1380
caattaaccc tcactaaagg gggtacctct agtcgactag atgaaggaga gcctttctct 1440
ctgggcaaga gcggtgcaat ggtgtgtaaa ggtagctgag aagacgaaaa gggcaagcat 1500
cttcctgcta ccaggctggg gaggcccagg cccacgaccc cgaggagagg gaacgcaggg 1560
agactgaggt gacccttctt tcccccgggg cccggtcgtg tggttcggtg tctcttttct 1620
gttggaccct taccttgacc caggcgctgc cggggcctgg gcccgggctg cggcgcacgg 1680
cactcccggg aggcagcgag actcgagtta ggcccaacgc ggcgccacgg cgtttcctgg 1740
ccgggaatgg cccgtacccg tgaggtgggg gtggggggca gaaaaggcgg agcgagcccg 1800
aggcggggag ggggagggcc aggggcggag ggggccggca ctactgtgtt ggcggactgg 1860
cgggactagg gctgcgtgag tctctgagcg caggcgggcg gcggccgccc ctcccccggc 1920
ggcggcagcg gcggcagcgg cggcagctca ctcagcccgc tgcccgagcg gaaacgccac 1980
tgaccgcacg gggattccca gtgccggcgc caggggcacg cgggacacgc cccctcccgc 2040
cgcgccattg gcctctccgc ccaccgcccc acacttattg gccggtgcgc cgccaatcag 2100
cggaggctgc cggggccgcc taaagaagag gctgtgcttt ggggctccgg ctcctcagag 2160
agcctcggct aggtagggga tcgggactct ggcgggaggg cggcttggtg cgtttgcggg 2220
gatccactag ttctagaact atagctagca tgcgcaaatt taaagcgctg atatcgatcg 2280
cgcgcagatc ctaagaactt ccaggggagg tttggggacc cttgattgtt ctttcttttt 2340
cgctattgta aaattcatgt tatatggagg gggcaaagtt ttcagggtgt tgtttagaat 2400
gggaagatgt cccttgtatc accatggacc ctcatgataa ttttgtttct ttcactttct 2460
actctgttga caaccattgt ctcctcttat tttcttttca ttttctgtaa ctttttcgtt 2520
aaactttagc ttgcatttgt aacgaatttt taaattcact tttgtttatt tgtcagattg 2580

taagtacttt ctctaatcac ttttttttca aggcaatcag ggtatattat attgtacttc 2640

agcacagttt tagagaacaa ttgttataat taaatgataa ggtagaatat ttctgcatat 2700

aaattctggc tggcgtggaa atattcttat tggtagaaac aactacaccc tggtcatcat 2760

cctgcctttc tctttatggt tacaatgata tacactgttt gagatgagga taaaatactc 2820

tgagtccaaa ccgggcccct ctgctaacca tgttcatgcc ttcttctctt tcctacagct 2880

cctgggcaac gtgctggtta tgtgctgtct catcaaatgg caaagaattc atggctggtg 2940

accacgtcgt ggaatgcctt cgaattcagc acctgcacat gggacgtcga cctgaggtaa 3000

ttataacccg ggccctatat atggatccag atcgatcatc aggatcggta ccgggccccc 3060

cctcgagaag cttccacc atg gac tac aag gac gac gat gac aag gaa ttc 3111
Met Asp Tyr Lys Asp Asp Asp Asp Lys Glu Phe
1               5                   10

tta tct ggg aag aag gcg gca gcc gcg gcg gcg gcg gct gca gcg gca 3159
Leu Ser Gly Lys Lys Ala Ala Ala Ala Ala Ala Ala Ala Ala Ala Ala
            15                  20                  25

gca acc ggg acg gag gct ggc cct ggg aca gca ggc ggc tcc gag aac 3207
Ala Thr Gly Thr Glu Ala Gly Pro Gly Thr Ala Gly Gly Ser Glu Asn
        30                  35                  40

ggg tct gag gtg gcc gcg cag ccc gcg ggc ctg tcg ggc cca gcc gag 3255
Gly Ser Glu Val Ala Ala Gln Pro Ala Gly Leu Ser Gly Pro Ala Glu
    45                  50                  55

gtc ggg ccg ggg gcg gtg ggg gag cgc aca ccc cgc aag aaa gag cct 3303
Val Gly Pro Gly Ala Val Gly Glu Arg Thr Pro Arg Lys Lys Glu Pro
60                  65                  70                  75

ccg cgg gcc tcg ccc ccc ggg ggc ctg gcg gaa ccg ccg ggg tcc gca 3351
Pro Arg Ala Ser Pro Pro Gly Gly Leu Ala Glu Pro Pro Gly Ser Ala
                80                  85                  90

ggg cct cag gcc ggc cct act gtc gtg cct ggg tct gcg acc ccc atg 3399
Gly Pro Gln Ala Gly Pro Thr Val Val Pro Gly Ser Ala Thr Pro Met
            95                  100                 105

gaa act gga ata gca gag act ccg gag ggg cgt cgg acc agc cgg cgc 3447
Glu Thr Gly Ile Ala Glu Thr Pro Glu Gly Arg Arg Thr Ser Arg Arg
        110                 115                 120

aag cgg gcg aag gta gag tac aga gag atg gat gaa agc ttg gcc aac 3495
Lys Arg Ala Lys Val Glu Tyr Arg Glu Met Asp Glu Ser Leu Ala Asn
    125                 130                 135

ctc tca gaa gat gag tat tat tca gaa gaa gag aga aat gcc aaa gca 3543
Leu Ser Glu Asp Glu Tyr Tyr Ser Glu Glu Glu Arg Asn Ala Lys Ala
140                 145                 150                 155

gag aag gaa aag aag ctt ccc cca cca ccc cct caa gcc cca cct gag 3591
Glu Lys Glu Lys Lys Leu Pro Pro Pro Pro Pro Gln Ala Pro Pro Glu
                160                 165                 170

gaa gaa aat gaa agt gag cct gaa gaa cca tcg ggt gtg gag ggc gca 3639
Glu Glu Asn Glu Ser Glu Pro Glu Glu Pro Ser Gly Val Glu Gly Ala
            175                 180                 185

gct ttc cag agc cga ctt cct cat gac cgg atg act tct caa gaa gca 3687

Ala Phe Gln Ser Arg Leu Pro His Asp Arg Met Thr Ser Gln Glu Ala
        190                 195                 200

gcc tgt ttt cca gat att atc agt gga cca caa cag acc cag aag gtt        3735
Ala Cys Phe Pro Asp Ile Ile Ser Gly Pro Gln Gln Thr Gln Lys Val
    205                 210                 215

ttt ctt ttc att aga aac cgc aca ctg cag ttg tgg ttg gat aat cca        3783
Phe Leu Phe Ile Arg Asn Arg Thr Leu Gln Leu Trp Leu Asp Asn Pro
220                 225                 230                 235

aag att cag ctg aca ttt gag gct act ctc caa caa tta gaa gca cct        3831
Lys Ile Gln Leu Thr Phe Glu Ala Thr Leu Gln Gln Leu Glu Ala Pro
                240                 245                 250

tat aac agt gat act gtg ctt gtc cac cga gtt cac agt tat tta gag        3879
Tyr Asn Ser Asp Thr Val Leu Val His Arg Val His Ser Tyr Leu Glu
            255                 260                 265

cgt cat ggt ctt atc aac ttc ggc atc tat aag agg ata aaa ccc cta        3927
Arg His Gly Leu Ile Asn Phe Gly Ile Tyr Lys Arg Ile Lys Pro Leu
        270                 275                 280

cca act aaa aag aca gga aag gta att att ata ggc tct ggg gtc tca        3975
Pro Thr Lys Lys Thr Gly Lys Val Ile Ile Ile Gly Ser Gly Val Ser
    285                 290                 295

ggc ttg gca gca gct cga cag tta caa agt ttt gga atg gat gtc aca        4023
Gly Leu Ala Ala Ala Arg Gln Leu Gln Ser Phe Gly Met Asp Val Thr
300                 305                 310                 315

ctt ttg gaa gcc agg gat cgt gtg ggt gga cga gtt gcc aca ttt cgc        4071
Leu Leu Glu Ala Arg Asp Arg Val Gly Gly Arg Val Ala Thr Phe Arg
                320                 325                 330

aaa gga aac tat gta gct gat ctt gga gcc atg gtg gta aca ggt ctt        4119
Lys Gly Asn Tyr Val Ala Asp Leu Gly Ala Met Val Val Thr Gly Leu
            335                 340                 345

gga ggg aat cct atg gct gtg gtc agc aaa caa gta aat atg gaa ctg        4167
Gly Gly Asn Pro Met Ala Val Val Ser Lys Gln Val Asn Met Glu Leu
        350                 355                 360

gcc aag atc aag caa aaa tgc cca ctt tat gaa gcc aac gga caa gct        4215
Ala Lys Ile Lys Gln Lys Cys Pro Leu Tyr Glu Ala Asn Gly Gln Ala
    365                 370                 375

gtt cct aaa gag aaa gat gaa atg gta gag caa gag ttt aac cgg ttg        4263
Val Pro Lys Glu Lys Asp Glu Met Val Glu Gln Glu Phe Asn Arg Leu
380                 385                 390                 395

cta gaa gct aca tct tac ctt agt cat caa cta gac ttc aat gtc ctc        4311
Leu Glu Ala Thr Ser Tyr Leu Ser His Gln Leu Asp Phe Asn Val Leu
                400                 405                 410

aat aat aag cct gtg tcc ctt ggc cag gca ttg gaa gtt gtc att cag        4359
Asn Asn Lys Pro Val Ser Leu Gly Gln Ala Leu Glu Val Val Ile Gln
            415                 420                 425

tta caa gag aag cat gtc aaa gat gag cag att gaa cat tgg aag aag        4407
Leu Gln Glu Lys His Val Lys Asp Glu Gln Ile Glu His Trp Lys Lys
        430                 435                 440

ata gtg aaa act cag gaa gaa ttg aaa gaa ctt ctt aat aag atg gta        4455
Ile Val Lys Thr Gln Glu Glu Leu Lys Glu Leu Leu Asn Lys Met Val
    445                 450                 455

aat ttg aaa gag aaa att aaa gaa ctc cat cag caa tac aaa gaa gca 4503
Asn Leu Lys Glu Lys Ile Lys Glu Leu His Gln Gln Tyr Lys Glu Ala
460                 465                 470                 475

tct gaa gta aag cca ccc aga gat att act gcc gag ttc tta gtg aaa 4551
Ser Glu Val Lys Pro Pro Arg Asp Ile Thr Ala Glu Phe Leu Val Lys
                480                 485                 490

agc aaa cac agg gat ctg acc gcc cta tgc aag gaa tat gat gaa tta 4599
Ser Lys His Arg Asp Leu Thr Ala Leu Cys Lys Glu Tyr Asp Glu Leu
            495                 500                 505

gct gaa aca caa gga aag cta gaa gaa aaa ctt cag gag ttg gaa gcg 4647
Ala Glu Thr Gln Gly Lys Leu Glu Glu Lys Leu Gln Glu Leu Glu Ala
        510                 515                 520

aat ccc cca agt gat gta tat ctc tca tca aga gac aga caa ata ctt 4695
Asn Pro Pro Ser Asp Val Tyr Leu Ser Ser Arg Asp Arg Gln Ile Leu
    525                 530                 535

gat tgg cat ttt gca aat ctt gaa ttt gct aat gcc aca cct ctc tca 4743
Asp Trp His Phe Ala Asn Leu Glu Phe Ala Asn Ala Thr Pro Leu Ser
540                 545                 550                 555

act ctc tcc ctt aag cac tgg gat cag gat gat gac ttt gag ttc act 4791
Thr Leu Ser Leu Lys His Trp Asp Gln Asp Asp Asp Phe Glu Phe Thr
                560                 565                 570

ggc agc cac ctg aca gta agg aat ggc tac tcg tgt gtg cct gtg gct 4839
Gly Ser His Leu Thr Val Arg Asn Gly Tyr Ser Cys Val Pro Val Ala
            575                 580                 585

tta gca gaa ggc cta gac att aaa ctg aat aca gca gtg cga cag gtt 4887
Leu Ala Glu Gly Leu Asp Ile Lys Leu Asn Thr Ala Val Arg Gln Val
        590                 595                 600

cgc tac acg gct tca gga tgt gaa gtg ata gct gtg aat acc cgc tcc 4935
Arg Tyr Thr Ala Ser Gly Cys Glu Val Ile Ala Val Asn Thr Arg Ser
    605                 610                 615

acg agt caa acc ttt att tat aaa tgc gac gca gtt ctc tgt acc ctt 4983
Thr Ser Gln Thr Phe Ile Tyr Lys Cys Asp Ala Val Leu Cys Thr Leu
620                 625                 630                 635

ccc ctg ggt gtg ctg aag cag cag cca cca gcc gtt cag ttt gtg cca 5031
Pro Leu Gly Val Leu Lys Gln Gln Pro Pro Ala Val Gln Phe Val Pro
                640                 645                 650

cct ctc cct gag tgg aaa aca tct gca gtc caa agg atg gga ttt ggc 5079
Pro Leu Pro Glu Trp Lys Thr Ser Ala Val Gln Arg Met Gly Phe Gly
            655                 660                 665

aac ctt aac aag gtg gtg ttg tgt ttt gat cgg gtg ttc tgg gat cca 5127
Asn Leu Asn Lys Val Val Leu Cys Phe Asp Arg Val Phe Trp Asp Pro
        670                 675                 680

agt gtc aat ttg ttc ggg cat gtt ggc agt acg act gcc agc agg ggt 5175
Ser Val Asn Leu Phe Gly His Val Gly Ser Thr Thr Ala Ser Arg Gly
    685                 690                 695

gag ctc ttc ctc ttc tgg aac ctc tat aaa gct cca ata ctg ttg gca 5223
Glu Leu Phe Leu Phe Trp Asn Leu Tyr Lys Ala Pro Ile Leu Leu Ala
700                 705                 710                 715

cta gtg gca gga gaa gct gct ggt atc atg gaa aac ata agt gac gat 5271

Leu Val Ala Gly Glu Ala Ala Gly Ile Met Glu Asn Ile Ser Asp Asp
                720                 725                 730

gtg att gtt ggc cga tgc ctg gcc att ctc aaa ggg att ttt ggt agc 5319
Val Ile Val Gly Arg Cys Leu Ala Ile Leu Lys Gly Ile Phe Gly Ser
            735                 740                 745

agt gca gta cct cag ccc aaa gaa act gtg gtg tct cgt tgg cgt gct 5367
Ser Ala Val Pro Gln Pro Lys Glu Thr Val Val Ser Arg Trp Arg Ala
        750                 755                 760

gat ccc tgg gct cgg ggc tct tat tcc tat gtt gct gca gga tca tct 5415
Asp Pro Trp Ala Arg Gly Ser Tyr Ser Tyr Val Ala Ala Gly Ser Ser
    765                 770                 775

gga aat gac tat gat tta atg gct cag cca atc act cct ggc ccc tcg 5463
Gly Asn Asp Tyr Asp Leu Met Ala Gln Pro Ile Thr Pro Gly Pro Ser
780                 785                 790                 795

att cca ggt gcc cca cag ccg att cca cga ctc ttc ttt gcg gga gaa 5511
Ile Pro Gly Ala Pro Gln Pro Ile Pro Arg Leu Phe Phe Ala Gly Glu
                800                 805                 810

cat acg atc cgt aac tac cca gcc aca gtg cat ggt gct ctg ctg agt 5559
His Thr Ile Arg Asn Tyr Pro Ala Thr Val His Gly Ala Leu Leu Ser
            815                 820                 825

ggg ctg cga gaa gcg gga aga att gca gac cag ttt ttg ggg gcc atg 5607
Gly Leu Arg Glu Ala Gly Arg Ile Ala Asp Gln Phe Leu Gly Ala Met
        830                 835                 840

tat acg ctg cct cgc cag gcc aca cca ggt gtt cct gca cag cag tcc 5655
Tyr Thr Leu Pro Arg Gln Ala Thr Pro Gly Val Pro Ala Gln Gln Ser
    845                 850                 855

cca agc atg tga gctaggatct tattaaagca gaacttgttt attgcagctt 5707
Pro Ser Met
860

ataatggtta caaataaagc aatagcatca caaatttcac aaataaagca tttttttcac 5767

tgcattctag ttgtggtttg tccaaactca tcaatgtatc ttatcatgtc tggtcgactc 5827

tagcagtgaa agtctgcaat gaattcgagt tggcgcgcct gtcattctaa atctctcttt 5887

cagcctaaag ctttttcccc gtatcccccc aggtgtctgc aggctcaaag agcagcgaga 5947

agcgttcaga ggaaagcgat cccgtgccac cttccccgtg cccgggctgt ccccgcacgc 6007

tgccggctcg gggatgcggg gggagcgccg gaccggagcg gagccccggg cggctcgctg 6067

ctgcccccta gcgggggagg gacgtaatta catccctggt gggctttggg aggggggctg 6127

tccccgtgag ctcccaggcg cgcctgtcat tctaaatctc tctttcagcc taaagctttt 6187

tccccgtatc cccccaggtg tctgcaggct caaagagcag cgagaagcgt tcagaggaaa 6247

gcgatcccgt gccaccttcc ccgtgcccgg gctgtccccg cacgctgccg gctcggggat 6307

gcggggggag cgccggaccg gagcggagcc ccgggcggct cgctgctgcc ccctagcggg 6367

ggagggacgt aattacatcc ctgggggctt tggggggggg ctgtccccgt gagctcccag 6427

gcgcgccaac tcgctagagg taccggttgt taacgttagc cggctacgta tactccggaa 6487

tattaatagg cctaggatgc atatggcggc cgccaccgcg gtggagctcc agcttttgtt 6547

gcgcgcttgg cgtaatcatg gtcatagctg tttcctgtgt gaaattgtta tccgctcaca 6607
attccacaca acatacgagc cggaagcata aagtgtaaag cctggggtgc ctaatgagtg 6667
agctaactca cattaattgc gttgcgctca ctgcccgctt tccagtcggg aaacctgtcg 6727
tgccagctgc attaatgaat cggccaacgc gcggggagag gcggtttgcg tattgggcgc 6787
tcttccgctt cctcgctcac tgactcgctg cgctcggtcg ttcggctgcg gcgagcggta 6847
tcagctcact caaaggcggt aatacggtta tccacagaat caggggataa cgcaggaaag 6907
aacatgtgag caaaaggcca gcaaaaggcc aggaaccgta aaaaggccgc gttgctggcg 6967
tttttccata ggctccgccc ccctgacgag catcacaaaa atcgacgctc aagtcagagg 7027
tggcgaaacc cgacaggact ataaagatac caggcgtttc cccctggaag ctccctcgtg 7087
cgctctcctg ttccgaccct gccgcttacc ggatacctgt ccgcctttct cccttcggga 7147
agcgtggcgc tttctcatag ctcacgctgt aggtatctca gttcggtgta ggtcgttcgc 7207
tccaagctgg gctgtgtgca cgaacccccc gttcagcccg accgctgcgc cttatccggt 7267
aactatcgtc ttgagtccaa cccggtaaga cacgacttat cgccactggc agcagccact 7327
ggtaacagga ttagcagagc gaggtatgta ggcggtgcta cagagttctt gaagtggtgg 7387
cctaactacg gctacactag aaggacagta tttggtatct gcgctctgct gaagccagtt 7447
accttcggaa aaagagttgg tagctcttga tccggcaaac aaaccaccgc tggtagcggt 7507
ggtttttttg tttgcaagca gcagattacg cgcagaaaaa aaggatctca agaagatcct 7567
ttgatctttt ctacggggtc tgacgctcag tggaacgaaa actcacgtta agggattttg 7627
gtcatgagat tatcaaaaag gatcttcacc tagatccttt taaattaaaa atgaagtttt 7687
aaatcaatct aaagtatata tgagtaaact tggtctgaca gttaccaatg cttaatcagt 7747
gaggcaccta tctcagcgat ctgtctattt cgttcatcca tagttgcctg actccccgtc 7807
gtgtagataa ctacgatacg ggagggctta ccatctggcc ccagtgctgc aatgataccg 7867
cgagacccac gctcaccggc tccagattta tcagcaataa accagccagc cggaagggcc 7927
gagcgcagaa gtggtcctgc aactttatcc gcctccatcc agtctattaa ttgttgccgg 7987
gaagctagag taagtagttc gccagttaat agtttgcgca acgttgttgc cattgctaca 8047
ggcatcgtgg tgtcacgctc gtcgtttggt atggcttcat tcagctccgg ttcccaacga 8107
tcaaggcgag ttacatgatc ccccatgttg tgcaaaaaag cggttagctc cttcggtcct 8167
ccgatcgttg tcagaagtaa gttggccgca gtgttatcac tcatggttat ggcagcactg 8227
cataattctc ttactgtcat gccatccgta agatgctttt ctgtgactgg tgagtactca 8287
accaagtcat tctgagaata gtgtatgcgg cgaccgagtt gctcttgccc ggcgtcaata 8347
cgggataata ccgcgccaca tagcagaact ttaaaagtgc tcatcattgg aaaacgttct 8407
tcggggcgaa aactctcaag gatcttaccg ctgttgagat ccagttcgat gtaacccact 8467
cgtgcaccca actgatcttc agcatctttt actttcacca gcgtttctgg gtgagcaaaa 8527

acaggaaggc aaaatgccgc aaaaaaggga ataagggcga cacggaaatg ttgaatactc 8587

atactcttcc tttttcaata ttattgaagc atttatcagg gttattgtct catgagcgga 8647

tacatatttg aatgtattta gaaaaataaa caaatagggg ttccgcgcac atttccccga 8707

aaagtgccac 8717

<210> 7
<211> 862
<212> PRT
<213> artificial sequence

<220>
<223> Synthetic Construct

<400> 7

Met Asp Tyr Lys Asp Asp Asp Asp Lys Glu Phe Leu Ser Gly Lys Lys
1               5                   10                  15

Ala Ala Ala Ala Ala Ala Ala Ala Ala Ala Ala Ala Thr Gly Thr Glu
            20                  25                  30

Ala Gly Pro Gly Thr Ala Gly Gly Ser Glu Asn Gly Ser Glu Val Ala
        35                  40                  45

Ala Gln Pro Ala Gly Leu Ser Gly Pro Ala Glu Val Gly Pro Gly Ala
    50                  55                  60

Val Gly Glu Arg Thr Pro Arg Lys Lys Glu Pro Pro Arg Ala Ser Pro
65                  70                  75                  80

Pro Gly Gly Leu Ala Glu Pro Pro Gly Ser Ala Gly Pro Gln Ala Gly
                85                  90                  95

Pro Thr Val Val Pro Gly Ser Ala Thr Pro Met Glu Thr Gly Ile Ala
            100                 105                 110

Glu Thr Pro Glu Gly Arg Arg Thr Ser Arg Arg Lys Arg Ala Lys Val
        115                 120                 125

Glu Tyr Arg Glu Met Asp Glu Ser Leu Ala Asn Leu Ser Glu Asp Glu
    130                 135                 140

Tyr Tyr Ser Glu Glu Glu Arg Asn Ala Lys Ala Glu Lys Glu Lys Lys
145                 150                 155                 160

Leu Pro Pro Pro Pro Pro Gln Ala Pro Pro Glu Glu Glu Asn Glu Ser
                165                 170                 175

Glu Pro Glu Glu Pro Ser Gly Val Glu Gly Ala Ala Phe Gln Ser Arg
            180                 185                 190

Leu Pro His Asp Arg Met Thr Ser Gln Glu Ala Ala Cys Phe Pro Asp
        195                 200                 205

Ile Ile Ser Gly Pro Gln Gln Thr Gln Lys Val Phe Leu Phe Ile Arg
    210                 215                 220

Asn Arg Thr Leu Gln Leu Trp Leu Asp Asn Pro Lys Ile Gln Leu Thr
225                 230                 235                 240

Phe Glu Ala Thr Leu Gln Gln Leu Glu Ala Pro Tyr Asn Ser Asp Thr
                245                 250                 255

Val Leu Val His Arg Val His Ser Tyr Leu Glu Arg His Gly Leu Ile
            260                 265                 270

Asn Phe Gly Ile Tyr Lys Arg Ile Lys Pro Leu Pro Thr Lys Lys Thr
        275                 280                 285

Gly Lys Val Ile Ile Ile Gly Ser Gly Val Ser Gly Leu Ala Ala Ala
    290                 295                 300

Arg Gln Leu Gln Ser Phe Gly Met Asp Val Thr Leu Leu Glu Ala Arg
305                 310                 315                 320

Asp Arg Val Gly Gly Arg Val Ala Thr Phe Arg Lys Gly Asn Tyr Val
                325                 330                 335

Ala Asp Leu Gly Ala Met Val Val Thr Gly Leu Gly Gly Asn Pro Met
            340                 345                 350

Ala Val Val Ser Lys Gln Val Asn Met Glu Leu Ala Lys Ile Lys Gln
        355                 360                 365

Lys Cys Pro Leu Tyr Glu Ala Asn Gly Gln Ala Val Pro Lys Glu Lys
    370                 375                 380

Asp Glu Met Val Glu Gln Glu Phe Asn Arg Leu Leu Glu Ala Thr Ser
385                 390                 395                 400

Tyr Leu Ser His Gln Leu Asp Phe Asn Val Leu Asn Asn Lys Pro Val
                405                 410                 415

Ser Leu Gly Gln Ala Leu Glu Val Val Ile Gln Leu Gln Glu Lys His
            420                 425                 430

Val Lys Asp Glu Gln Ile Glu His Trp Lys Lys Ile Val Lys Thr Gln
        435                 440                 445

Glu Glu Leu Lys Glu Leu Leu Asn Lys Met Val Asn Leu Lys Glu Lys
    450                 455                 460

Ile Lys Glu Leu His Gln Gln Tyr Lys Glu Ala Ser Glu Val Lys Pro
465                 470                 475                 480

Pro Arg Asp Ile Thr Ala Glu Phe Leu Val Lys Ser Lys His Arg Asp
                485                 490                 495

Leu Thr Ala Leu Cys Lys Glu Tyr Asp Glu Leu Ala Glu Thr Gln Gly
            500                 505                 510

Lys Leu Glu Glu Lys Leu Gln Glu Leu Glu Ala Asn Pro Pro Ser Asp
        515                 520                 525

Val Tyr Leu Ser Ser Arg Asp Arg Gln Ile Leu Asp Trp His Phe Ala
    530                 535                 540

Asn Leu Glu Phe Ala Asn Ala Thr Pro Leu Ser Thr Leu Ser Leu Lys
545                 550                 555                 560

His Trp Asp Gln Asp Asp Asp Phe Glu Phe Thr Gly Ser His Leu Thr
                565                 570                 575

Val Arg Asn Gly Tyr Ser Cys Val Pro Val Ala Leu Ala Glu Gly Leu
            580                 585                 590

Asp Ile Lys Leu Asn Thr Ala Val Arg Gln Val Arg Tyr Thr Ala Ser
        595                 600                 605

Gly Cys Glu Val Ile Ala Val Asn Thr Arg Ser Thr Ser Gln Thr Phe
    610                 615                 620

Ile Tyr Lys Cys Asp Ala Val Leu Cys Thr Leu Pro Leu Gly Val Leu
625                 630                 635                 640

Lys Gln Gln Pro Pro Ala Val Gln Phe Val Pro Pro Leu Pro Glu Trp
                645                 650                 655

Lys Thr Ser Ala Val Gln Arg Met Gly Phe Gly Asn Leu Asn Lys Val
            660                 665                 670

Val Leu Cys Phe Asp Arg Val Phe Trp Asp Pro Ser Val Asn Leu Phe
        675                 680                 685

Gly His Val Gly Ser Thr Thr Ala Ser Arg Gly Glu Leu Phe Leu Phe
    690                 695                 700

Trp Asn Leu Tyr Lys Ala Pro Ile Leu Leu Ala Leu Val Ala Gly Glu
705                 710                 715                 720

Ala Ala Gly Ile Met Glu Asn Ile Ser Asp Asp Val Ile Val Gly Arg
                725                 730                 735

Cys Leu Ala Ile Leu Lys Gly Ile Phe Gly Ser Ser Ala Val Pro Gln
            740                 745                 750

Pro Lys Glu Thr Val Val Ser Arg Trp Arg Ala Asp Pro Trp Ala Arg
        755                 760                 765

Gly Ser Tyr Ser Tyr Val Ala Ala Gly Ser Ser Gly Asn Asp Tyr Asp
    770                 775                 780

Leu Met Ala Gln Pro Ile Thr Pro Gly Pro Ser Ile Pro Gly Ala Pro
785                 790                 795                 800

Gln Pro Ile Pro Arg Leu Phe Phe Ala Gly Glu His Thr Ile Arg Asn
                805                 810                 815

Tyr Pro Ala Thr Val His Gly Ala Leu Leu Ser Gly Leu Arg Glu Ala
            820                 825                 830

Gly Arg Ile Ala Asp Gln Phe Leu Gly Ala Met Tyr Thr Leu Pro Arg
        835                 840                 845

Gln Ala Thr Pro Gly Val Pro Ala Gln Gln Ser Pro Ser Met
    850                 855                 860